# EUROPÄISCHE PATENTSCHRIFT

(11) **EP 2 529 633 B1**
(45) Veröffentlichungstag und Bekanntmachung des Hinweises auf die Patenterteilung: **06.08.2014**
(21) Anmeldenummer: 11168468.4
(22) Anmeldetag: 01.06.2011
(51) Int. Cl.: A23L 1/236, C07H 15/256

(54) **Oral konsumierbare Zubereitungen umfassend bestimmte süß schmeckende Triterpene und Triterpenglycoside**
Orally consumed preparations comprising particular sweet tasting triterpenes and triterpene glycosides
Préparations consommables oralement comprenant du triterpène et du triterpènglycoside au goût sucré

(43) Veröffentlichungstag der Anmeldung: 05.12.2012
(73) Patentinhaber: Symrise AG, 37603 Holzminden (DE)
(72) Erfinder: Ley, Dr. Jakob, 37603 Holzminden (DE); Reichelt, Dr. Katharina, 37603 Holzminden (DE); Obst, Katja, 37603 Holzminden (DE); Wessjohann, Prof. Dr. Ludger, 06120 Halle (Saale) (DE); Wessjohann, Sabine, 06120 Halle (Saale) (DE); Van Sung, Prof. Dr. Tran, Hanoi (VN); Nguyen, The Anh, Hanoi (VN); Van Trai, Ngo, Hanoi (VN)
(74) Vertreter: Fabry, Bernd

(56) Entgegenhaltungen:
- US-A1- 2011 027 413
- CHOI Y-H ET AL: "ABRUSOSIDES A-D FOUR NOVEL SWEET-TASTING TRITERPENE GLYCOSIDES FROM THE LEAVES OF ABRUS-PRECATORIUS", JOURNAL OF NATURAL PRODUCTS (LLOYDIA), Bd. 52, Nr. 5, 1989, Seiten 1118-1127, XP002670036, ISSN: 0163-3864
- SUTTISRI R ET AL: "Periandrin V, a further sweet triterpene glycoside from Periandra dulcis", PHYTOCHEMISTRY, PERGAMON PRESS, GB, Bd. 34, Nr. 2, 1. September 1993 (1993-09-01), Seiten 405-408, XP026632048, ISSN: 0031-9422, DOI: 10.1016/0031-9422(93)80018-N [gefunden am 1993-09-01]
- HIURA AKI ET AL: "Taste-modifying triterpene glycosides from Staurogyne merguensis", PHYTOCHEMISTRY (OXFORD), Bd. 43, Nr. 5, 1996, Seiten 1023-1027, XP002670037, ISSN: 0031-9422
- YOSHIKAWA MASAYUKI ET AL: "Characterization of new sweet triterpene saponins from Albizia myriophylla.", JOURNAL OF NATURAL PRODUCTS NOV 2002 LNKD- PUBMED:12444690, Bd. 65, Nr. 11, November 2002 (2002-11), Seiten 1638-1642, XP002670038, ISSN: 0163-3864
- SUTTISRI RUTT ET AL: "Plant-derived triterpenoid sweetness inhibitors", JOURNAL OF ETHNOPHARMACOLOGY, Bd. 47, Nr. 1, 1995, Seiten 9-26, XP002670039, ISSN: 0378-8741

## Beschreibung

Die Erfindung betrifft Triterpene und Triterpenglycoside der nachfolgenden Formel (I) und/oder deren physiologisch akzeptable Salze, vorzugsweise natürlich vorkommende Triterpene und Triterpenglycoside aus *Mycetia balansae* und/oder deren physiologisch akzeptable Salze sowie oral konsumierbare Zubereitungen umfassend ein oder mehrere dieser Triterpene bzw. Triterpenglycoside und/oder deren physiologisch akzeptablen Salze. Die Erfindung betrifft ferner die Verwendung dieser Triterpene und Triterpenglycoside bzw. deren physiologisch akzeptablen Salze, vorzugsweise eines Extraktes von *Mycetia balansae,* zur Erzeugung eines Süßeindruckes in einer oral konsumierbaren Zubereitung oder zur Verstärkung des Süßeindruckes einer oral konsumierbaren Zubereitung umfassend mindestens einen weiteren, vorzugsweise natürlich vorkommenden, süß schmeckenden Stoff. Schließlich betrifft die Erfindung auch ein Verfahren zum Herstellen einer oral konsumierbaren Zubereitung sowie ein Verfahren zum Erzeugen und/oder Verstärken eines Süßeindruckes einer oral konsumierbaren Zubereitung.

Nahrungs- oder Genussmittel, die einen hohen Zuckergehalt (vor allem Sucrose (= Saccharose), Lactose, Glucose oder Fructose oder deren Mischungen) aufweisen, werden in der Regel von Verbrauchern auf Grund der Süße stark präferiert. Auf der anderen Seite ist allgemein bekannt, dass ein hoher Gehalt an leicht verstoffwechselbaren Kohlenhydraten den Blutzuckerspiegel stark ansteigen lässt, zur Bildung von Fettdepots führt und letztendlich zu gesundheitlichen Problemen wie Übergewicht, Fettleibigkeit, Insulinresistenz, Altersdiabetes und deren Folgeerkrankungen führen kann. Insbesondere kommt erschwerend hinzu, dass viele der oben genannten Kohlenhydrate zusätzlich die Zahngesundheit beeinträchtigen können, da sie von bestimmten Bakteriensorten in der Mundhöhle zu beispielsweise Milchsäure abgebaut werden und den Zahnschmelz der juvenilen oder adulten Zähne angreifen können (Karies).

Daher ist es schon lange ein Ziel, den Zuckergehalt von Nahrungs- und/oder Genussmitteln so weit wie möglich zu reduzieren, wobei bevorzugtes Ziel ist, diese Reduktion mit möglichst geringer Verringerung des Süßeindruckes zu erreichen. Eine entsprechende Maßnahme besteht im Einsatz von Süßstoffen: das sind chemisch einheitliche Substanzen, die selbst keinen oder nur einen sehr geringen kalorischen Brennwert haben und gleichzeitig einen starken süßen Geschmackseindruck verursachen; die Stoffe sind in der Regel nicht-kariogen (eine Übersicht ist z.B. zu finden in Journal of the American Dietetic Association 2004, 104 (2), 255-275). Die sogenannten Bulk-Süßstoffe wie Sorbitol, Mannitol oder andere Zuckeralkohole sind zwar teilweise ausgezeichnete Süßungsmittel und können auch die übrigen nahrungsmitteltechnischen Eigenschaften von Zuckern teilweise ersetzen, führen aber bei zu häufiger Aufnahme bei einem Anteil der Bevölkerung zu osmotisch bedingten Verdauungsproblemen. Die nicht-nutritiven, hochintensiven Süßstoffe sind zwar durch ihre geringe Einsatzkonzentration gut geeignet, Süße in Nahrungsmittel zu bringen. Eine Reihe dieser Süßstoffe sind jedoch nicht natürlichen Ursprungs (Sucralose, Cyclamat, Acesulfam K, Saccharin, Aspartam), zeigen geschmackliche Probleme durch im Vergleich zu Zucker unähnliche Zeit-Intensitätsprofile (z.B. Sucralose, Stevioside, Cyclamat), einen bitteren und/oder adstringierenden Nachgeschmack (z.B. Acesulfam K, Saccharin, Stevioside, Rebaudioside), ausgeprägte zusätzliche Aromaeindrücke (z.B. Glycerrhyzinsäureammoniumsalz). Einige der Süßstoffe sind gegenüber Hitze nicht besonders stabil (z.B. Thaumatin, Brazzein, Monellin), sind nicht in allen Anwendungen stabil (z.B. Aspartam) und sind teilweise sehr langanhaltend in ihrer süßen Wirkung (starker süßer Nachgeschmack, z.B. Saccharin, Sucralose, Stevioside, Rebaudioside, Neotam, Advantam, Superaspartam).

Daher ist es wünschenswert Süßstoffe zu finden, die einen intensiven, dem Rohrzucker ähnlichen Süßgeschmack aufweisen bzw. vermitteln, welche vorzugsweise zudem natürlich vorkommen oder leicht aus natürlich vorkommenden Stoffen erhältlich sind und darüberhinaus vorzugsweise in oral konsumierbaren Zubereitungen stabil und/oder breit anwendbar sind.

Eine andere Möglichkeit den kalorischen Gehalt von Nahrungsmitteln oder Getränken zu senken - ohne den Einsatz von nicht-nutritiven Süßstoffen - besteht darin, den Zuckergehalt von Nahrungs- und/oder Genussmitteln zu reduzieren und sensorisch schwach oder nicht wahrnehmbare Stoffe zuzusetzen, die die Süße mittelbar oder unmittelbar verstärken, wie z.B. in WO 2005/041684 beschrieben. Die in WO 2005/041684 beschriebenen Stoffe sind aber ausdrücklich nicht-natürlichen Ursprungs und somit aus toxikologischer Sicht schwieriger zu bewerten als Stoffe natürlicher Herkunft, insbesondere wenn letztere in Nahrungs- oder Genussmitteln vorkommen oder aus Rohstoffen zur Nahrungs- oder Genussmittelgewinnung stammen. In EP 1 291 342 werden solche Stoffe natürlicher Herkunft (Pyridinium-Betaine) beschrieben; allerdings wird durch sie nicht selektiv der süße Geschmack, sondern auch andere Geschmacksrichtungen wie Umami oder Salzigkeit beeinflusst. Zudem sind die offenbarten Stoffe nur mit hohem Aufwand zu reinigen und/oder schwierig synthetisch herzustellen.

In WO 2007/014879 A1 wird der Einsatz von Hesperetin und in WO 2007/107596 A1 wird Phloretin als Verstärker des süßen Geschmacks von Zucker-reduzierten, der Ernährung oder dem Genuss dienenden Zubereitungen empfohlen. Bisweilen nachteilig ist allerdings bei Einsatz von Hesperetin und Phloretin die vergleichsweise schwach ausgeprägte Süßverstärkung in Nahrungs- und Genussmitteln, die hohe Anteile an Proteinen, insbesondere denaturierte Proteine oder Polysaccharide enthalten wie z.B. Joghurt-Produkte. Hesperetin zeigt zudem den Nachteil, in sehr sauren und carbonisierten Anwendungen wie Limonaden oder Cola-Getränken nicht ausreichend wirksam zu sein.

Daher war es wünschenswert, eine Möglichkeit zu finden, bei gleichbleibendem Süßeindruck den Gehalt an süß schmeckenden Verbindungen in oral konsumierbaren Zubereitungen senken zu können, so dass Zucker-reduzierte Zubereitungen entstehen. Insbesondere besteht ein Bedarf an Mitteln, die einen gegebenen Süßeindruck verstärken, insbesondere auch über einen rein additiven Effekt hinaus.

Dementsprechend war es die primäre Aufgabe der vorliegenden Erfindung, Stoffe (einzelne Substanzen oder Substanzgemische) zu finden, die einen Süßeindruck erzeugen können (d.h. eine Eigensüße aufweisen) und/oder den Süßeindruck anderer süß schmeckender Stoffe verstärken können.

Ferner sollten entsprechende oral konsumierbare Zubereitungen angegeben werden, bei denen der Süßeindruck durch diese Stoffe erzeugt wird oder deren Süßeindruck durch diese Stoffe verstärkt wird bei weitgehend unveränderter Konzentration an weiteren süß schmeckenden Verbindungen oder bei denen bei gleichbleibendem Süßeindruck der Gehalt an weiteren süßen Geschmackseindruck erzeugenden Verbindungen verringert ist, wobei bevorzugt keine oder lediglich in geringfügigem Ausmaß negative sensorische Nebeneffekte auftreten sollten.

Dabei wäre es zudem von Vorteil, wenn diese Stoffe natürlich vorkommen würden bzw. auf Basis natürlicher und nachwachsender Stoffe oder Rohstoffe herstellbar wären. Insbesondere sollten die gesuchten Süßeindruck vermittelnden oder süßeindruckverstärkenden Stoffe vorzugsweise bereits in geringen Konzentrationen wirksam sein. Ferner sollten die gesuchten Stoffe möglichst breit einsetzbar sein, d.h. in viele verschiedene oral anwendbare Applikations- und Produktformen eingearbeitet werden können und entsprechend mit vielen verschiedenen oral konsumierbaren Grund-, Hilfs-, Träger-, Zusatz- und/oder Wirkstoffen kombinierbar und verträglich sein.

Erfindungsgemäß wird diese Aufgabe gelöst durch die Verbindungen der Formel (I) (im Rahmen dieses Textes als Balansine bezeichnet) oder die physiologisch akzeptablen Salze der Verbindungen der Formel (I) 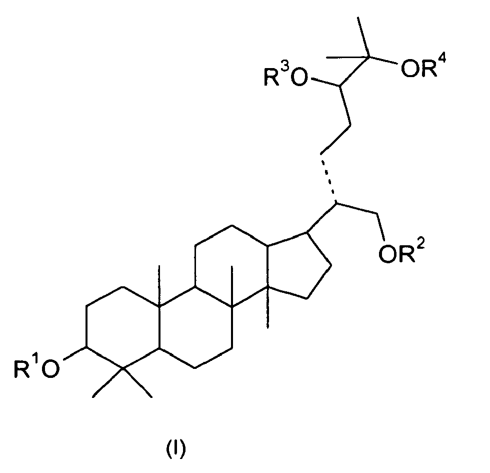 wobei
die gestrichelte Linie eine Einfach- oder eine Doppelbindung darstellt, und
R¹, R², R³ und R⁴ jeweils unabhängig voneinander Wasserstoff oder einen Zuckerrest darstellen, dabei vorzugsweise einen Monosaccharidrest oder einen Oligosaccharidrest,
wobei das Gegenkation des physiologisch akzeptablen Salzes der Verbindung der Formel (I) vorzugsweise ausgewählt ist aus der Gruppe bestehend aus einfach positiv geladenen Kationen der ersten Haupt- und Nebengruppe, Ammoniumoin, Trialkylammoniumionen, zweiwertig geladenen Kationen der zweiten Nebengruppe, dreiwertigen Kationen der dritten Haupt- und Nebengruppe, und bevorzugt ausgewählt ist aus der Gruppe bestehend aus Na⁺, K⁺, NH₄⁺, Ca²⁺, Mg²⁺, Al³⁺ und Zn²⁺.

Die erfindungsgemäßen Verbindungen der Formel (I), ebenso wie die nachfolgend beschriebenen erfindungsgemäß bevorzugten Verbindungen der Formel (II) und (III), können in Form ihrer verschiedenen möglichen Stereoisomere und beliebigen Mischungen der verschiedenen möglichen Stereoisomere vorliegen und im Rahmen der vorliegenden Erfindung eingesetzt werden.

Sofern eine Verbindung der Formel (I) einen oder mehrere Zuckerreste umfasst, können die Zuckerreste jeweils unabhängig voneinander α- oder β-konfiguriert sein. Dies gilt sowohl bezüglich der Verknüpfung an das Sauerstoffatom, über welches die Reste R¹, R², R³ und R⁴ an das Triterpengerüst gebunden sind, als auch für die Verknüpfung der jeweiligen Einfachzuckerbausteine untereinander, welche den jeweiligen Zuckerrest bilden.

Vorzugsweise bedeuten in der Formel (I) ein, zwei oder drei der Reste R¹, R², R³ und R⁴ Wasserstoff und ein, zwei oder drei der Reste R¹, R², R³ und R⁴ einen Zuckerrest.

Bevorzugte Verbindungen der Formel (I) bzw. physiologisch akzeptable Salze einer Verbindungen der Formel (I) sind solche, in denen zumindest einer der Reste R¹, R², R³ und R⁴ Wasserstoff bedeutet, vorzugsweise zumindest zwei der Reste R¹, R², R³ und R⁴ Wasserstoff bedeuten, und
zumindest einer der Reste R¹, R², R³ und R⁴ einen Zuckerrest bedeutet, wobei vorzugsweise der Zuckerrest ausgewählt ist bzw. die Zuckerreste jeweils unabhängig voneinander ausgewählt sind aus der Gruppe bestehend aus
(i) den Monosaccharidresten Glucosyl, Mannosyl, Galactosyl, Rhamnosyl, Fucosyl, Arabinosyl und Ribosyl,
   und
(ii) den Oligosaccharidresten aus 2 bis 10 Einfachzuckerbausteinen, wobei die Einfachzuckerbausteine vorzugsweise ausgewählt sind aus der Gruppe bestehend aus Glucose, Mannose, Galactose, Rhamnose, Fucose, Arabinose und Ribose.

Vorzugsweise bedeuten in der Formel (I) zwei oder drei der Reste R², R³ und R⁴ Wasserstoff und der Rest R¹ sowie optional einer der Reste R², R³ und R⁴ einen Zuckerrest, wobei vorzugsweise der Zuckerrest ausgewählt ist bzw. die Zuckerreste jeweils unabhängig voneinander ausgewählt sind aus der Gruppe bestehend aus
(i) den Monosaccharidresten Glucopyranosyl, Mannopyranosyl, Galactopyranosyl, Rhamnopyranosyl, Fucopyranosyl, Arabinosyl und Ribosyl,
   und
(ii) den Oligosaccharidresten aus 2 bis 8 Einfachzuckerbausteinen, wobei die Einfachzuckerbausteine vorzugsweise ausgewählt sind aus der Gruppe bestehend aus Glucopyranose, Mannopyranose, Galactopyranose, Rhamnopyranose, Fucopyranose, Arabinose und Ribose.

Vorzugsweise bedeuten ein, zwei oder sämtliche Reste R², R³ und R⁴ in Formel (I) Wasserstoff.

Vorzugsweise stellt die gestrichelte Linie in Formel (I) eine Doppelbindung dar.

Erfindungsgemäß bevorzugt sind Verbindungen der Formel (II) oder ein physiologisch akzeptables Salz einer Verbindung der Formel (II) 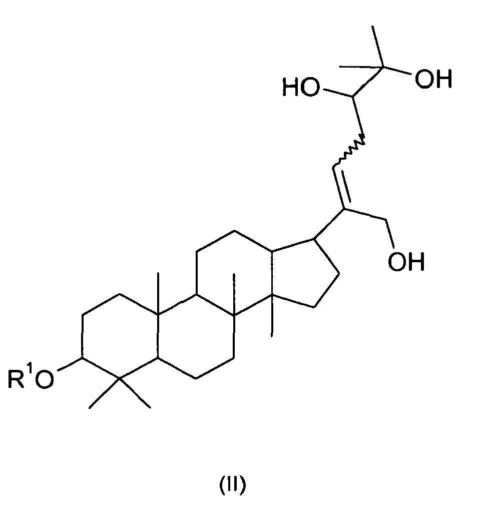 wobei
R¹ ein Mono-, Di-, Tri-, Tetra- oder Pentasaccharidrest ist, vorzugsweise ausgewählt aus der Gruppe bestehend aus
(i) Glucosyl, Mannosyl, Galactosyl, Rhamnosyl, Fucosyl, Arabinosyl und Ribosyl, und
(ii) den Oligosaccharidresten aus 2 bis 5 Einfachzuckerbausteinen, wobei die Einfachzuckerbausteine ausgewählt sind aus der Gruppe bestehend aus Glucose, Mannose, Galactose, Rhamnose, Fucose, Arabinose und Ribose.

Die geschlängelte Linie in der Formel (II) steht dabei gemäß der allgemein gebräuchlichen Zeichnungsweise für eine (E)- oder eine (Z)-konfigurierte Doppelbindung.

Erfindungsgemäß besonders bevorzugt sind Verbindungen der Formel (III) oder ein physiologisch akzeptables Salz einer Verbindung der Formel (III) 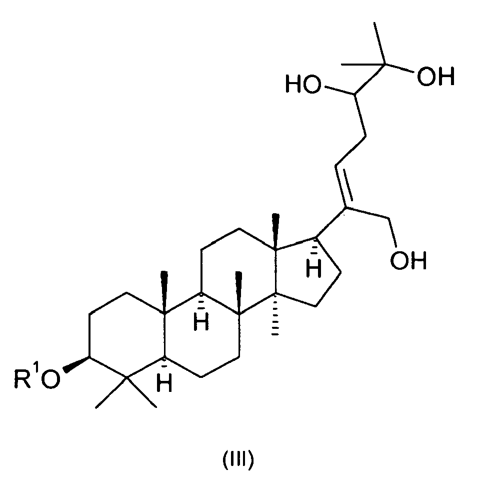 wobei
R¹ ein Mono-, Di-, Tri-, Tetra- oder Pentasaccharidrest ist ausgewählt aus der Gruppe bestehend aus
(i) Glucosyl, Mannosyl, Galactosyl, Rhamnosyl, Fucosyl, Arabinosyl und Ribosyl, und
(ii) den Oligosaccharidresten aus 2 bis 5 Einfachzuckerbausteinen, wobei die Einfachzuckerbausteine ausgewählt sind aus der Gruppe bestehend aus Glucose, Mannose, Galactose, Rhamnose, Fucose, Arabinose und Ribose.

Erfindungsgemäß weiter bevorzugt sind Verbindungen der obigen Formel (III) oder ein physiologisch akzeptables Salz einer Verbindung der obigen Formel (III), wobei
R¹ ein Mono-, Di-, Tri-, Tetra- oder Pentasaccharidrest ist ausgewählt aus der Gruppe bestehend aus
(i) Glucosyl, Galactosyl und Rhamnosyl,
   und
(ii) den Oligosaccharidresten aus 2 bis 4 Einfachzuckerbausteinen, wobei die Einfachzuckerbausteine ausgewählt sind aus der Gruppe bestehend aus Glucose, Galactose und Rhamnose, wobei vorzugsweise die Einfachzuckerbausteine ihrerseits miteinander an den Positionen 2, 4 und/oder 6 verknüpft sind.

Erfindungsgemäß ganz besonders bevorzugt sind die beiden nachfolgenden Verbindungen oder ein physiologisch akzeptables Salz dieser Verbindungen (nachfolgend als Balansin A bzw. Balansin B bezeichnet).

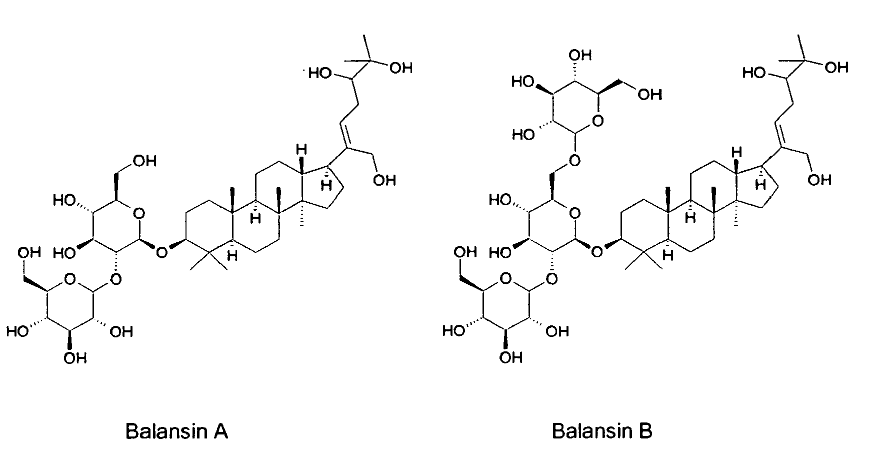

Erfindungsgemäß können auch Gemische von zwei, drei oder mehr verschiedenen Verbindungen der Formeln (I), (II) oder (III) eingesetzt werden, wobei ein solches Gemisch vorzugsweise zwei, drei oder mehr der oben als bevorzugt gekennzeichneten Verbindungen umfasst.

Die erfindungsgemäßen Verbindungen der Formel (I), (II) bzw. (III) können ebenfalls bevorzugt als ein-, oder beispielsweise im Fall mehrerer Hydroxygruppen, mehrwertige Anionen vorliegen, wobei vorzugsweise als Gegenkation die einfach positiv geladenen Kationen der ersten Haupt- und Nebengruppe, das Ammoniumion, ein Trialkylammoniumion, die zweiwertig geladenen Kationen der zweiten Nebengruppe, sowie die dreiwertigen Kationen der 3. Haupt- und Nebengruppe dienen, bevorzugt Na⁺, K⁺, NH₄⁺, Ca²⁺, Mg²⁺, Al³⁺ und Zn²⁺.

Sofern im Rahmen des vorliegenden Textes bevorzugte bzw. besonders bevorzugte Verbindungen der Formeln (I), (II) bzw. (III) genannt sind, sind selbstverständlich auch die Salze solcher Verbindungen erfindungsgemäß bevorzugt bzw. besonders bevorzugt.

Erfindungsgemäß bevorzugt sind daher auch ein, zwei oder mehrere verschiedene Salze einer, zweier oder mehrerer verschiedener Verbindungen der Formel (I), (II) bzw. (III) wie oben definiert
oder
ein Gemisch von ein, zwei oder mehreren verschiedenen Verbindungen der Formel (I), (II) bzw. (III) wie oben definiert und einem, zwei oder mehreren verschiedenen Salzen einer, zweier oder mehrerer verschiedener Verbindungen der Formel (I), (II) bzw. (III) wie oben definiert,
wobei das bzw. die Gegenkationen des bzw. eines, mehrerer oder sämtlicher der Salze vorzugsweise ausgewählt ist bzw. sind aus der Gruppe bestehend aus Na⁺, K⁺, NH₄⁺, Ca²⁺, Mg²⁺, Al³⁺ und Zn²⁺.

Entsprechend betrifft die vorliegende Erfindung auch ein Gemisch umfassend oder bestehend aus
- zwei, drei oder mehreren Verbindungen wie vorstehend definiert, vorzugsweise in einer der als bevorzugt gekennzeichneten Ausgestaltungen,
   oder
- ein, zwei oder mehreren verschiedene Verbindungen wie vorstehend definiert und einem, zwei oder mehreren verschiedenen physiologisch akzeptablen Salzen einer, zweier oder mehrerer verschiedener Verbindungen wie vorstehend definiert, vorzugsweise in einer der als bevorzugt gekennzeichneten Ausgestaltungen,
   oder
- zwei, drei oder mehreren physiologisch akzeptablen Salzen der Verbindungen wie vorstehend definiert, vorzugsweise in einer der als bevorzugt gekennzeichneten Ausgestaltungen,
   wobei, sofern in dem Gemisch ein oder mehrere physiologisch akzeptable Salze einer oder mehrerer Verbindungen wie vorstehend definiert vorhanden ist bzw. sind, vorzugsweise die Gegenkationen sämtlicher physiologisch akzeptabler Salze der Verbindungen wie vorstehend definiert ausgewählt sind aus der Gruppe bestehend aus Na⁺, K⁺, NH₄⁺, Ca²⁺, Mg²⁺, Al³⁺ und Zn²⁺.

Besonders bevorzugte erfindungsgemäße Gemische umfassen Balansin A und/oder Balansin B, vorzugsweise Balansin A und Balansin B, bevorzugt Balansin A und Balansin B und optional ein, zwei, drei oder mehr weitere Verbindungen der Formeln (I), (II) und/oder (III) und/oder optional ein, zwei, drei oder mehrere physiologisch akzeptable Salze einer oder mehrerer Verbindungen der Formeln (I), (II) und/oder (III).

Besonders bevorzugte erfindungsgemäße Gemische umfassen ein physiologisch akzeptables Salz von Balansin A und/oder von Balansin B, vorzugsweise ein Na⁺, K⁺, NH₄⁺, Ca²⁺, Mg²⁺, Al³⁺ oder Zn²⁺- Salz von Balansin A und von Balansin B sowie optional ein, zwei, drei oder mehrere weitere physiologisch akzeptable Salze einer oder mehrerer Verbindungen der Formeln (I), (II) und/oder (III).

Die Verbindungen der Formeln (I), (II) und (III) können aus der Pflanze *Mycetia balansae* Drake erhalten und beispielsweise aus den Extrakten aus *Mycetia balansae* angereichert bzw. isoliert werden, oder sind durch Derivatisierung bzw. Umwandlung der in dieser Pflanze vorkommenden und in den Extrakten vorhandenen Triterpenen bzw. Triterpenglycosiden herstellbar, beispielsweise mittels Hydrierung, Glycosidierung, Glykolyse, Salzbildung und ähnlicher dem Fachmann bekannter Verfahren.

Insbesondere das Glycosidmuster kann beispielsweise durch Glycosidierung oder Transglycosidierung bzw. Transferglycosidierung mit Hilfe der entsprechenden Enzyme verändert werden, z.B. unter Verwendung von Glycosidasen oder Transglycosidasen als Enzyme oder auch Organismen wie z.B. Bakterien mit entsprechenden Aktivitäten eingesetzt werden (z.B. nach KR 888694). Dabei können auch andere, erfindungsgemäß nicht bevorzugte Glycoside oder Glycane als Reaktanden eingesetzt werden, bevorzugt sind jedoch aus Glucose, Mannose, Galactose, Rhamnose, Fucose, Arabinose und Ribose zusammengesetzte Glycoside oder Glycane.

Die Verbindungen der Formel (I), bei denen die gestrichelte Linie eine Einfachbindung darstellt können aus den entsprechenden Verbindungen der Formel (I) bei denen die gestrichelte Linie eine Doppelbindung darstellt, durch dem Fachmann geläufige homogen oder heterogen metallkatalytisierte Hydrierung oder Transferhydrierung oder auch durch eine enzymatisch oder durch Organismen bewerkstelligte Wasserstoffaddition hergestellt werden.

*Mycetia balansae* Drake (Familie Rubiaceae, Genus: Mycetia; Global Biodiversity Information Facility: bestätigt durch Kew Garden Record-Nummer Kew-130690, http://data.gbif.org/species/14242490/) ist eine Pflanze, die in Südostasien verbreitet ist.

Ein Verzehr von *Mycetia balansae* Drake ist in der Literatur nicht beschrieben. Ein Kauen der frischen, d.h. nicht getrockneten, Blätter durch die lokale Bevölkerung wurde wohl, wenn überhaupt, lediglich anekdotisch mündlich überliefert. Eine Verwendung zur Süßung von oral verzehrbaren Zubereitungen wie beispielsweise Lebens- oder Genussmitteln ist dabei nicht berichtet worden. Ebenso ist die chemische Zusammensetzung weder von der Spezies *Mycetia balansae* Drake noch von anderen *Mycetia* Arten beschrieben worden. Die Balansine der Formel (I) sind daher neu. Die Verbindungen der Formel (I) sowie deren Salze sind bislang weder als Bestandteil von *Mycetia balansae* beschrieben worden, noch aus anderen natürlichen oder synthetischen Quellen bekannt.

Daher war es überraschend und für den Fachmann nicht voraussehbar, dass die oben beschriebenen erfindungsgemäßen Balansine der Formel (I) einen starken Süßeindruck verursachen bzw. den Süßeindruck anderer süß schmeckender Stoffe, wie z.B. einer wässrigen Sucroselösung, in signifikantem und ausgeprägtem Maße verstärken können.

Die erfindungsgemäßen Verbindungen der Formel (I), (II) bzw. (III) und/oder deren Salze bzw. ein erfindungsgemäßes Gemisch (wie oben definiert) werden erfindungsgemäß vorzugsweise - im Vergleich zu Pflanzenteilen, insbesondere den (vorzugsweise frischen) Blättern, von *Mycetia balansae* - in angereicherter Form eingesetzt, bevorzugt in aufgereinigter oder in isolierter Form.

Nicht Gegenstand der vorliegenden Erfindung ist die frische oder getrocknete Pflanze *Mycetia balansae* als solche. Auch nicht Gegenstand der vorliegenden Erfindung ist ein frischer oder getrockneter Pflanzenteil (wie beispielsweise Blätter) von *Mycetia balansae* als solcher, dabei insbesondere nicht die frischen oder getrockneten Blätter von *Mycetia balansae*.

Vorzugsweise ebenfalls nicht Gegenstand der vorliegenden Erfindung ist die beim Kauen, insbesondere die beim Kauen durch einen Menschen, der frischen Blätter von *Mycetia balansae* entstehende Mischung. Vorzugsweise ebenfalls nicht Gegenstand der vorliegenden Erfindung ist ein beim Kauen der frischen Blätter von *Mycetia balansae* entstehender Speichelextrakt, bevorzugt kein menschlicher Speichelextrakt. In einer bevorzugten Ausgestaltung ist eine erfindungsgemäße oral konsumierbare Zubereitung frei von menschlichem Speichel, bevorzugt frei von Speichel.

Erfindungsgemäß können auch die Pflanze oder Pflanzenteile von *Mycetia balansae* in getrockneter und gemahlener, vorzugsweise pulverisierter, Form eingesetzt werden.

Eine erfindungsgemäße oral konsumierbare Zubereitung, beispielsweise in Form einer Halbfertigware, kann beispielsweise die Pflanze oder Pflanzenteile von *Mycetia balansae,* vorzugsweise in getrockneter und pulverisierter Form, umfassen sowie ein oder mehrere zum Verzehr geeignete Zusatz-, Träger- und/oder Hilfsstoffe. Vorzugsweise umfasst eine erfindungsgemäße Halbfertigware eine oder mehrere der Verbindungen der Formel (I) bzw. ein oder mehrere physiologisch akzeptable Salze der Verbindungen der Formel (I) und ein oder mehrere zum Verzehr geeignete bei 20°C und 1013 mbar feste Zusatz-, Träger- und/oder Hilfsstoffe.

Die Erfindung betrifft auch einen pflanzlichen Extrakt, vorzugsweise einen pflanzlichen Extrakt von *Mycetia balansae,* umfassend eine oder mehrere Verbindungen der Formel (I) oder ein physiologisch akzeptables Salz einer Verbindung der Formel (I), oder ein erfindungsgemäßes Gemisch, wobei vorzugsweise die Gesamtmenge dieser Verbindungen und der physiologisch akzeptablen Salze dieser Verbindungen im Bereich von 0,00001 bis 99 Gew.-% liegt, bevorzugt im Bereich von 0,0001 bis 95 Gew.-%, besonders bevorzugt im Bereich von 0,0005 bis 80 Gew.-%, insbesondere bevorzugt im Bereich von 0,001 bis 30 Gew.-%, bezogen auf die Trockenmasse des Extraktes.

Unter dem Begriff "Trockenmasse" im weiteren Sinne wird die wasser- und extraktionsmittelfreie Masse eines erfindungsgemäßen Extraktes verstanden. Eine solche Masse kann beispielsweise erhalten werden, indem nach Beendung des oder der Extraktionsschritte eines erfindungsgemäßen Extraktionsverfahrens (siehe unten) das Extraktionsmittel durch destillative oder andere evaporative Verfahren vollständig entfernt wird (einschließlich des Wassers, welches gegebenenfalls aus dem pflanzlichen Material stammt).

Unter dem Begriff "Trockenmasse" im engeren Sinne wird die Gesamtmasse aller Feststoffe des Extraktes verstanden, bezogen auf 20°C bei 1013 mbar.

Im Rahmen des vorliegenden Textes bezieht sich der Zustand "fest" bzw. der Begriff "Feststoff" auf 20°C bei 1013 mbar.

Für den Bereich der Nahrungs- und Genussmittel geeignete Extraktionsmittel sind beispielsweise Wasser, Ethanol, Methanol, 1-Propanol, 2-Propanol, 1,2-Propandiol, Glycerin, Aceton, Dichlormethan, Essigsäureethylester (Ethylacetat), Diethylether, Hexan, Heptan, Triacetin, pflanzliche Öle oder Fette, superkritisches Kohlendioxid und deren Gemische. Besonders bevorzugt sind dabei Wasser, Ethanol, Methanol, 1-Propanol, 2-Propanol, Glycerin, 1,2-Propandiol oder Gemische der vorgenannten nicht-wässrigen Extraktionsmittel mit Wasser.

Ein erfindungsgemäß bevorzugter pflanzlicher Extrakt ist erhältlich oder wird erhalten durch ein Verfahren mit folgendem Schritt:
a) ein- oder mehrfache Extraktion von pflanzlichem Material von *Mycetia balansae* mit einem, vorzugsweise flüssigen, Extraktionsmittel ausgewählt aus der Gruppe bestehend aus Wasser, Ethanol, Methanol, 1-Propanol, 2-Propanol, Glycerin, 1,2-Propandiol, superkritischem Kohlendioxid, Essigsäureethylester und deren Mischungen,
   sowie gegebenenfalls einem oder mehreren weiteren der folgenden Schritte:
b) gegebenenfalls Aufkonzentrierung des in Schritt a) erhaltenen Primärextraktes, vorzugsweise durch ein oder mehrere evaporative oder pervaporative Verfahren,
c) gegebenenfalls Behandlung des (gegebenenfalls in Schritt b) aufkonzentrierten) Primärextraktes mit oder an Adsorbentien, vorzugsweise ausgewählt aus der Gruppe bestehend aus Kieselgel, modifiziertes Kieselgel, Aktivkohle, Zeolith, Bentonit, Kieselgur, Aluminiumoxid, basischer oder saurer oder neutraler, optional makroporöser, Ionentauscher, bevorzugt im Batch- oder Säulenverfahren, gegebenenfalls unter Zuhilfenahme weiterer Extraktionsmittel, wodurch ein aufgereinigter Extrakt (Sekundärextrakt) erhalten wird,
d) gegebenenfalls Trocknung des in Schritt c) erhaltenen Sekundärextraktes, vorzugsweise durch ein evaporatives oder pervaporatives Verfahren,
e) gegebenenfalls Mischen des in Schritt d) erhaltenen getrockneten Sekundärextraktes mit einem geeigneten Verdünnungsmittel oder einem Gemisch zweier oder mehrerer Verdünnungsmittel, bevorzugt ausgewählt aus der Gruppe bestehend aus Ethanol, Isopropanol, 1,2-Propylenglycol, Pflanzenöltriglyceriden, Diacetin, Triacetin und Glycerin, wobei vorzugsweise eine Lösung erhalten wird.

Ein erfindungsgemäßer Extrakt aus *Mycetia balansae,* der vorzugsweise zur Herstellung einer erfindungsgemäßen oral konsumierbaren Zubereitung eingesetzt wird, kann vorzugsweise aus der frischen und/oder getrockneten Pflanze oder deren Teilen (beispielswiese Wurzeln, Stängel, Rinde, Mark, Blätter, Blüten und/oder Früchten), bevorzugt aus den getrockneten oberirdischen Pflanzenteilen (dabei vorzugsweise Blätter, Stängel, Blüten und/oder Früchten) von *Mycetia balansae* mittels Extraktion gewonnen werden.

Zur Herstellung eines erfindungsgemäßen Extraktres können auch die Pflanze oder Pflanzenteile von *Mycetia balansae* in getrockneter und gemahlener, vorzugsweise pulverisierter, Form eingesetzt werden.

Ein erfindungsgemäßer pflanzlicher Extrakt, vorzugsweise aus *Mycetia balansae,* wird vorzugsweise durch ein erfindungsgemäßes Extraktionsverfahren, vorzugsweise aus *Mycetia balansae,* erhalten.

In einer bevorzugten Ausgestaltung umfasst ein erfindungsgemäßer pflanzlicher Extrakt neben einer oder mehreren Verbindungen der Formel (I) ferner nur solche Verbindungen, die bei 20°C und 1013 mbar fest sind und die vorzugsweise keine freien Kohlenhydrate sind, insbesondere keine süß schmeckenden freien Kohlenhydrate.

Bevorzugt zählen zu dem erfindungsgemäßen pflanzlichen Extrakt lediglich solche Verbindungen im Sinne des Vorgesagten, die aus *Mycetia balansae* mittels Ethylacetat, Ethanol oder Methanol sowie eines Methanol-Ethanol-, Methanol-Wasser-, Methanol-Ethanol-Wasser- oder Ethanol-Wasser-Gemisches, oder superkritischem Kohlendioxid (gegebenenfalls in Kombination mit einem polaritätssteigernden Mittel wie Ethanol) extrahiert werden können.

Die Extraktionsdauer des oder der Extraktionsschritte beträgt vorzugsweise jeweils 5 Minuten bis 24 h, bevorzugt jeweils 15 Minuten bis 18 h.

Vorzugsweise erfolgt Schritt a) durch Ausrühren, Soxhlet-Extraktion, GegenstromExtraktion, Perkolation oder in einem Siebkorbverfahren.

Ein, mehrere oder sämtliche Extraktionen (Extraktionsschritte) eines erfindungsgemäßen Verfahrens werden vorzugsweise jeweils bei Temperaturen im Bereich von -80 °C bis zur jeweiligen Siedetemperatur des Extraktionsmittels bzw. Extraktionsmittelgemisches, vorzugsweise im Bereich von 0 bis 200°C, durchgeführt. Jeder Extraktionsschritt wird vorzugsweise für die Dauer von 5 Minuten bis 24 h durchgeführt, vorzugsweise durch Rühren (vorzugsweise einfaches Rühren), Perkolieren oder Gegenstromextraktion. Evaporative oder pervaporative Verfahren können dabei z.B. Destillation, Sublimation, Wasserdampfdestillation, Gefriertrocknung, pervaporative Membranverfahren oder Sprühtrocknung sein, wobei dazu auch vor und/oder während dieser Verfahren geeignete Hilfs- und/oder Trägerstoffe zugesetzt werden können.

Der oder die Extraktionsschritte können vorzugsweise durch Rühren (z.B. einfaches Rühren), Hochdruckextraktion, Perkolieren und/oder Gegenstromextraktion erfolgen.

Ein erfindungsgemäßer pflanzlicher Extrakt von *Mycetia balansae* wird bevorzugt aus frischen und/oder getrockneten oberirdischen Pflanzenteilen, bevorzugt Blättern, Stängeln, Blüten und/oder Früchten, vorzugsweise in getrockneter und zerkleinerter Form, gewonnen, vorzugsweise umfassend folgenden Schritt:
a) Extraktion von pflanzlichem Material von *Mycetia balansae* mit einem flüssigen Extraktionsmittel ausgewählt aus der Gruppe bestehend aus Wasser, Ethanol, Methanol, 1-Propanol, 2-Propanol, Glycerin, 1,2-Propandiol, Essigsäureethylester und deren Mischungen, vorzugsweise bei einer Temperatur im Bereich von 0°C bis zur Siedetemperatur des eingesetzten Extraktionsmittel bzw. Extraktionsmittelgemisches.

Das oder die Extraktionsmittel bzw. Extraktionsmittelgemische können vorzugsweise jeweils einzeln oder in binären oder ternären Mischungen oder auch konsekutiv in einer auf- oder absteigenden Polaritätsabfolge eingesetzt werden.

Bevorzugt umfasst ein erfindungsgemäßes Extraktionsverfahren zur Herstellung eines erfindungsgemäßen pflanzlichen Extraktes einen Extraktionsschritt mit einem weniger polaren, bevorzugt unpolaren Extraktionsmittel, der vor dem Schritt a) durchgeführt wird. Durch diese Art werden in einem solchen, dem Schritt a) vorgeschalteten, Vorbehandlungsschritt zunächst unerwünschte Begleitstoffe wie Fette und Wachse aus dem Pflanzenmaterial extrahiert und abgetrennt, so dass ein auf diese Weise erhaltener erfindungsgemäßer pflanzlicher Extrakt leichter weiterzuverarbeiten ist.

Es hat sich ferner in eigenen Untersuchungen überraschenderweise gezeigt, dass der gemäß dieser Vorgehensweise der konsekutiven Extraktion in einem nach Schritt a) vorliegenden Extrakt eine Vielzahl von Begleitsubstanzen und Nebenkomponenten mit störenden Geschmacksnoten, wie beispielsweise merklich bittere Noten, nicht bzw. nicht mehr vorhanden sind. In einer bevorzugten Ausgestaltung werden auf diese Weise sämtliche Substanzen mit störenden Geschmacksnoten entfernt bzw. vermieden.

Das Pflanzenmaterial kann vor der Extraktion gemäß Schritt a) bereits einer Extraktion durch ein weniger polares Mittel unterworfen werden. Besonders bevorzugt für eine solche Vorbehandlung des pflanzlichen Materials sind die weniger polaren Extraktionsmittel wie z.B. Butan, Propan, Isobutan, tert-Butylmethylether, Dichlormethan, n-Heptan, n-Hexan oder deren Mischungen, wobei die Extraktion vorzugsweise bei einer Temperatur im Bereich von -80 °C bis zur jeweiligen Siedetemperatur des Extraktionsmittels bzw. Extraktionsmittelgemisches bei dem während des oder der Extraktionsschritte verwendeten Druck erfolgt. Der Druck während des oder der Extraktionsschritte zur Vorbehandlung des pflanzlichen Materials liegt vorzugsweise im Bereich von 0,1 bis 1000 bar, bevorzugt im Bereich von 0,5 bis 250 bar.

Bevorzugt wird die Extraktion zur Vorbehandlung des pflanzlichen Materials vor Durchführung des oder der Extraktionen gemäß Schritt a) bei einer Temperatur im Bereich von -80 bis 200°C durchgeführt.

Im Falle des Erreichen des kritischen Punkts des Extraktionsmittels bzw. Extraktionsmittelgemisches kann die Maximaltemperatur auch bis 200 °C gewählt werden, so z.B. bei der Extraktion mit überkritischem Kohlendioxid, Propan, Butan oder Isobutan, gegebenenfalls unter Zusatz von weiteren Extraktions- und/oder Lösungsmitteln. Die Extraktion insbesondere mit wasserhaltigen Gemischen kann auch in Gegenwart von pHregulierenden Säuren, Basen oder Puffergemischen erfolgen.

Bevorzugt ist ein konsekutives Extraktionsverfahren mit zwei, drei, vier oder mehrerer hintereinander erfolgender Extraktionen, wobei vorzugsweise mit jeder folgenden Extraktion die verwendeten Extraktionsmittel zunehmend polarer werden. Vorzugsweise wird dabei mit einem unpolaren Extraktionsmittel begonnen und die zu extrahierenden Pflanzenteile einer Extraktion mit einem unpolaren Extraktionsmittel unterworfen. Das unpolare Extraktionsmittel wird nach Beendigung der ersten Extraktion abgezogen und ein Primärextrakt dieses Extraktionsschrittes gewonnen. Anschließend wird wenigstens in einem weiteren Schritt die Extraktion der bereits dem ersten Extraktionsschritt unterworfenen Pflanzenteile wiederholt, wobei ein Extraktionsmittel höherer Polarität eingesetzt wird und ein entsprechender Primärextrakt aus dieser Extraktionsstufe gewonnen wird. Die konsekutive Extraktion kann dabei mehrere Extraktionsschritte umfassen, wobei bevorzugt der letzte Extraktionsschritt eine Extraktion mit Wasser, Ethanol, Methanol, 1-Propanol, 2-Propanol, Glycerin, 1,2-Propandiol oder einem Gemisch der vorgenannten nicht-wässrigen Extraktionsmittel mit Wasser ist. Dabei ist es bevorzugt, wenn der in den erfindungsgemäßen oral konsumierbaren Zubereitungen eingesetzte erfindungsgemäße Extrakt aus einem Primärextrakt des letzten Extraktionsschrittes gewonnen wurde.

Der gemäß Schritt a) bzw. nach Schritt b) vorliegende bzw. erhältliche Extrakt kann optional noch weiter aufgeschlossen werden, z.B. durch enzymatische Behandlung (z.B. mit Cellulasen zum Aufschluss der Zellen), durch Behandlung mit Säure (z.B. unter Druck), durch Behandlung mit geeigneten basischen Lösungen z.B. von Hydroxiden, Carbonaten oder Hydrogencarbonaten von Natrium, Kalium, Calcium, Magnesium und Zink, mit sauren Ionentauschern oder mit Wasserdampf, vorzugsweise bei Drücken im Bereich von 0,01 mbar bis 100 bar, vorzugsweise im Bereich von 1 mbar bis 20 bar.

Der Extrakt aus Schritt a) kann optional in einem Schritt b) gegebenenfalls durch destillative oder andere evaporative oder pervaporative Verfahren aufkonzentriert werden, gegebenenfalls bis nur noch Feststoffe oder kaum bzw. nicht flüchtige Flüssigkeiten vorliegen.

Der aufkonzentrierte bzw. trockene Primärextrakt aus *Mycetia balansae* nach Schritt b) enthält vorzugsweise 0,001 bis 80 Gew.-%, bevorzugt 0,005 bis 50 Gew.-%, besonders bevorzugt 0,01 bis 25 Gew.-%, an Balansinen der Formel (I), jeweils bezogen auf die Trockenmasse des aufkonzentrierten Primärextraktes.

Optional kann der gegebenenfalls aufkonzentrierte Primärextrakt in einem Schritt c) durch Behandlung mit oder an Adsorbentien (Kieselgel, modifizierte Kieselgele (z.B. RP-Phasen), Aktivkohle, Zeolithe, Bentonit, Kieselgur, Alumina, basische oder saure oder neutrale [makroporöse] Ionentauscher) im Batch- oder Säulenverfahren, gegebenenfalls auch unter Zuhilfenahme weiterer Extraktionsmittel, bevorzugt einem oder mehreren Extraktionsmittel ausgewählt aus der Gruppe bestehend aus Wasser, n-Hexan, Dichlormethan, Ameisensäure, Methanol, Ethanol und 1,2-Propylenglycol, aufgereinigt werden, wodurch ein Sekundärextrakt erhalten wird.

Der aus *Mycetia balansae* gemäß Schritt c) resultierende Sekundärextrakt kann auch mit einem Anteil von 1 - 99 Gew.-%, bezogen auf den getrockneten Sekundärextrakt, an zum Verzehr geeigneten Hilfs- und Trägerstoffen (z.B. Maltodextrin, Stärke, natürliche oder künstliche Polysaccharide und/oder Pflanzengummen wie modifizierte Stärken oder Gummi Arabicum) versetzt werden, um die Herstellung eines getrockenten Sekundärextraktes gemäß Schritt d) zu optimieren.

Bevorzugte Hilfs- oder Trägerstoffe in Schritt d) sind Maltodextrin, Stärke, natürliche oder künstliche Polysaccharide und/oder Pflanzengummen wie modifizierte Stärken oder Gummi Arabicum, wobei gegebenenfalls zusätzlich ein oder mehrere weitere zum Verzehr geeignete Stoffe eingesetzt werden können, vorzugsweise ausgewählt aus der Gruppe bestehend aus:
(i) Verdünnungssmitteln wie Ethanol, 1,2-Propylenglycol, Glycerin, Diacetin, Triacetin und/oder Pflanzenöltriglyceride,
(ii) färbenden Mitteln, z.B. zugelassene Lebensmittelfarbstoffe und färbende Pflanzenextrakte,
(iii) Stabilisitoren, Konservierungsstoffen, Antioxidantien und viskositätsbeeinflussenden Stoffen.

Der aufkonzentrierte bzw. trockene Sekundärextrakt aus *Mycetia balansae* nach Schritt d) enthält vorzugsweise eine Gesamtmenge an Verbindungen der Formel (I) im Bereich von 0,001 bis 99 Gew.-%, bevorzugt im Bereich von 1 bis 99 Gew.-%, weiter bevorzugt im Bereich von 5 bis 95 Gew.-%, besonders bevorzugt 10 bis 90 Gew.-%, jeweils bezogen auf die Trockenmasse des aufkonzentrierten Sekundärextraktes.

Die erfindungsgemäßen pflanzlichen Extrakte werden, vorzugsweise in Schritt e), bevorzugt mit einem Verdünnungsmittel oder Verdünnungsmittelgemisch gemischt, vorzugsweise ausgewählt aus der Gruppe bestehend aus Wasser, Propylenglycol, Glycerin und Ethanol und deren Gemischen, und eine Lösung hergestellt, wobei das vollständige Lösen der Verbindungen der Balansine durch leichtes Erwärmen erleichtert bzw. beschleunigt werden kann.

Die Gesamtmenge eines erfindungsgemäßen pflanzlichen Extraktes in einer solchen Lösung liegt vorzugsweise im Bereich von 1 - 20 Gew.-%, bevorzugt im Bereich von 2 - 10 Gew.-%, besonders bevorzugt im Bereich von 4 - 6 Gew.-%.

Eine solche Lösung wird vorzugsweise mit einem Verdünnungsmittelgemisch der vorgenannten Verdünnungsmittel hergestellt, vorzugsweise ausgewählt aus der Gruppe bestehend aus Wasser-Propylenglycol, Wasser-Glycerin, Wasser-Ethanol, Glycerin-Ethanol, Glycerin-Propylenglycol und Propylenglycol-Ethanol.

Die in Schritt e) eines erfindungsgemäßen Extraktionsverfahrens eingesetzten Verdünnungsmittel oder Verdünnungsmittelgemische sind vorzugsweise zum Verzehr geeignet, da in einem solchen Falle die nach Schritt e) vorliegenden erfindungsgemäßen Extrakte, insbesondere die als Lösung vorliegenden Extrakte, zum unmittelbaren Einsatz in oral konsumierbare Zubereitungen besonders geeignet sind, wobei bevorzugte oral konsumierbare Zubereitungen ausgewählt sind aus der Gruppe bestehend aus der Ernährung dienenden Zubereitungen, der Nahrungsergänzung dienenden Zubereitungen, dem Genuss dienenden Zubereitungen, oralen pharmazeutischen Zubereitungen, Mundpflegeprodukten (Mundhygieneprodukten) und Aromakompositionen.

Die Erfindung betrifft ferner eine oral konsumierbare Zubereitung, umfassend eine sensorisch wirksame Menge einer erfindungsgemäßen Verbindung wie oben definiert bzw. eines erfindungsgemäßen physiologisch akzeptablen Salzes einer Verbindung wie oben definiert, eines erfindungsgemäßen Gemisches wie oben definiert, und/oder eines erfindungsgemäßen Extraktes wie oben definiert, wobei die oral konsumierbare Zubereitung vorzugsweise ausgewählt ist aus der Gruppe bestehend aus der Ernährung dienenden Zubereitungen, der Nahrungsergänzung dienenden Zubereitungen, dem Genuss dienenden Zubereitungen, den oralen pharmazeutischen Zubereitungen, den Mundpflegeprodukten, den Aromakompositionen und den kosmetischen Zubereitungen.

Zur Bestimmung des Süßeindruckes der erfindungsgemäßen Extrakte oder der erfindungsgemäßen Verbindungen der Formel (I) im Vergleich mit einer wässrigen Sucroselösung wird eine wässrige Lösung einer oder mehrerer Verbindungen der Formel (I) in gleicher Konzentration hergestellt, wie sie in der zu untersuchenden oral konsumierbaren Zubereitung vorliegt. Diese wässrige Lösung wird dann von einer Gruppe von wenigstens fünf Personen geschmacklich gegen eine Vergleichsreihe verschiedener Saccharosekonzentrationen in Wasser verglichen und eingeordnet. Auf diese Art wird die Saccharoseäquivalenz bestimmt (angegeben in Saccharose (=Sucrose) - Äquivalenten), also die Konzentration der Saccharoselösung, deren Süßeindruck äquivalent dem Süßeindruck der Konzentration der Verbindungen der Formel (I) ist.

Bevorzugte Konzentrationen der Saccharosevergleichslösung sind 0; 0,25; 0,5; 0,75; 1; 1,5; 2; 3; 4 und 5 Gew.-% Saccharose in Wasser. Gegebenenfalls können auch andere Konzentrationen, insbesondere höhere Konzentrationen für die Vergleichsreihe eingesetzt werden. Die ermittelte Saccharoseäquivalenz ergibt sich aus dem Mittelwert der Einzeleinstufung der einzelnen Panelisten. Bevorzugt sind die Panelisten Personen, die im Bereich des Abschmeckens (Verkostens) über Erfahrung verfügen, verschiedenen Alters und Geschlechts sowie verschiedener Herkunft.

Überraschenderweise wurde gefunden, dass die erfindungsgemäßen Verbindungen der Formel (I) bzw. die erfindungsgemäßen Extrakte, vorzugsweise die erfindungsgemäßen Extrakte von *Mycetia balansae,* bereits in sehr geringen Konzentrationen den süßen Geschmack bzw. die süße Geschmacksqualität von süß schmeckenden Stoffen in erfindungsgemäßen oral konsumierbaren Zubereitungen (vorzugsweise der Ernährung, der Mundpflege oder dem Genuss dienenden oder kosmetischen Zubereitungen zur Applikation im Bereich des Kopfes) erheblich verstärken können.

Die süßverstärkende Wirkung eines erfindungsgemäßen Extraktes, vorzugsweise eines erfindungsgemäßen Extraktes von *Mycetia balansae,* wird bereits bei einer Menge an erfindungsgemäßem Extrakt von kleiner oder gleich 1 Gew.-% (d.h. ≤ 1 Gew.-%), bevorzugt ≤ 0,5 Gew.-%, besonders bevorzugt ≤ 0,1 Gew.-% beobachtet, jeweils bezogen auf das Gesamtgewicht einer erfindungsgemäßen oral konsumierbaren Zubereitung.

Die süßverstärkende Wirkung der erfindungsgemäßen Verbindungen der Formel (I), vorzugsweise als Bestandteil des erfindungsgemäßen Extraktes, vorzugsweise eines erfindungsgemäßen Extraktes von *Mycetia balansae,* wird bereits bei einer Gesamtmenge an erfindungsgemäßem Verbindungen der Formel (I) von kleiner 0,01 Gew.-% (entsprechend 100 ppm) (ppm = Gewichtsteile pro Million), bevorzugt von kleiner 0,0050 Gew.-% (entsprechend 50 ppm) beobachtet, jeweils bezogen auf das Gesamtgewicht einer erfindungsgemäßen oral konsumierbaren Zubereitung.

Wie oben bereits ausgeführt, sind erfindungsgemäße Gemische, erfindungsgemäße pflanzliche Extrakte und erfindungsgemäße oral konsumierbare Zubereitungen bevorzugt, die eine oder mehrere der oben genannten als bevorzugt bzw. besonders bevorzugt gekennzeichneten Verbindungen der Formeln (I), (II) bzw. (III) oder deren Salze enthalten. Insbesondere bevorzugt sind erfindungsgemäße Gemische, erfindungsgemäße pflanzliche Extrakte und erfindungsgemäße oral konsumierbare Zubereitungen, die Balansin A und/oder Balansin B enthalten.

Die vorliegende Erfindung betrifft auch eine Verfahren zum (a) Vermitteln eines süßen Geschmackseindrucks und/oder Verstärken eines süßen Geschmackseindrucks eines, zweier oder mehrerer süß schmeckender Stoffe und/oder (b) Herstellen einer erfindungsgemäßen oral konsumierbaren Zubereitung, vorzugsweise in einer der als bevorzugt gekennzeichneten Ausgestaltungen, mit folgenden Schritten:
a) Bereitstellen einer erfindungsgemäßen Verbindung, vorzugsweise in einer der als bevorzugt gekennzeichneten Ausgestaltungen, bzw. eines physiologisch akzeptablen Salzes einer erfindungsgemäßen Verbindung, vorzugsweise in einer der als bevorzugt gekennzeichneten Ausgestaltungen, eines erfindungsgemäßen Gemisches, vorzugsweise in einer der als bevorzugt gekennzeichneten Ausgestaltungen, und/oder eines erfindungsgemäßen Extraktes, vorzugsweise in einer der als bevorzugt gekennzeichneten Ausgestaltungen,
b) Bereitstellen einer oral konsumierbaren Zubereitung, vorzugsweise umfassend einen, zwei oder mehrere weitere süß schmeckende Stoffe,
c) in Kontakt bringen oder Vermischen der in Schritt a) und b) bereitgestellten Bestandteile.

Ein erfindungsgemäß bevorzugtes Verfahren umfasst die Schritte
a-i) Herstellen eines erfindungsgemäßen Extraktes, vorzugsweise in einer der als bevorzugt gekennzeichneten Ausgestaltungen,
   umfassend ein erfindungsgemäße Verbindung, vorzugsweise in einer der als bevorzugt gekennzeichneten Ausgestaltungen, bzw. eines physiologisch akzeptablen Salzes einer erfindungsgemäßen Verbindung, vorzugsweise in einer der als bevorzugt gekennzeichneten Ausgestaltungen, oder eines erfindungsgemäßen Gemisches, vorzugsweise in einer der als bevorzugt gekennzeichneten Ausgestaltungen,
   durch Extrahieren von pflanzlichem Material, vorzugsweise pflanzlichem Material von *Mycetia balansae;*
   a-ii) optional Weiterverarbeiten des in Schritt (a-i) hergestellten Extraktes zu einem weiterverarbeiteten Produkt umfassend eine erfindungsgemäße Verbindung, eines physiologisch akzeptablen Salzes einer erfindungsgemäßen Verbindung, oder eines erfindungsgemäßen Gemisches,
b) Bereitstellen einer oral konsumierbaren Zubereitung, vorzugsweise umfassend einen, zwei oder mehrere weitere süß schmeckende Stoffe,
c) in Kontakt bringen oder Vermischen der oral konsumierbaren Zubereitung aus Schritt b), welche vorzugsweise einen, zwei oder mehrere weitere süß schmeckende Stoffe umfasst, mit dem in Schritt a-i) hergestellten Extrakt bzw. dem in Schritt a-ii) hergestellten weiterverarbeiteten Produkt.

Bevorzugt sind erfindungsgemäße oral zu konsumierende Zubereitungen, umfassend eine oder mehrere der Verbindungen der Formel (I), (II) oder (III) und/oder ein oder mehrere physiologisch akzeptable Salze einer erfindungsgemäßen Verbindung der Formel (I), (II) oder (III), in einer Gesamtmenge, die ausreicht, um in der oral zu konsumierenden Zubereitungen eine Süßwahrnehmung zu erzeugen, die mindestens derjenigen einer Vergleichszubereitung entspricht, welche aus einer 2 Gew.-%igen Lösung von Saccharose in Wasser besteht.

Ein weiterer Aspekt der Erfindung betrifft oral zu konsumierende (oral konsumierbare) Zubereitungen, enthaltend eine oder mehrere der Verbindungen der Formel (I) bzw. deren physiologisch akzeptablen Salze bzw. Gemische davon in Kombination mit mindestens einem weiteren süß schmeckenden Stoff.

Bevorzugt sind erfindungsgemäße oral zu konsumierende Zubereitungen, umfassend eine oder mehrere der Verbindungen der Formel (I), (II) oder (III) bzw. ein oder mehrere physiologisch akzeptable Salze einer erfindungsgemäßen Verbindung der Formel (I), (II) oder (III), sowie mindestens einen weiteren süß schmeckenden Stoff, wobei dieser in einer Konzentration vorliegt, die ausreicht, um in der oral zu konsumierenden Zubereitungen eine Süßwahrnehmung zu erzeugen, die mindestens derjenigen einer Vergleichszubereitung entspricht, welche aus einer 2 Gew.-%igen Lösung von Saccharose in Wasser besteht.

Bevorzugt erfolgt dabei die Verstärkung des Süßeindruckes synergistisch.

Gegenstand der vorliegenden Erfindung ist auch ein Verfahren zum Verstärken des beim Verkosten (Abschmecken) entstehenden Süßeindruckes einer oral konsumierbaren Zubereitung, umfassend die Schritte:
a) Bereitstellen einer oral konsumierbaren Zubereitung, umfassend einen oder mehrere weitere süß schmeckende Stoffe die gemeinsam einen Süßeindruck vermittelt, der gleich dem oder stärker als der einer wässrigen Sucroselösung mit einer Konzentration von 2 Gew.-% Sucrose ist,
b) Bereitstellen
   - einer oder mehrerer erfindungsgemäßer Verbindungen der Formel (I), oder
   - eines erfindungsgemäßen Extraktes, vorzugsweise aus *Mycetia balansae,* umfassend ein oder mehrere Verbindungen der Formel (I), dabei vorzugsweise 0,00001 bis 99 Gew.-%, bevorzugt 0,0001 bis 95 Gew.-%, besonders bevorzugt 0,0005 bis 80 Gew.-%, insbesondere bevorzugt 0,001 bis 30 Gew.-%, bezogen auf die Trockenmasse des Extraktes,
c) Mischen der in Schritt a) und b) bereitgestellten Bestandteile.

Weitere süß schmeckende Stoffe im Sinne der Erfindung können natürlich vorkommende, süß schmeckende Stoffe oder Pflanzenextrakte oder aber auch synthetische süß schmeckende Stoffe sein.

Natürlich vorkommende süß schmeckende Stoffe (inklusive Pflanzenextrakte) können beispielsweise sein süß schmeckende Kohlenhydrate (z.B. Saccharose, Trehalose, Lactose, Maltose, Melizitose, Melibiose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glyceraldehyd, Maltodextrine), Zuckeralkohole (z.B. Erythritol, Threitol, Arabitol, Ribitol, Xylitol, Sorbitol, Mannitol, Maltitol, Isomaltit, Dulcitol, Lactitol), Proteine (z.B. Miraculin, Pentaidin, Monellin, Thaumatin, Curculin, Brazzein, Mabinlin), D-Aminosäuren (z.B. D-Phenylalanin, D-Tryptophan) oder aus natürlichen Quellen gewonnenen Extrakte oder Fraktionen, enthaltend diese Aminosäuren und/oder Proteine, Neohesperidindihydrochalkon, Naringindihydrochalkon, Steviolgylcoside, Stevioside, Steviolbiosid, Rebaudioside, Rebaudiosid A, Rebaudiosid B, Rebaudiosid C, Rebaudiosid D, Rebaudiosid E, Rebaudiosid F, Rebaudiosid G, Rebaudiosid H, Dulcoside, Rubusosid, Suavioside A, Suavioside B, Suavioside G, Suavioside H, Suavioside I, Suavioside J, Baiyunoside 1 Baiyunoside 2, Phlomisoside 1, Phlomisoside 2, Phlomisoside 3, sowie Phlomisoside 4, Abrusoside A, Abrusoside B, Abrusoside C, Abrusoside D, Cyclocaryoside A und Cyclocaryoside I, Oslandin, Polypodosid A, Strogin 1, Strogin, 2 Strogin 4, Selligueanin A, Dihydroquercetin-3-acetat, Perillartin, Telosmosid A₁₅, Periandrin I-V, Pterocaryoside, Cyclocaryoside, Mukurozioside, trans-Anethol, trans-Cinnamaldehyd, Bryoside, Bryonoside, Bryonodulcoside, Carnosifloside, Scandenoside, Gypenoside, Trilobatin, Phloridzin, Dihydroflavanole, Hematoxylin, Cyanin, Chlorogensäure, Albiziasaponin, Telosmoside, Gaudichaudiosid, Mogroside, Mogrosid V, Hernandulcine, Monatin, Glycyrrhetinsäure und deren Derivate insbesondere Glycyrrhizin (bevorzugt als Ammoniumsalz), und Phyllodulcin, wobei im Falle der natürlich vorkommenden Süßstoffe auch Extrakte oder angereicherte Fraktionen dieser Extrakte verwendet werden können, z.B. Thaumatococcus-Extrakte (Katemfestaude), Extrakte aus *Stevia* ssp. (insbesondere *Stevia rebaudiana*), Swingle-Extrakt (*Momordica* bzw. *Siratia grosvenorii,* Luo-Han-Guo), Extrakte aus *Glycerrhyzia* ssp. (insb. *Glycerrhyzia glabra*), *Rubus* ssp. (insbesondere *Rubus suavissimus*), Citrus-Extrakte, Extrakte aus *Lippia dulcis.*

Erfindungsgemäß bevorzugt ist eine oral konsumierbare Zubereitung, die zusätzlich ein, zwei, drei, vier, fünf, sechs, sieben, acht, neun, zehn oder mehrere weitere Stoffe umfasst, ausgewählt aus den folgenden Gruppen (a1) bis (a5):
(a1) Aromastoffe, wobei vorzugsweise ein, zwei, drei, vier, fünf oder mehrere der Aromastoffe ausgewählt sind aus der Gruppe bestehend aus: Vanillin, Ethylvanillin, 2-Hydrox-4-methoxybenzaldehyd, Ethylvanillinisobutyrat (= 3-Ethoxy-4-isobutyryloxybenzaldehyd), Furaneol^{®} (2,5-Dimethyl-4-hydroxy-3(2H)-furanon) und Abkömmlinge (z.B. Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und dessen Abkömmlinge (z.B. Ethylmaltol), Cumarin und dessen Abkömmlinge, gamma-Lactone (z.B. gamma-Undecalacton, gamma-Nonalacton), delta-Lactone (z.B. 4-Methyldeltalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Fruchtester und Fruchtlactone (z.B. Essigsäure-n-butylester, Essigsäureisoamylester, Propionsäureethylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat), 4-(p-Hydroxyphenyl)-2-butanon, 1,1-Dimethoxy-2,2,5-trimethyl-4-hexan, 2,6-Dimethyl-5-hepten-1-al und Phenylacetaldehyd;
(a2) Kohlenhydrate ausgewählt aus der Gruppe bestehend aus Saccharose, Trehalose, Lactose, Maltose, Melizitose, Melibiose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glyceraldehyd, Maltodextrine und pflanzliche Zubereitungen enthaltend einen oder mehrere der genannten Kohlenhydrate, vorzugsweise in einem Anteil von zumindest 5 Gew. %, bevorzugt zumindest 15 Gew.-%, wobei die Kohlenhydrate auch als natürlich vorkommende oder künstlich hergestellte Mischung vorliegen können, dabei insbesondere als Honig, Invertzuckersirup oder hochangereicherter Fructose-Sirup aus Maisstärke, und den physiologisch akzeptablen Salzen dieser Kohlenhydrate, insbesondere den Natrium-, Kalium-, Calcium oder Ammoniumsalzen;
(a3) Zuckeralkohole, vorzugsweise natürlich vorkommende Zuckeralkohole ausgewählt aus der Gruppe bestehend aus Glycerin, Erythritol, Threitol, Arabitol, Ribitol, Xylitol, Sorbitol, Mannitol, Maltitol, Isomaltit, Dulcitol, Lactitol, und den physiologisch akzeptablen Salzen dieser Zuckeralkohole, insbesondere den Natrium-, Kalium-, Calcium oder Ammoniumsalzen;
(a4) natürlich vorkommende Süßstoffe, vorzugsweise ausgewählt aus der Gruppe bestehend aus
(a4-1)Miraculin, Monellin, Mabinlin, Thaumatin, Curculin, Brazzein, Pentaidin, D-Phenylalanin, D-Tryptophan, und aus natürlichen Quellen gewonnene Extrakte oder Fraktionen, enthaltend diese Aminosäuren und/oder Proteine, und den physiologisch akzeptablen Salzen dieser Aminosäuren und/oder Proteine, insbesondere den Natrium-, Kalium-, Calcium oder Ammoniumsalzen;
(a4-2) Neohesperidindihydrochalkon, Naringindihydrochalkon, Steviosid, Steviolbiosid, Rebaudiosiden, insbesondere Rebaudiosid A, Rebaudiosid B, Rebaudiosid C, Rebaudiosid D, Rebaudiosid E, Rebaudiosid F, Rebaudiosid G, Rebaudiosid H, Dulcosiden und Rubusosid, Suavioside A, Suavioside B, Suavioside G, Suavioside H, Suavioside I, Suavioside J, Baiyunoside 1 Baiyunoside 2, Phlomisoside 1, Phlomisoside 2, Phlomisoside 3, sowie Phlomisoside 4, Abrusoside A, Abrusoside B, Abrusoside C, Abrusoside D, Cyclocaryoside A und Cyclocaryoside I, Oslandin, Polypodosid A, Strogin 1, Strogin 2, Strogin 4, Selligueanin A, Dihydroquercetin-3-acetat, Perillartin, Telosmosid A₁₅, Periandrin I-V, Pterocaryosiden, Cyclocaryosiden, Mukuroziosiden, trans-Anethol, trans-Cinnamaldehyd, Bryosiden, Bryonosiden, Bryonodulcosiden, Carnosiflosiden, Scandenosiden, Gypenosiden, Trilobatin, Phloridzin, Dihydroflavanolen, Hematoxylin, Cyanin, Chlorogensäure, Albiziasaponin, Telosmosiden, Gaudichaudiosid, Mogrosiden, Mogrosid V, Hernandulcinen, Monatin, Phyllodulcin, Glycyrrhetinsäure und deren Derivaten, insbesondere deren Glycosiden wie Glycyrrhizin, und den physiologisch akzeptablen Salzen dieser Verbindungen, insbesondere den Natrium-, Kalium-, Calcium oder Ammoniumsalzen;
(a4-3) Extrakte oder angereicherte Fraktionen der Extrakte, ausgewählt aus der Gruppe bestehend aus Thaumatococcus-Extrakten (Katemfestaude), Extrakten aus *Stevia* ssp. (insbesondere *Stevia rebaudiana*), Swingle-Extrakten (*Momordica* bzw. *Siratia grosvenorii,* Luo-Han-Guo), Extrakten aus *Glycerrhyzia* ssp. (insbesondere *Glycerrhyzia glabra*), Extrakten aus *Rubus* ssp. (insbesondere *Rubus suavissimus),* Citrus-Extrakten und Extrakten aus *Lippia dulcis;*
(a5) synthetische süß schmeckende Stoffe, vorzugsweise ausgewählt aus der Gruppe bestehend aus Magap, Natriumcyclamat oder anderen physiologisch akzeptablen Salzen der Cyclamsäure, Acesulfam K oder anderen physiologisch akzeptablen Salzen, Neohesperidindihydrochalkon, Naringindihydrochalkon, Saccharin, Saccharin-Natriumsalz, Aspartam, Superaspartam, Neotam, Alitam, Advantam, Perillartin, Sucralose, Lugduname, Carrelame, Sucrononate und Sucrooctate.

Die Aromastoffe der obigen Gruppe (a1) verursachen oder verstärken einen süßen Geruchs- und/oder Geschmackseindruck und können als Geschmackskorrigentien eingesetzt werden.

Es hat sich ferner in eigenen Untersuchungen herausgestellt, dass sich der süße Geschmackseindruck der vorstehend genannten süß schmeckenden Stoffe durch die erfindungsgemäß einzusetzenden Extrakte aus *Mycetia balansae* besonders gut verstärken lässt.

In einer bevorzugten Ausgestaltung liegt die Gesamtmenge an Verbindungen der Formel (I) und die Gesamtmenge an süß schmeckenden Stoffe der oben definierten Gruppen (a4) und (a5) im Bereich von 0,001 bis 1 Gew.-%, bevorzugt im Bereich von 0,001 bis 0,5 Gew.-%, weiter bevorzugt im Bereich von 0,003 bis 0,1 Gew.-%, bezogen auf die Gesamtmasse der direkt zum Verzehr vorgesehenen oral konsumierbaren Zubereitung. Eine bevorzugte erfindungsgemäße oral konsumierbare Zubereitung umfasst neben einer erfindungsgemäßen Verbindung bzw. deren physiologisch akzeptablem Salz und/oder deren Gemisch zusätzlich einen oder mehrere weitere Stoffe zum Verstärken eines süßen Geschmackseindrucks.

Weitere Stoffe zum Verstärken des süßen Geschmackseindrucks sind - ohne die vorliegende Erfindung darauf zu beschränken - vorzugsweise ausgewählt aus der Gruppe bestehend aus Hydroxydeoxybenzoinen, wie zum Beispiel 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon) (insbesondere solche wie beschrieben in WO 2006/106023, die bezüglich der darin offenbarten entsprechenden Verbindungen auf dem Wege der Verweisung Bestandteil dieser Anmeldung wird); Hydroxyphenylalkandionen, wie zum Beispiel Isogingerdion-[2] (insbesondere solche wie beschrieben in WO 2007/003527, die bezüglich der darin offenbarten entsprechenden Verbindungen auf dem Wege der Verweisung Bestandteil dieser Anmeldung wird); Diacetyltrimeren (insbesondere solche wie beschrieben in WO 2006/058893, die bezüglich der darin offenbarten entsprechenden Verbindungen auf dem Wege der Verweisung Bestandteil dieser Anmeldung wird); Divanilline (insbesondere solche wie beschrieben in WO 2004/078302, die bezüglich der darin offenbarten entsprechenden Verbindungen auf dem Wege der Verweisung Bestandteil dieser Anmeldung wird), Hesperetin wie in der WO 2007/014879 offenbart, die hinsichtlich dieser Verbindungen auf dem Wege der Verweisung Bestandteil dieser Anmeldung wird, 4-Hydroxydihydrochalkonen, dabei insbesondere Phloretin, wie in der EP 1 998 636 B1 offenbart, die hinsichtlich dieser Verbindungen auf dem Wege der Verweisung Bestandteil dieser Anmeldung wird, oder Propenylphenylglycosiden (Chavicolglycosiden) wie in EP 1 955 601 A1 beschrieben, die hinsichtlich dieser Verbindungen auf dem Wege der Verweisung Bestandteil dieser Anmeldung wird, Hydroxyflavane wie in EP 2 253 226 A1 offenbart, die hinsichtlich dieser Verbindungen auf dem Wege der Verweisung Bestandteil dieser Anmeldung wird, bestimmte Extrakte aus *Hydrangea macrophylla* wie in EP 2 298 084 A1 offenbart, die hinsichtlich dieser Verbindungen auf dem Wege der Verweisung Bestandteil dieser Anmeldung wird, 1-(2,4-Dihydroxyphenyl)-3-(3-hydroxy-4-methoxyphenyl)-propan-1-on wie in der europäischen Patentanmeldung mit der Anmeldenummer 10152331.4 offenbart (Symrise), die hinsichtlich dieser Verbindung auf dem Wege der Verweisung Bestandteil dieser Anmeldung wird.

Dabei sind bevorzugte süßen Geschmack verstärkende Stoffe insbesondere Hesperetin, Phloretin, 3',7-Dihydroxy-4'-methoxyflavan und (S)-3',7-Dihydroxy-4'-methoxyflavan, bestimmte Hydrangea-Extrakte oder Phyllodulcin, sowie 1-(2,4-Dihydroxyphenyl)-3-(3-hydroxy-4-methoxyphenyl)-propan-1-on.

Mit den Stoffen der letztgenannten Gruppen lassen sich nochmals verstärkende Effekte hinsichtlich des Süßeindruckes der erfindungsgemäßen Zubereitungen erzielen.

Bevorzugt ist ferner eine erfindungsgemäße Zubereitung, umfassend einen oder mehrere weitere aromatisierende Pflanzenextrakte, Aroma-, Hilfs- oder Trägerstoffe.

Selbstverständlich umfassen bevorzugte erfindungsgemäße Zubereitungen auch weitere übliche Lebensmittelbestandteile.

In erfindungsgemäß bevorzugten oral konsumierbaren Zubereitungen liegt die Gesamtmenge an Verbindungen der Formel (I) im Bereich von 0,01 ppm bis 95 Gew.-%, vorzugsweise im Bereich von 0,1 ppm bis 90 Gew.-%, bevorzugt im Bereich von 1 ppm bis 50 Gew.-%, weiter bevorzugt im Bereich von 1 ppm bis 20 Gew.-%, insbesondere bevorzugt im Bereich von 1 ppm bis 5 Gew.-%, bezogen auf die Gesamtmasse der Zubereitung.

Ganz besonders bevorzugt ist eine erfindungsgemäße oral konsumierbare Zubereitung, insbesondere eine direkt zum Verzehr geeignete oral konsumierbare Zubereitung, wobei die Gesamtmenge der Verbindungen der Formel (I) im Bereich von 0,01 ppm bis 1000 ppm liegt, bevorzugt im Bereich von 0,1 ppm bis 500 ppm, besonders bevorzugt im Bereich von 1 bis 100 ppm, bezogen auf das Gesamtgewicht der Zubereitung.

In bereits sehr geringen Konzentrationen lässt sich - wie oben bereits angedeutet - die süßverstärkende Funktion der Verbindungen der Formel (I) ausnutzen, wobei die Verstärkung - wie ebenfalls oben angedeutet - synergistisch ist.

Bevorzugt ist eine erfindungsgemäße oral konsumierbare Zubereitung, wobei diese ausgewählt ist aus der Gruppe bestehend aus pharmazeutischer Zubereitung, der Mundpflege dienende Zubereitung, der Ernährung oder dem Genuss dienende flüssige und feste Lebensmittelzubereitung, aber auch kosmetische Zubereitungen zur Applikation im Bereich des Kopfes sein, die mit der Mundhöhle in Kontakt kommen können.

Die erfindungsgemäßen Zubereitungen können auch in Form einer Halbfertigware vorliegen, die normalerweise nicht direkt oral konsumiert wird, aber zur Herstellung einer erfindungsgemäßen direkt zum Verzehr vorgesehenen oral konsumierbaren Zubereitung dienen.

Erfindungsgemäße Halbfertigwaren werden dabei vorzugsweise in einer Konzentration im Bereich von 0,0001 bis 90 Gew.-%, bevorzugt im Bereich von 0,001 bis 50 Gew.-%, besonders bevorzugt im Bereich von 0,01 bis 20 Gew.-%, bezogen auf die Gesamtmasse der resultierenden oral konsumierbaren Zubereitung eingesetzt. Solche Halbfertigwaren können z.B. als Aromakompositionen vorliegen.

Aromakompositionen im Sinne der vorliegenden Erfindung enthalten neben einer oder mehreren Verbindungen der Formel (I), (II) bzw. (III) und/oder ein oder mehreren deren physiologisch akzeptablen Salze dieser Verbindungen zumindest (i) ein, zwei, drei, vier, fünf, sechs, sieben, acht, neun, zehn oder mehrere (weitere) Aromastoffe und/oder (ii) ein, zwei, drei, vier, fünf, sechs, sieben oder mehrere (weitere) Geschmackstoffe.

Wie bereits erwähnt ist nicht Gegenstand der vorliegenden Erfindung die frische oder getrocknete Pflanze *Mycetia balansae* als solche. Eine erfindungsgemäße oral konsumierbare Zubereitung hingegen kann neben einem oder mehreren getrockneten Pflanzenteilen von *Mycetia balansae,* bevorzugt Pflanzenteile in getrockeneter und zerkleinerter Form, zusätzlich ein oder mehrere weitere zum Verzehr geeignete Bestandteile enthalten, wobei die weiteren zum Verzehr geeigneten Bestandteile vorzugsweise nicht aus *Mycetia balansae* extrahierbar sind, und bevorzugt nicht in *Mycetia balansae* vorhanden sind.

Dabei besonders bevorzugt ist eine erfindungsgemäße oral konsumierbare Zubereitung, die neben einem oder mehreren getrockneten Pflanzenteilen von *Mycetia balansae,* zusätzlich ein, zwei, drei, vier, fünf oder mehrere (weitere) Aromastoffe enthält, vorzugsweise ausgewählt aus der oben definierten Gruppe (a1) und/oder der nachfolgende definierten Gruppe (A).

Wie oben bereits ausgeführt, betrifft ein Aspekt der vorliegenden Erfindung die Verwendung einer erfindungsgemäßen Verbindung der Formel (I), eines erfindungsgemäßen Gemisches, oder einer erfinundungsgemäßen oral konsumierbaren Zubereitung in Form einer Halbfertigware
- zur Erzielung eines süßen Geschmackseindrucks,
- zur Verstärkung eines süßen Geschmackseindrucks,
   und/oder
- als Geschmackskorrigenz.

Entsprechend betrifft die Erfindung auch die Verwendung einer erfindungsgemäßen Verbindung, vorzugsweise in einer der als bevorzugt gekennzeichneten Ausgestaltungen, bzw. eines physiologisch akzeptablen Salzes einer erfindungsgemäßen Verbindung, vorzugsweise in einer der als bevorzugt gekennzeichneten Ausgestaltungen, eines erfindungsgemäßen Gemisches, oder eines erfindungsgemäßen Extraktes, vorzugsweise in einer der als bevorzugt gekennzeichneten Ausgestaltungen,
zum Erzeugung eines Süßeindruckes in einer oral konsumierbaren Zubereitung oder zur Verstärkung des Süßeindruckes einer oral konsumierbaren Zubereitung umfassend mindestens einen weiteren, vorzugsweise natürlich vorkommenden, süß schmeckenden Stoff.

Oral konsumierbare Zubereitungen können dann bevorzugt süß schmeckende, der Ernährung, der Nahrungsergänzung, der Mundpflege oder dem Genuss dienende Zubereitungen, kosmetische Zubereitungen, vorzugsweise zur Applikation im Bereich des Kopfes, oder orale pharmazeutische Zubereitungen (d.h. zur oralen Aufnahme bestimmte pharmazeutische Zubereitungen) sein.

Aromakompositionen im Sinne der Erfindung können neben den erfindungsgemäß einzusetzenden Extrakten aus *Mycetia balansae* ein oder mehrere verschiedene natürliche oder nicht-natürliche Aromastoffe und/oder aromatisierende Lebensmittel, Reaktionsaromen, Aromazubereitungen, Geschmackstoffe, weitere geschmacksmodulierende Stoffe, Prekursoren, sonstige Aromastoffe, Zusatzstoffe, Süßungs-, Färbungs- und Säuerungsmitteln, Stabilisatoren sowie Lösungsmittel, Hilfs- und Trägerstoffe enthalten.

Im Rahmen der vorliegenden Erfindung bevorzugt einzusetzende (ein oder mehrere) Aromastoffe werden vorzugsweise gewählt aus der folgenden Gruppe (A) bestehend aus:
Acetophenon, Allylcapronat, alpha-Ionon, beta-Ionon, Anisaldehyd, Anisylacetat, Anisylformiat, Benzaldehyd, Benzothiazol, Benzylacetat, Benzylalkohol, Benzylbenzoat, beta-Ionon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion®), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al, Phenylacetaldehyd, 2-Methyl-3-(methylthio)furan, 2-Methyl-3-furanthiol, Bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

In einer bevorzugten Ausgestaltung liegt die Gesamtmenge einer erfindungsgemäßen Aromakomposition, vorzugsweise enthaltend ein, zwei, drei, vier, fünf oder mehrere der Aromastoffe aus der oben definierten Gruppe (a1) bzw. der Gruppe (A), im Bereich von 0,01 bis 1 Gew.-%, bevorzugt im Bereich von 0,01 bis 0,5 Gew.-%, weiter bevorzugt im Bereich von 0,01 bis 0,1 Gew.-%, bezogen auf die Gesamtmasse der direkt zum Verzehr vorgesehenen oral konsumierbaren Zubereitung.

Die der Ernährung oder dem Genuss dienenden flüssige und feste Lebensmittelzubereitung im Sinne der Erfindung sind z.B. Backwaren (z.B. Brot, Trockenkekse, Kuchen, sonstiges Gebäck), Süßwaren im engeren Sinne (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais-oder Erdnussbasis), Produkte auf Fett- und Ölbasis oder Emulsionen derselben (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Der Mundpflege dienende Zubereitungen (Mundhygieneprodukte) im Sinne der vorliegenden Erfindung sind insbesondere Mund- und/oder Zahnpflegemittel wie Zahnpasten, Zahngele, Zahnpulver, Mundwässer, Kaugummis und andere Mundpflegemittel.

Orale pharmazeutische Zubereitungen im Sinne der Erfindung sind Zubereitungen, die z.B. in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen und als verschreibungspflichtige, apothekenpflichtige oder sonstige Arzneimittel oder als Nahrungsergänzungsmittel verwendet werden.

Kosmetische Zubereitungen zur Applikation im Bereich des Kopfes sind insbesondere solche, die selbst bei sachgemäßer Auftragung auf die Haut mit der Mundhöhle in Kontakt treten können, also beispielsweise - wie bereits erwähnt - kosmetische Zubereitungen zur Applikation im Bereich des Kopfes wie Seifen, andere Reinigungs- oder Pflegemittel für den Gesichtsbereich, Gesichtscremes, -lotionen oder -salben, Sonnenschutzmittel, Bartreinigungs- oder -pflegemittel, Rasierschäume, -seifen oder -gele, Lippenstifte oder andere Lippenkosmetika oder Lippenpflegemittel.

Weitere übliche Wirk-, Grund-, Hilfs- und Zusatzstoffe für die erfindungsgemäßen oral konsumierbaren Zubereitungen können in Mengen von bis zu 99,999999 Gew.-%, vorzugsweise 10 bis 80 Gew.-%, bezogen auf das Gesamtgewicht der Zubereitung, enthalten sein. Die erfindungsgemäßen oral konsumierbaren Zubereitungen können dabei vorzugsweise Wasser in einer Menge bis zu 99,999999 Gew.-% enthalten, bevorzugt 1 bis 95 Gew.-%, bevorzugt 5 bis 80 Gew.-%, bezogen auf das Gesamtgewicht der Zubereitung.

Die erfindungsgemäßen Zubereitungen enthaltend Extrakte aus *Mycetia balansae,* werden gemäß einer bevorzugten Ausgestaltung hergestellt, indem die Extrakte aus *Mycetia balansae* bevorzugt als Aromakomposition in Form eines Gemischs mit einem festen oder flüssigen Trägerstoff in eine oral konsumierbare Basis-Zubereitung eingearbeitet werden. Vorteilhafterweise können als Lösung vorliegende erfindungsgemäße Zubereitungen auch durch Sprühtrocknung in eine feste Zubereitung überführt werden.

Gemäß einer weiteren bevorzugten Ausführungsform werden zur Herstellung erfindungsgemäßer oral konsumierbarer Zubereitungen die Extrakte aus *Mycetia balansae* oder diese enthaltende Aromakompositionen auch vorher in Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten, Cellulose oder Cellulosederivaten (z.B. Hydroxypropylcellulose), anderen Polysacchariden (z.B. Alginat), natürlichen Fetten, natürlichen Wachsen (z.B. Bienenwachs, Carnaubawachs) oder aus Proteinen, z.B. Gelatine, eingearbeitet.

In einem weiteren bevorzugten Herstellungsverfahren werden die die Extrakte aus *Mycetia balansae* oder diese enthaltende Aromakompositionen vorher mit einem oder mehreren geeigneten Komplexbildnern, beispielsweise mit Cyclodextrinen oder Cyclodextrinderivaten, bevorzugt α- oder β-Cyclodextrin, komplexiert und in dieser komplexierten Form eingesetzt.

Besonders bevorzugt ist eine erfindungsgemäße oral konsumierbare Zubereitung, bei der die Matrix so gewählt wird, dass die Extrakte aus *Mycetia balansae* bzw. die Balansine der Formel (I) verzögert von der Matrix freigegeben werden, so dass man eine langanhaltende Wirkung erhält.

Als weitere Bestandteile für erfindungsgemäße oral konsumierbare Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche, nicht süße Kohlenhydrate (z.B. Dextrine, Amylose, Amylopektin, Inulin, Xylane, Cellulose), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ -Aminobuttersäure, Taurin), Peptide (z.B. Glutahthion), native oder prozessierte Proteine (z.B. Gelatine), Enzyme (z.B. Peptidasen), Nukleinsäuren, Nucleotide, andere Geschmackskorrigentien für unangenehme Geschmackseindrücke, Geschmacksmodulatoren für weitere, in der Regel nicht unangenehme Geschmackseindrücke, weitere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), zusätzliche Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Zahnpflegemittel (als Basis für der Mundpflege dienende Zubereitungen) umfassen im allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Geschmackskorrigentien für unangenehme Geschmackseindrücke, Geschmackskorrigentien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für der Mundpflege dienende Zubereitungen), umfassen im allgemeinen eine Kaugummibase, d.h. eine beim Kauen plastisch werdende Kaumasse, andere Geschmackskorrigentien für unangenehme Geschmackseindrücke, Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, weitere Aromen und Stabilisatoren oder Geruchskorrigentien.

Als Bestandteile für erfindungsgemäße orale pharmazeutische Zubereitungen können alle üblicherweise weiteren Wirk-, Grund-, Hilfs- und Zusatzstoffe für orale pharmazeutische Zubereitungen verwendet werden. Als Wirkstoffe können insbesondere auch unangenehm schmeckende oral formulierbare pharmazeutische Wirkstoffe verwendet werden. Die Wirk-, Grund-, Hilfs- und Zusatzstoffe können in an sich bekannter Weise in die oralen Applikationsformen überführt werden. Dies geschieht regelmäßig unter Verwendung inerter, nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) und Geruchskorrigentien sowie nicht den bitteren Geschmack betreffende Geschmackskorrigentien.

Bevorzugt können die erfindungsgemäßen oral konsumierbare Zubereitungen auch eine weitere Aromakomposition enthalten, um den Geschmack und/oder Geruch der Zubereitung abzurunden und zu verfeinern. Geeignete Aromakompositionen enthalten z.B. synthetische, natürliche oder naturidentische Aroma-, Riech- und/oder Geschmacksstoffe sowie geeignete Hilfs- und Trägerstoffe. Als besonders vorteilhaft wird dabei angesehen, dass ein eventuell vorhandener bitterer oder metallischer Geschmackseindruck, der von in den erfindungsgemäßen Zubereitungen enthaltenen Aroma-, Riech- und/oder Geschmacksstoffen ausgeht, vermindert oder unterdrückt werden kann und damit das gesamte Aroma- oder Geschmacksprofil verbessert wird.

Erfindungsgemäße Halbfertigwaren können zur Verstärkung des süßen Geschmackseindrucks von oral konsumierbaren Fertigwaren (d.h. von zum direkten Verzehr vorgesehenen Zubereitungen) dienen, die unter Verwendung der erfindungsgemäßen Halbfertigware hergestellt werden.

Erfindungsgemäße Halbfertigwaren enthalten vorzugsweise die Verbindungen der Formel (I) oder eines erfindungsgemäßen pflanzlichen Extraktes, vorzugsweise aus *Mycetia balansae,* in einer Gesamtmenge im Bereich von 0,001 bis 99 Gew.-% bevorzugt im Bereich von 1 bis 95 Gew.-%, weiter bevorzugt im Bereich von 5 bis 95 Gew.-%, besonders bevorzugt im Bereich von 10 bis 95 Gew.-%, bezogen auf das Gesamtgewicht der Halbfertigware.

Erfindungsgemäß zu verwendende Zubereitungen, die als Halbfertigwaren vorliegen, können zur Erzielung und/oder Verstärkung des süßen Geschmackseindrucks von Fertigware-Zubereitungen dienen, die unter Verwendung der Halbfertigware-Zubereitung hergestellt werden.

In einer besonders bevorzugten Ausführung der Erfindung umfasst eine erfindungsgemäßen Zubereitung neben einer erfindungsgemäßen Verbindung der Formel (I), einem physiologisch akzeptablen Salz einer Verbindung der Formel (I), einem erfindungsgemäßen Gemisch oder einem erfindungsgemäßen Extrakt ein oder mehrere Geschmackskorrigentien, d.h. eine oder mehrere weitere Substanzen, die nicht der Formel (I) entsprechen, wobei das Geschmackskorrigenz geeignet ist bzw. die Geschmackskorrigentien geeignet sind zum
- Verändern oder Maskieren (Maskieren bedeutet dabei Vermindern oder vollständig Unterdrücken) des unangenehmen Geschmackseindrucks eines oder mehrerer unangenehm schmeckender Stoffe, oder
- Verstärken eines angenehmen Geschmackseindrucks, vorzugsweise eines weiteren Geschmackseindrucks zusätzlich zu einem süßen Geschmackseindruck, oder
- Verstärken eines angenehm schmeckenden Stoffes, vorzugsweise eines angenehm schmeckenden Stoffes,der zusätzlich zu einem süßen Geschmackseindruck einen weiteren angenehmen Geschmackseindruck vermittelt.

Unangenehme Geschmackseindrücke im Sinne der vorliegenden Erfindung sind dabei:
- bitter, adstringierend, sehr scharf, beißend, pappig, kalkig, staubig, trocken, mehlig, ranzig, stark sauer und/oder metallisch sowie
- ein entsprechender (gegebenenfalls stark anhaltenden) Nachgeschmack.

Angenehme Geschmackseindrücke (außer bzw. zusätzlich zu einem süßen Geschmackseindruck) im Sinne der vorliegenden Erfindung sind dabei:
- fettig, umami, würzig, leicht wärmend, angenehm kühlend, cremig, kokumi

Die weiteren Geschmackskorrigentien können bevorzugt aus der folgenden Liste ausgewählt werden: Nucleotide (z.B. Adenosin-5'-monophosphat, Cytidin-5'-monophosphat) oder deren pharmazeutisch akzeptablen Salze, Lactisole, Natriumsalze (z.B. Natriumchlorid, Natriumlactat, Natriumcitrat, Natriumacetat, Natriumgluconoat), Hydroxyflavanonen, wie zum Beispiel Eriodictyol, Sterubin (Eriodictyol-7-methylether), Homoeriodictyol, und deren Natrium-, Kalium-, Calcium-, Magnesium- oder Zinksalzen (insbesondere solche wie beschrieben in EP 1 258 200 A2, die bezüglich der darin offenbarten entsprechenden Verbindungen auf dem Wege der Verweisung Bestandteil dieser Anmeldung wird), Hydroxybenzoesäureamiden, wie zum Beispiel 2,4-Dihydroxybenzoesäurevanillylamid, 2,4-Dihydroxybenzoesäure-N-(4-hydroxy-3-methoxybenzyl)amid, 2,4,6-Trihydroxybenzoesäure-N-(4-hydroxy-3-methoxybenzyl)amid, 2-Hydroxy-benzoesäure-N-4-(hydroxy-3-methoxybenzyl)amid, 4-Hydroxybenzoesäure-N-(4-hydroxy-3-methoxybenzyl)amid, 2,4-Dihydroxybenzoesäure-N-(4-hydroxy-3-methoxybenzyl)amid-Mono-natriumsalz, 2,4-Dihydroxybenzoesäure-N-2-(4-hydroxy-3-methoxyphenyl)ethylamid, 2,4-Dihydroxybenzoesäure-N-(4-hydroxy-3-ethoxybenzyl)amid, 2,4-Dihydroxybenzoesäure-N-(3,4-dihydroxybenzyl)amid und 2-Hydroxy-5-methoxy-N-[2-(4-hydroxy-3-methoxyphenyl)ethyl]amid; 4-Hydroxybenzoesäurevanillylamid (insbesondere solche wie beschrieben in WO 2006/024587, die bezüglich der darin offenbarten entsprechenden Verbindungen auf dem Wege der Verweisung Bestandteil dieser Anmeldung wird); Hydroxydeoxybenzoinen, wie zum Beispiel 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon) (insbesondere solche wie beschrieben in WO 2006/106023, die bezüglich der darin offenbarten entsprechenden Verbindungen auf dem Wege der Verweisung Bestandteil dieser Anmeldung wird); Hydroxyphenlaalkandionen, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche wie beschrieben in WO 2007/003527, die bezüglich der darin offenbarten entsprechenden Verbindungen auf dem Wege der Verweisung Bestandteil dieser Anmeldung wird); Diacetyltrimeren (insbesondere solche wie beschrieben in WO 2006/058893, die bezüglich der darin offenbarten entsprechenden Verbindungen auf dem Wege der Verweisung Bestandteil dieser Anmeldung wird); γ-Aminobuttersäuren (insbesondere solche wie beschrieben in WO 2005/096841, die bezüglich der darin offenbarten entsprechenden Verbindungen auf dem Wege der Verweisung Bestandteil dieser Anmeldung wird); Divanillinen (insbesondere solche wie beschrieben in WO 2004/078302, die bezüglich der darin offenbarten entsprechenden Verbindungen auf dem Wege der Verweisung Bestandteil dieser Anmeldung wird) und 4-Hydroxydihydrochalconen, vorzugsweise wie beschrieben in US 2008/227867 A1, die bezüglich der darin offenbarten entsprechenden Verbindungen auf dem Wege der Verweisung Bestandteil dieser Anmeldung wird, dabei insbesondere Phloretin und Davidigenin, Aminosäuren oder Gemische von Molkeproteinen mit Lecithinen, Hesperetin wie in der WO 2007/014879 offenbart, die hinsichtlich dieser Verbindungen auf dem Wege der Verweisung Bestandteil dieser Anmeldung wird, 4-Hydroxydihydrochalkone wie in der WO 2007/107596 offenbart, die hinsichtlich dieser Verbindungen auf dem Wege der Verweisung Bestandteil dieser Anmeldung wird, oder Propenylphenylglycoside (Chavicolglycoside) wie in EP 1 955 601 A1 beschrieben, die hinsichtlich dieser Verbindungen auf dem Wege der Verweisung Bestandteil dieser Anmeldung wird, Pellitorin, insbesondere trans-Pellitorin, und davon abgeleitete Aromakompositionen wie in EP 2 008 530 A1 beschrieben, die hinsichtlich dieser Verbindungen und Aromakompositionen auf dem Wege der Verweisung Bestandteil dieser Anmeldung werden, bestimmte Extrakte aus *Rubus suavissimus* wie in US Provisional Application 61/333,435 (Symrise) und den darauf basierenden Patentanmeldungen (z.B. die europäische Patentanmeldung mit der Anmeldenummer 11165566.8 (Symrise)) beschrieben, die hinsichtlich dieser Extrakte auf dem Wege der Verweisung Bestandteil dieser Anmeldung werden, Umami - Verbindungen wie die in WO 2008/046895 A1 und EP 1 989 944 A1 beschrieben, die jeweils hinsichtlich dieser Verbindungen auf dem Wege der Verweisung Bestandteil dieser Anmeldung werden sowie Umami-Verbindungen wie beschrieben in EP 2 064 959 A1 bzw. EP 2 135 516 A1, die bezüglich der darin offenbarten entsprechenden Verbindungen auf dem Wege der Verweisung Bestandteil dieser Anmeldung werden, Phyllodulcin oder Extrakte aus *Hydrangea macrophylla* var. *thunbergii* makino und davon abgeleitete Aromakompositionen, wie beschrieben in EP 2 298 084 A1 bzw. US 2011/0076239 A1, die hinsichtlich dieser Extrakte, bzw. Phyllodulcin auf dem Wege der Verweisung Bestandteil dieser Anmeldung werden, Hydroxyflavan-Derivate und davon abgeleitete Aromakompositionen, wie beschrieben in US 2010/0292175 A1, die hinsichtlich dieser Verbindungen und der davon abgeleiteten Aromakompositionen auf dem Wege der Verweisung Bestandteil dieser Anmeldung werden.

Sofern eine erfindungsgemäße Zubereitung beispielsweise eine vergleichsweise hohe Konzentration einer erfindungsgemäßen Verbindung der Formel (I), eines physiologisch akzeptablen Salzes einer Verbindung der Formel (I), eines erfindungsgemäßen Gemisches oder eines erfindungsgemäßen Extraktes enthält, kann es vorkommen, dass neben den beschriebenen gewünschten sensorischen Effekten zusätzlich unerwünschte und sogar unangenehme Geschmackseindrücke auftreten, wie beispielsweise bittere Noten. Diese unerwünschten bzw. unangenehmen Geschmackseindrücke können durch Geschmackskorrigenzien zumindest teilweise reduziert oder sogar vollständig unterdrückt werden.

Daher betrifft die Erfindung in einer bevorzugten Ausgestaltung auch eine erfindungsgemäße Zubereitung, enthaltend
(a) eine erfindungsgemäße Verbindung der Formel (I), ein physiologisch akzeptables Salz einer Verbindung der Formel (I), ein erfindungsgemäßes Gemisch oder einen erfindungsgemäßen Extrakt, und
(b) eine oder mehrere (weitere) Substanzen zum Maskieren eines unangenehmen Geschmackseindrucks, insbesondere eines bitteren, stark sauren oder adstringierenden Geschmackeindrucks.

Vorzugsweise sind dabei eine oder mehrere Substanzen des Bestandteils (b) ausgewählt aus der Gruppe bestehend aus Natriumsalzen (dabei vorzugsweise Natriumchlorid, Natriumlactat, Natriumcitrat, Natriumacetat, Natriumgluconoat), Homoeriodictyol oder dessen Natriumsalzen, Eriodictyol, trans-Pellitorin und Rubus-Extrakten, vorzugsweise Rubus-Extrakten wie in der europäischen Patentanmeldung mit der Anmeldenummer 11165566.8 (Symrise) beschrieben.

### Beispiele

Die Beispiele dienen nur zur Verdeutlichung der Erfindung, ohne diese damit einzuschränken. Sofern nicht anders angegeben, beziehen sich alle Angaben auf das Gewicht.

### Beispiel 1: Mycetia balansae-Extrakt durch konsekutive Extraktion

Getrocknete und gemahlene oberirdische Pflanzenteile von *Mycetia balansae* (100 g) wurden konsekutiv zweimal mit je 1L Heptan, Methylenchlorid, tert-Butylmethylether, Ethylacetat und Ethanol/Wasser (4:1) (Volumenteile; v/v) in aufsteigender Polarität unter Rühren bei Raumtemperatur für je 1 h extrahiert. Konsekutiv heißt in diesem Zusammenhang, dass das Pflanzenmaterial in der genannten Reihenfolge mit dem jeweiligen Extraktionsmittel behandelt wurde, wobei das jeweilige Extraktionsmittel abgezogen wurde und das dann bereits extrahierte Pflanzenmaterial im nächsten Extraktionsschritt dem nächsten Extraktionsmittel unterworfen wurde. Das Extraktionsmittel wurde unter Vakuum entfernt und die erhaltenen getrockneten Extrakte in einer Dosierung von 500 ppm auf Zuckerlösung (5%) verkostet und sensorisch bewertet.

| **Extraktionsmittel** | **Ausbeute (Gew.-% bez. auf das Gesamtgewicht der Blätter)** | **Gehalt (Gew.-%) an Balansin A und Balansin B*** | **Geschmacksbeschreibung** |
|---|---|---|---|
| Heptan | 0,3 | nicht nachweisbar | krautig, bitter |
| Methylenchlorid | 0,25 | nicht nachweisbar | Erdig, muffig, bitter, rauchig |
| tert-Butylmethyl-ether | 0,2 | nicht nachweisbar | bitter, adstringierend, rauchig, fleischig |
| Ethylacetat | 0,12 | Balansin A 3,8% | Süßstoff, bitter, haftfest, krautig |
| Ethanol/Wasser (4:1) (v/v) | 4,7 | Balansin A 4,6% Balansin B 4,5% | Süßstoff, süß, blumig, leicht fruchtig, schwach bitter |

| | | | |
|---|---|---|---|
| * Quantifizierung mittels LC/MS gegen einen internen Standard | | | |

Aufgrund des angenehmen Geschmacks, der Entfernung des unangenehmen Geschmacks durch die vorhergehende Extraktion mit weniger polaren Extraktionsmitteln und der deutlich höheren Ausbeute wurde der Ethanol/Wasser-Extrakt für weitere Tests ausgewählt.

### Beispiel 2: Angereicherter Mycetia balansae-Extrakt

Der in Beispiel 1 beschriebene ethanolisch-wässrige Extrakt wurde für eine Trennung mittels Hochtemperatur-Flüssigkeitschromatographie (HTLC) in einer Konzentration von 250 mg/ml in Ethanol/Wasser (1:2) (v/v) gelöst. 100 µl dieser Lösung wurden injiziert und über eine Polymer-Säule (PS-DVB, Hamilton PRP-1; 250 x 10 mm) bei 120 °C (isotherm) fraktioniert. Der nachfolgend beschriebene Gradient wurde verwendet:

| t [min] | Wasser [%] | Ethanol [%] |
|---|---|---|
| 0,0 | 100 | 0 |
| 25,0 | 75 | 25 |
| 40,0 | 0 | 100 |
| 50,0 | 0 | 100 |

Das Eluat wurde in 12 Fraktionen ä 4 Minuten geschnitten und mittels LC-MS analysiert. Nach Entfernung des Elutionsmittels wurden die Fraktionen in je 2 ml Wasser aufgenommen und von einem geschulten Panel sensorisch bewertet.

Fraktion 9, welche unter anderem die beiden erfindungsgemäßen Verbindungen Balansin A und B enthielt, wurde von den Prüfern dieses Panels als deutlich süß, süßstoff- bzw. lakritz-artig beschrieben.

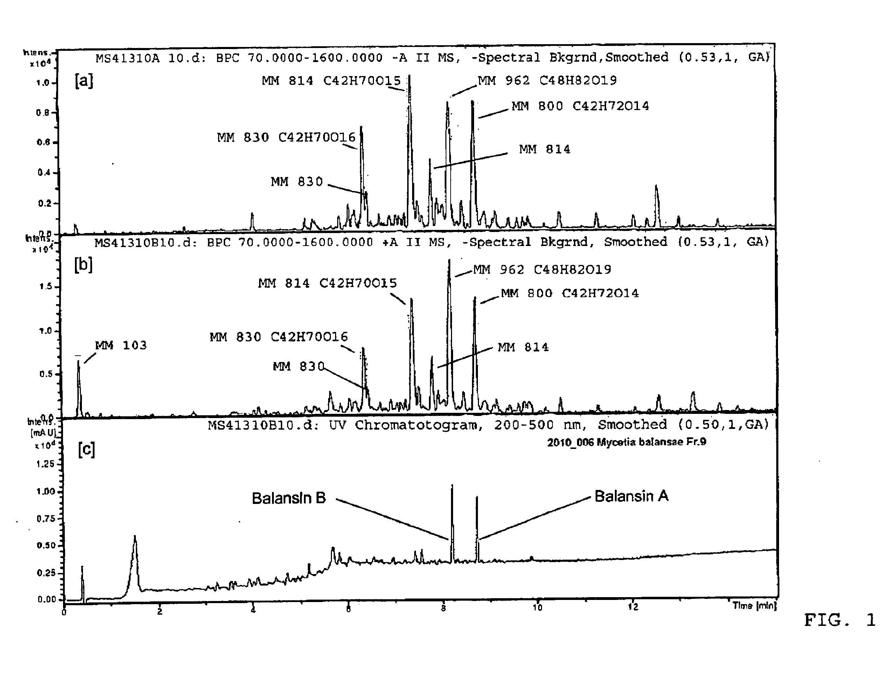

Das LC-MS Chromatogramm der Fraktion 9 enthaltend Balansin A und Balansin B ist in Figur 1 dargestellt. In Figur 1 zeigt das oberste Diagramm [a] das Massenspektrum negativ mode, das mittlere Diagramm [b] das Massenspektrum positiv mode und das untere Diagramm [c] das UV Spektrum.

### Beispiel 3: Herstellung eines wässrig/ethanolischen Mycetia balansae-Extraktes

200 g getrocknete Blätter von *Mycetia balansae* wurden 4 x erschöpfend mit je 2 Litern eines Ethanol/Wasser - Gemisches im Verhältnis (4:1) (v/v) für je 1 Stunde bei Raumtemperatur (ca. 23°C) extrahiert. Nach Filtration wurde das Extraktionsmittel unter Vakuum entfernt. Die anschließend bestimmte Ausbeute an Trockenextrakt betrug 18,23 g; der Gehalt an Balansin A lag bei 5,8%, der Gehalt an Balansin B bei 5,7%, wobei die Quantifizierung mittels LC/MS gegen einen internen Standard erfolgte.

### Beispiel 4: Isolierung von Balansin A, B und C aus Mycetia balansae Extrakt

Die Isolierung der Verbindungen Balansin A und Balansin B erfolgte mittels präparativer Hochdruckchromatographie (pHPLC). Dazu wurde der ethanolisch-wässrige Extrakt in der Konzentration 150 mg/ml in einem Wasser/Acetonitril - Gemisch (1:1) (v/v) gelöst. Die Trennung erfolgte über eine Grom Saphir 110 C-18 Säule (5 µm, 150 x 20 mm; Vorsäule 10 x 20 mm; Injektionsvolumen 1000µl). Die Elution erfolgte isokratisch mit einem Wasser/Acetonitril - Gemisch (7:3) (v/v) bei einer Flussrate von 25 ml/min und einer Detektion bei 210 nm.

Die Fraktionen von 50 Zyklen wurden im Bereich von 10,5 bis 13,0 min (Balansin B) und im Bereich von 19,5 bis 23,0 min (Balansin A) gesammelt und am Rotationsverdampfer bei 40 °C und 0,1 mbar vom Lösungsmittel befreit. Die Ausbeute an Balansin A lag dabei bei 180 mg, die Ausbeute von Balansin B lag bei 230 mg.

| **Substanz** | **Summenformel** | **Masse** | **m/z** | **UV-Maximum** |
|---|---|---|---|---|
| Balansin A | C₄₂H₇₁O₁₄ | 799.4824 | 711.4, 637.4, 549.4, 221.1, 179.1, 161.0, 143.0, 113.0, 101.0 | 200 nm |
| Balansin B | C₄₈H₈₁O₁₉ | 961.5421 | 799.5, 711.4, 637.4, 549.4, 221.1, 179.1, 161.0, 143.0, 113.0, 101.0 | 200 nm |

### Beispiel 5: Charakterisierung von Balansin A mittels NMR Spektroskopie

Für die Strukturaufklärung der isolierten Verbindung Balansin A aus Beispiel 4 wurden verschiedene Verfahren der ein- und zweidimensionalen ¹H- und ¹³C-NMR-Spektroskopie durchgeführt. Die dabei gewonnen Daten sind im Folgenden aufgeführt.

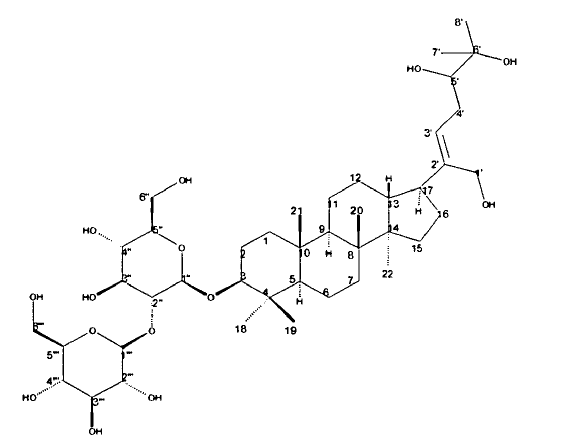

¹H-NMR; CD₃OD, TMS; 600 MHz

| C | δ in ppm | Multiplizität | Anzahl H | *J* in Hz |
|---|---|---|---|---|
| 1a | 1.71 | m | 1 | |
| 1b | 1.00 | m | 1 | |
| 2a | 1.96 | m | 1 | |
| 2b | 1.71 | m | 1 | |
| 3 | 3.24 | m | 1 | |
| 5 | 0.78 | d | 1 | 11.5 |
| 6a | 1.56 | m | 1 | |
| 6b | 1.48 | m | 1 | |
| 7a | 1.62 | m | 1 | |
| 7b | 1.30 | m | 1 | |
| 9 | 1.36 | m | 1 | |
| 11a | 1.54 | m | 1 | |
| 11b | 1.25 | m | 1 | |
| 12a | 1.64 | m | 1 | |
| 12b | 1.07 | m | 1 | |
| 13 | 1.77 | d,d,d | 1 | 3.3, 12.2, 12.2 |
| 15a | 1.64 | m | 1 | |
| 15b | 1.12 | m | 1 | |
| 16a | 1.90 | m | 1 | |
| 16b | 1.41 | m | 1 | |
| 17 | 2.30 | d,d,d | 1 | 6.8, 11.0,11.0 |
| 18 | 1.07 | s | 3 | |
| 19 | 0.85 | s | 3 | |
| 20 | 1.02 | s | 3 | |
| 21 | 0.89 | s | 3 | |
| 22 | 0.91 | s | 3 | |
| 1'a | 4.13 | d | 1 | 11.7 |
| 1'b | 3.90 | d | 1 | 11.7 |
| 3' | 5.44 | d,d | 1 | 6.9.8.7 |
| 4'a | 2.39 | d,d,d | 1 | 2.4, 6.5, 14.4 |
| 4'b | 2.20 | d,d,d | 1 | 8.9, 10.3,14.4 |
| 5' | 3.24 | m | 1 | |
| 7' | 1.18 | s | 3 | |
| 8' | 1.16 | s | 3 | |
| 1" | 4.43 | d | 1 | 7.5 |
| 2" | 3.57 | m | 1 | |
| 3" | 3.35 | d,d | 1 | 8.6,9.3 |
| 4" | 3.15-3.29 | m | 1 | |
| 5" | 3.15-3.29 | m | 1 | |
| 6"a | 3.85 | d,d | 1 | 2.0, 12.0 |
| 6"b | 3.65, | d,d | 1 | 5.5, 12.0 |
| 1"' | 4.67 | d | 1 | 7.7 |
| 2'" | 3.15-3.29 | m | 1 | |
| 3'" | 3.56 | m | 1 | |
| 4'" | 3.15-3.29 | m | 1 | |
| 5'" | 3.15-3.29 | m | 1 | |
| 6"'a | 3.82 | d,d | 1 | 2.3, 11.9 |
| 6"'b | 3.61 | d,d | 1 | 6.4, 11.9 |

| | | | | |
|---|---|---|---|---|
| ¹³C-Shiftwerte 125 MHz, CD₃OD | | | | |

| C | δ in ppm | Multiplizität |
|---|---|---|
| 1 | 40.49 | T |
| 2 | 27.36 | T |
| 3 | 91.50 | D |
| 4 | 40.69 | S |
| 5 | 57.72 | D |
| 6 | 19.31 | T |
| 7 | 36.72 | T |
| 8 | 41.79 | S |
| 9 | 52.45 | D |
| 10 | 38.15 | S |
| 11 | 22.57 | T |
| 12 | 26.05 | T |
| 13 | 46.83 | D |
| 14 | 50.37 | S |
| 15 | 32.67 | T |
| 16 | 29.56 | T |
| 17 | 49.60 | D |
| 18 | 28.43 | Q |
| 19 | 16.79 | Q |
| 20 | 16.24 | Q |
| 21 | 16.88 | Q |
| 22 | 16.35 | Q |
| 1' | 58.94 | T |
| 2' | 144.38 | S |
| 3' | 127.82 | D |
| 4' | 30.94 | T |
| 5' | 78.70 | D |
| 6' | 73.87 | S |
| 7' | a 26.02 | Q |
| 8' | a 24.78 | Q |
| 1" | 105.45 | D |
| 2" | 81.08 | D |
| 3" | b 78.57 | D |
| 4" | c 71.98 | D |
| 5" | b 78.41 | D |
| 6" | 62.90 | T |
| 1"' | 104.54 | D |
| 2"' | 76.35 | D |
| 3"' | b 77.94 | D |
| 4"' | c 71.65 | D |
| 5"' | b 77.74 | D |
| 6"' | 63.18 | T |

### Beispiel 6: Charakterisierung von Balansin B mittels NMR Spektroskopie

Für die Strukturaufklärung der isolierten Verbindung Balansin B aus Beispiel 4 wurden verschiedene Verfahren der ein- und zweidimensionalen ¹H- und ¹³C-NMR-Spektroskopie durchgeführt. Die dabei gewonnen Daten sind im Folgenden aufgeführt. 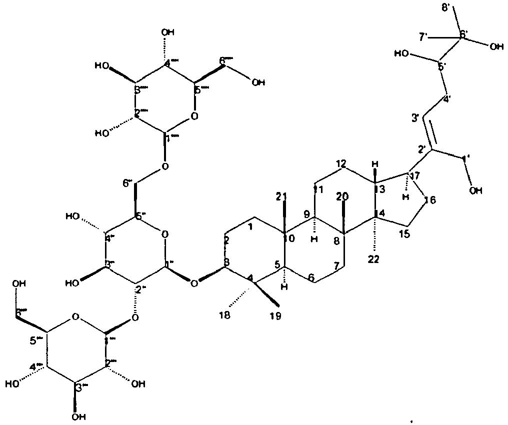 ¹H-NMR; CD₃OD, TMS; 600 MHz

| C | δ in ppm | Multiplizität | Anzahl H | J in Hz |
|---|---|---|---|---|
| 1a | 1.72 | m | 1 | |
| 1b | 1.02 | m | 1 | |
| 2a | 1.95 | m | 1 | |
| 2b | 1.71 | m | 1 | |
| 3 | 3.23 | m | 1 | |
| 5 | 0.79 | d,d | 1 | 1.7, 11.6 |
| 6a | 1.56 | m | 1 | |
| 6b | 1.49 | d,d,d | 1 | 2.4, 12.6, 12.6 |
| 7a | 1.62 | m | 1 | |
| 7b | 1.31 | m | 1 | |
| 9 | 1.37 | d,d | 1 | 2.7, 12.8 |
| 11a | 1.56 | m | 1 | |
| 11b | 1.24 | m | 1 | |
| 12a | 1.63 | m | 1 | |
| 12b | 1.07 | m | 1 | |
| 13 | 1.77 | d,d,d | 1 | 3.5, 12.0, 12.0 |
| 15a | 1.65 | m | 1 | |
| 15b | 1.13 | m | 1 | |
| 16a | 1.90 | d,d,d,d | 1 | 8.8, 8.8, 10.1, 13.4 |
| 16b | 1.41 | m | 1 | |
| 17 | 2.30 | d,d,d | 1 | 6.9, 10.6, 10.6 |
| 18 | 1.07 | s | 3 | |
| 19 | 0.85 | s | 3 | |
| 20 | 1.02 | s | 3 | |
| 21 | 0.89 | s | 3 | |
| 22 | 0.91 | s | 3 | |
| 1'a | 4.13 | d | 1 | 11.5 |
| 1'b | 3.91 | d | 1 | 11.5 |
| 3' | 5.44 | d,d | 1 | 6.8,8.6 |
| 4'a | 2.39 | d,d,d | 1 | 2.1, 6.7, 14.3, |
| 4'b | 2.20 | d,d,d | 1 | 8.9, 10.0, 14.3 |
| 5' | 3.23 | m | 1 | |
| 7' | 1.18 | s | 3 | |
| 8' | 1.16 | s | 3 | |
| 1" | 4.45 | d | 1 | 7.5 |
| 2" | 3.58 | m | 1 | |
| 3" | 3.55 | d,d | 1 | 8.8, 8.8 |
| 4" | 3.35 | m | 1 | |
| 5" | 3.46 | d,d,d | 1 | 2.0, 5.9, 9.9 |
| 6"a | 4.12 | d,d | 1 | 1.9, 11.8 |
| 6"b | 3.78 | d,d | 1 | 5.9, 11.8 |
| 1"' | 4.67 | d | 1 | 7.7 |
| 2"' | 3.17 - 3.29 | m | 1 | |
| 3"' | 3.17 - 3.29 | m | 1 | |
| 4"' | 3.17 - 3.29 | m | 1 | |
| 5"' | 3.17 - 3.29 | m | 1 | |
| 6"'a | 3.82 | d,d | 1 | 2.4, 11.8 |
| 6"'b | 3.61 | d,d | 1 | 6.1, 11.8 |
| 1"' | 4.39 | d | 1 | 7.9 |
| 2"" | 3.17 - 3.29 | m | 1 | |
| 3"" | 3.17 - 3.29 | m | 1 | |
| 4"" | 3.17 - 3.29 | m | 1 | |
| 5"" | 3.17 - 3.29 | m | 1 | |
| 6""a | 3.86 | d,d | 1 | 2.1, 11.9 |
| 6""b | 3.66 | d,d | 1 | 5.4, 11.9 |

¹³C-NMR, 125 MHz, CD₃OD

| C | δ in ppm | Multiplizität |
|---|---|---|
| 1 | 40.48 | T |
| 2 | 27.44 | T |
| 3 | 91.45 | D |
| 4 | 40.71 | S |
| 5 | 57.68 | D |
| 6 | 19.33 | T |
| 7 | 36.71 | T |
| 8 | 41.79 | S |
| 9 | 52.38 | D |
| 10 | 38.18 | S |
| 11 | 22.56 | T |
| 12 | 26.06 | T |
| 13 | 46.84 | D |
| 14 | 50.40 | S |
| 15 | 32.67 | T |
| 16 | 29.59 | T |
| 17 | 49.61 | D |
| 18 | 28.42 | Q |
| 19 | 16.81 | Q |
| 20 | 16.24 | Q |
| 21 | 16.90 | Q |
| 22 | 16.38 | Q |
| 1' | 58.97 | T |
| 2' | 144.39 | S |
| 3' | 127.78 | D |
| 4' | 30.95 | T |
| 5' | 78.72 | D |
| 6' | 73.87 | S |
| 7' | a 26.02 | Q |
| 8' | a 24.78 | Q |
| 1" | 105.35 | D |
| 2" | 80.91 | D |
| 3" | b 78.38 | D |
| 4" | 71.52 | D |
| 5" | 76.95 | D |
| 6" | 70.11 | T |
| 1"' | 104.51 | D |
| 2"' | 76.35 | D |
| 3"' | b 78.39 | D |
| 4'" | 72.00 | D |
| 5'" | b 78.12 | D |
| 6"' | 63.19 | T |
| 1"" | 104.93 | D |
| 2"" | 75.25 | D |
| 3"" | b 78.08 | D |
| 4"" | 71.74 | D |
| 5"" | b 77.94 | D |
| 6"" | 62.87 | T |

### Anwendungsbeispiel 1: Sensorische Bewertung von Balansin A und Balansin B

Die beiden isolierten Verbindungen Balansin A und Balansin B aus Beispiel 4 wurden jeweils bei einer Konzentration von 50 ppm auf Wasser, sowie einer 5 Gew.-%igen wässrigen Sucroselösung von einer Expertengruppe verkostet und sensorisch bewertet.

Balansin A wurde in wässriger Lösung als deutlich süß beschrieben, ebenso als leicht bitter und wies eine Süßstoff-artige Note auf.

Balansin B wurde als leicht bitter, leicht süßstoffartig und schwach ethanolisch beschrieben, wies ansonsten jedoch keinen merklichen Eigengeschmack auf.

### Anwendungsbeispiel 2: Eigensüße von Balansin A

Um die Eigensüße der süßschmeckenden Verbindung Balansin A zu bewerten, wurden verschiedene Konzentrationen von Balansin A gegen eine Sucrose-Referenzreihe (10 verschiedene Konzentrationen in aufsteigender Reihenfolge) verkostet. Diese Referenzreihe bestand aus Proben mit den Balansin A wurde in den folgenden Konzentrationen getestet: 0; 0,25; 0,5; 0,75; 1; 1,5; 2; 3; 4 und 5 Gew.-% Sucrose in Wasser.

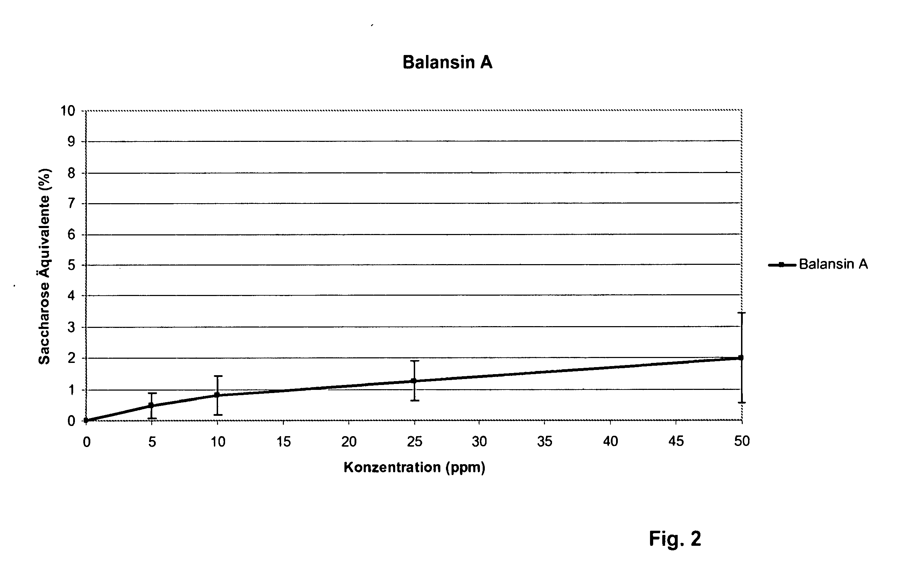

Figur 2 veranschaulicht die intrinsische Süße von Balansin A bei Konzentrationen von 5, 10, 25 und 50 ppm (aufgetragen auf der Abszisse) im Vergleich mit einer Saccharose-Referenzreihe. Die Abszisse gibt die Konzentration von Balansin A in ppm an, die Ordinate die Saccharose-Äquivalente (= Sucrose-Äquivalente).

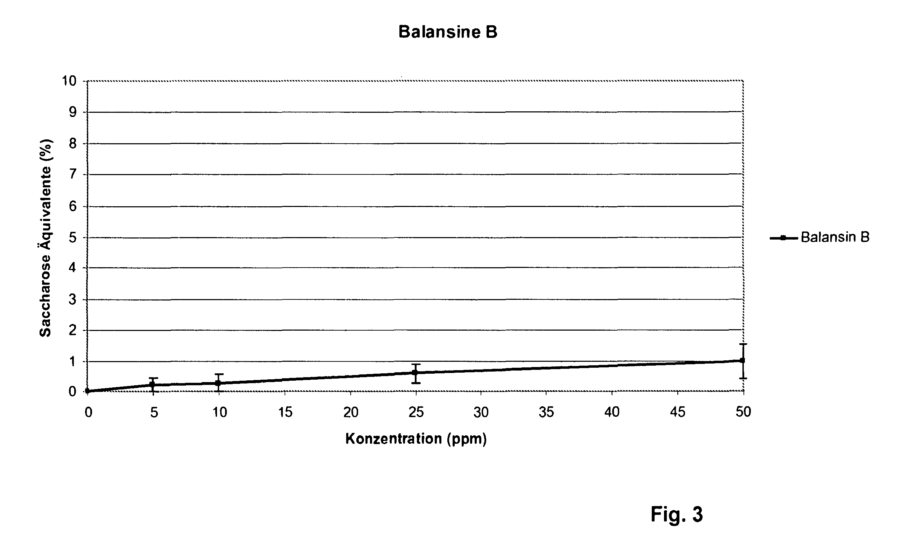

Figur 3 veranschaulicht die intrinsische Süße von Balansin B bei Konzentrationen von 5, 10, 25 und 50 ppm (aufgetragen auf der Abszisse) im Vergleich mit einer Saccharose-Referenzreihe. Die Abszisse gibt die Konzentration von Balansin A in ppm an, die Ordinate die Saccharose-Äquivalente (= Sucrose-Äquivalente).

Aus den Figuren 2 und 3 ist ersichtlich, dass sowohl Balansin A als auch Balansin B eine gewisse Eigensüße auf. Bei einer Konzentration von 50 ppm entspricht die Eigensüße von Balansin A der Süße von 2% Saccharose, die Eigensüße von Balansin B der Süße von 1 % Saccharose.

### Anwendungsbeispiel 3: Verstärkung des Süßeindrucks einer Zuckerlösung

Um die Verstärkung des Süß-Eindrucks zu quantifizieren, wurde die Süße einer 5 Gew.-% enthaltenden Sucroselösung und einer Probe, die 5 Gew.-% Sucrose und eine Menge der Testsubstanz bzw. des Testextraktes enthielt, von einer Expertengruppe bestimmt (Einstufung 1 [nicht süß] bis 10 [extrem süß]). Die Auswertung erfolgte als Berechnung der Reduktion (in %) des Süßeindrucks aus den Durchschnittswerten der Einschätzungen der Sucroselösung bzw. der Sucrose und Verbindung 2 enthaltenden Lösung.

| **Substanz/Extrakt** | **Süß-Eindruck (1-10)** | | **% Verstärkung des Süß-Eindrucks** |
|---|---|---|---|
| | **ohne** | **mit** | |
| Balansin A, 25 ppm | 4,9 ± 1,5 | 7,2 ± 1,4 | 48 % (p < 0,00005) |
| Balansin B, 50 ppm | 5,1 ± 1,1 | 6,7 ± 1,1 | 31 % (p < 0,0005) |
| *Mycetia balansae* Extrakt, 550 ppm (entsprechend 25 ppm Balansin A) | 4,8 ± 1,2 | 7,1 ± 2,4 | 46,6 % (p < 0,001) |

### Anwendungsbeispiel 4: Zuckerreduziertes Erfrischungsgetränk Typ Zitrone

Zubereitung A: Vergleichszubereitung mit 10 Gew.-% Zucker
Zubereitung B: Vergleichszubereitung mit 8 Gew.-% Zucker
Zubereitung C-I: erfindungsgemäße zuckerreduzierte Zubereitungen mit 8 Gew.-% Zucker

| **Inhaltsstoff** | **Einsatz in Gew.-%** | | | | | | | | |
|---|---|---|---|---|---|---|---|---|---|
| **Zubereitung** | **A** | **B** | **C** | **D** | **E** | **F** | **G** | **H** | **I** |
| Zucker (Sucrose) | 10 | 8 | 8 | 8 | 8 | 8 | 8 | 7 | 0 |
| Zitronensäure | 0,15 | 0,15 | 0,15 | 0,15 | 0,15 | 0,15 | 0,15 | 0,15 | 0,15 |
| Zitronenaroma | 0,1 | 0,1 | 0,1 | 0,1 | 0,1 | 0,1 | 0,1 | 0,1 | 0,1 |
| Balansin A | - | - | 0,005 | - | 0,0025 | - | 0,001 | 0,001 | 0,010 |
| Balansin B | - | - | - | - | - | 0,002 | - | - | 0,050 |
| Phloretin | - | - | - | - | - | - | 0,001 | - | - |
| Hesperetin | - | - | - | - | 0,010 | - | - | - | - |
| 3',7-Dihydroxy-4'-methoxyflavan gemäß EP 2 253 226 | - | - | - | - | - | - | - | 0,0025 | - |
| Extrakt aus *Rubus suavissimus,* enthaltend 5 Gew.-% Rubusosid bez. auf das Gesamtgewicht des Extraktes | - | - | - | - | - | 0,010 | - | - | - |
| Extrakt aus Extrakt aus *Hydrangea dulcis* enthaltend 8 % Phyllodulcin, bez. auf das Gesamtgewicht des Extraktes | - | | | - | - | - | - | 0,010 | - |
| Wasser | auf 100 auffüllen | | | | | | | | |

Die Zutaten wurden in der angegebenen Reihenfolge gemischt und mit Wasser auf 100 % aufgefüllt. Die Mischungen werden in Glasflaschen gefüllt und carbonisiert.

Die Zubereitungen wurden sensorisch von Experten eingeschätzt. Durch Verringern von Zucker (20 Gew.-% bezogen auf die Sucrosemenge) wurde eine Abnahme der Süße von ca. 36 % beobachtet (Zubereitungen B zu A). In den Zubereitungen C bis I konnte der süße Geschmack nicht von der Vollzuckerzubereitung A unterschieden werden.

### Anwendungsbeispiel 5: Halbfertigwaren (Aromakompositionen), enthaltend natürliche Süßstoffe

| | Zubereitung (Einsatz in Gew.-%) | | | | | | | |
|---|---|---|---|---|---|---|---|---|
| Inhaltsstoff | A | B | C | D | E | F | G | H |
| "Flüssigzucker", enthält 80 % Sucrose | 99,9 | - | - | - | - | - | 99,85 | - |
| Rebaudiosid A 98 % | - | 85 | - | - | - | 25 | 0,01 | 73,5 |
| Steviosid 95 % | - | - | 73 | - | - | - | - | - |
| Balansin A | 0,05 | 10 | | - | 80 | 10 | 0,02 | 10 |
| Balansin B | - | 5 | 20 | - | - | 10 | - | 10 |
| Extrakt aus *Mycetia balansae* nach Beispiel 3 | - | - | - | 30 | | | | |
| Extrakt aus *Rubus suavissimus,* enthaltend 5 Gew.-% Rubusosid, bezogen auf das Gesamtgewicht des Extraktes z.B. von PlantExtrakt | - | - | - | 25 | - | 25 | 0,07 | - |
| Extrakt aus Extrakt aus *Hydrangea dulcis* enthaltend 8 % Phyllodulcin, bez. auf das Gesamtgewicht des Extraktes | - | - | - | 25 | - | 25 | - | - |
| Phloretin | 0,02 | - | 4 | 5 | 3,2 | 3,5 | 0,02 | 5 |
| Hesperetin | 0,02 | - | 1 | 5 | 0,8 | 1 | 0,02 | 1 |
| 3',7-Dihydroxy-4'-methoxyflavan gemäß EP 2 253 226 | 0,01 | - | 2 | 8 | - | - | 0,01 | - |
| Neohesperidin-dihydrochalkon | - | - | - | - | - | 0,5 | - | - |
| Homoeriodictyol-Natriumsalz | - | - | - | - | 16 | - | - | - |
| Vanillin, natürlich | - | - | - | 2 | - | - | - | - |
| Zuckerdestillat aus Rohrzucker (z.B. Treatt) | - | - | - | - | - | - | - | 0,5 |

Die Inhaltsstoffe werden in den oben angegebenen Mengenverhältnissen gemischt und können dann in dieser Form (weiter) verwendet werden. Die typische Dosierung der Zubereitungen A und G im Fertigprodukt liegt im Bereich von 7 bis 15 Gew.-%, bezogen auf das Gesamtgewicht des Fertigproduktes. Die typische Dosierung der übrigen Zubereitungen des Anwendungsbeispiels 5 liegt im Bereich von 0,01 bis 0,1 Gew.-%, bevorzugt bei 0,03 bis 0,06 Gew.-%, bezogen auf das Gesamtgewicht des Fertigproduktes.

### Anwendungsbeispiel 6: Erfindungsgemäße Gemische bzw. Halbfertigwaren

| | Zubereitung (Einsatz in Gew.-%) | | | | | | | |
|---|---|---|---|---|---|---|---|---|
| Inhaltsstoff | A | B | C | D | E | F | G | H |
| Balansin A | 80 | 20 | 50 | 50 | 20 | 40 | 20 | 20 |
| Balansin B | 20 | 80 | | | | | | 20 |
| Rebaudiosid A 98 % | | | 50 | 25 | | | | 20 |
| Steviosid 95 % | | | | 25 | | | | |
| Saccharin^{®}-Natriumsalz | | | | | | 20 | | |
| Cyclamat^{®} | | | | | 75 | | | |
| Acesulfam^{®} K | | | | | | 20 | | |
| Aspartam^{®} | | | | | | 20 | | |
| Neotam^{®} | | | | | 5 | | | |
| Thaumatin | | | | | | | | 20 |
| Sucralose^{®} | | | | | | | 80 | |
| Glycyrrhizin-Ammoniumsaz | | | | | | | | 20 |

Die Inhaltsstoffe werden in den oben angegebenen Mengenverhältnissen gemischt und zur Süßung von oral konsumierbaren Zubereitungen verwendet. Die typische Dosierung der Mischungen A bis H im Fertigprodukt liegt im Bereich von 0,001 bis 0,5 Gew.-%, bevorzugt im Bereich von 0,003 bis 0,1 Gew.-%, bezogen auf das Gesamtgewicht des Fertigproduktes.

### Anwendungsbeispiel 7: Halbfertigwaren (mit nicht-kalorischen Zuckern und/oder Zuckeralkoholen)

| | Zubereitung (Einsatz in Gew.-%) | | | | | | | |
|---|---|---|---|---|---|---|---|---|
| Inhaltsstoff | A | B | C | D | E | F | G | H |
| Mischung A aus Anwendungsbeispiel 6 | | 0,05 | | | 0,05 | | | 0,05 |
| Mischung C aus Anwendungsbeispiel 6 | | | 0,05 | | | 0,05 | | |
| Mischung H aus Anwendungsbeispiel 6 | | | | 0,05 | | | 0,05 | |
| Balansin A | 0,05 | | | | | | | |
| Maltit | 50 | 10 | | | | | | |
| Mannit | | 10 | | | | | | |
| Sorbitol | 20 | 20 | | | | | | |
| Erythritol | 29,95 | 59,95 | 99,95 | 99,95 | | 50 | 50 | 99,95 |
| Xylitol | | | | | | | 49,95 | |
| Palatinose | | | | | 50 | | | |
| Tagatose | | | | | 49,95 | 49,95 | | |

Die Stoffe werden in den oben angegebenen Mengenverhältnissen gemischt und zur Süßung von oral konsumierbaren Zubereitungen verwendet. Die typische Dosierung der Halbfertigwaren A bis H im Fertigprodukt liegt im Bereich von 0,01 bis 80 Gew.-%, bevorzugt liegt im Bereich von 0,1 bis 50 Gew.-%, besonders bevorzugt liegt im Bereich von 0,1 bis 10 Gew.-%, jeweils bezogen auf das Gesamtgewicht des Fertigproduktes. Die Halbfertigwaren A bis H können als Süßungsmittel z.B. auch direkt für Kaffee oder Tee eingesetzt werden.

### Anwendungsbeispiel 8: Sprühgetrocknete Zubereitung als Halbfertigware zur Aromatisierung von Fertigwaren

| **Inhaltsstoff** | **Einsatz in Gew.-%** | | | | |
|---|---|---|---|---|---|
| **Zubereitung** | **A** | **B** | **C** | **D** | **E** |
| Trinkwasser | 60,8 | 60,8 | 60,8 | 60,8 | 60,8 |
| Maltodextrin aus Weizen | 24,3 | 24,3 | 24,3 | 24,3 | 24,3 |
| Gummi Arabicum | 6,1 | 6,1 | 6,1 | 6,1 | 6,1 |
| Balansin A | 8,8 | 3,3 | 4,0 | - | - |
| Balansin B | - | 3,3 | 1,5 | 3,3 | 4,4 |
| Hesperetin | - | 2,2 | - | - | 1,1 |
| Homoeriodictyol-Natriumsalz | - | - | - | 5,5 | 3,3 |
| Phloretin | - | - | 3,3 | - | - |

Das Trinkwasser wird in einem Behälter vorgelegt und das Maltodextrin und das Gummi Arabicum darin gelöst. Anschließend werden die Aromastoffe mit einem Turrax in die Trägerstofflösung emulgiert. Die Temperatur der Sprühlösung sollte 30°C nicht überschreiten. Das Gemisch wird dann sprühgetrocknet (Solltemperatur Eingang: 185 - 195°C, Solltemperatur Ausgang: 70 - 75°C).

### Anwendungsbeispiel 9: Lösungen der Halbfertigwaren

Die Gemische und Halbfertigwaren aus den obigen Anwendungsbeispielen 5, 6 und 7 können auch mit Wasser, Propylenglycol, Glycerin bzw. Ethanol oder bevorzugt mit Gemischen der vorgenannten Lösungsmittel (z.B. Wasser-Propylenglycol, Wasser-Glycerin, Wasser-Ethanol, Glycerin-Ethanol, Glycerin-Propylenglycol, Propylenglycol-Ethanol) beispielsweise als 1 - 20%ige Lösung, bevorzugt 2 - 10 %ige, besonders bevorzugt 5 %ige Lösung aufgenommen und durch leichtes Erwärmen vollständig gelöst werden.

### Anwendungsbeispiel 10: Softdrink Typ "Cola"

| | Zubereitung (Einsatz in Gew.-%) | | | | | | |
|---|---|---|---|---|---|---|---|
| Inhaltsstoff | **A** | **B** | **C** | **D** | **E** | **F** | **G** |
| Saccharose | 0 | 8 | 7 | 7 | - | 7 | - |
| Glucose/Fructose-Sirup aus Mais, enthaltend 55 Gew.-% Fructose | - | - | - | - | 8 | - | 7 |
| Aromakomposition A aus Anwendungsbeispiel 5 | 10 | - | - | - | - | - | - |
| Aromakomposition B aus Anwendungsbeispiel 4 | - | 0,05 | - | - | - | - | - |
| Aromakomposition C aus Anwendungsbeispiel 4 | - | - | 0,05 | - | - | - | - |
| Aromakomposition D aus Anwendungsbeispiel 4 | - | - | - | 0,05 | - | - | - |
| Aromakomposition E aus Anwendungsbeispiel 4 | - | - | - | - | 0,05 | - | - |
| Aromakomposition F aus Anwendungsbeispiel 4 | - | - | - | - | - | 0,05 | - |
| Aromakomposition H aus Anwendungsbeispiel 4 | - | - | - | - | - | - | 0,05 |
| Phosphorsäure | 0,07 | 0,07 | 0,07 | 0,07 | 0,07 | 0,07 | 0,07 |
| Citronensäure | 0,06 | 0,06 | 0,06 | 0,06 | 0,06 | 0,06 | 0,06 |
| Zuckercouleur | 0,14 | 0,14 | 0,14 | 0,14 | 0,14 | 0,14 | 0,14 |
| Coffein | 0,01 | 0,01 | 0,01 | 0,01 | 0,01 | 0,01 | 0,01 |
| Getränke-Emulsion Typ "Cola" | 0,05 | 0,05 | 0,05 | 0,05 | 0,05 | 0,05 | 0,05 |
| Wasser | auf 100% auffüllen | | | | | | |

Die Zutaten wurden in der angegebenen Reihenfolge gemischt, in Flaschen gefüllt und carbonisiert.

### Anwendungsbeispiel 11: Zuckerreduziertes Erfrischungsgetränk Typ "Cola"

Zubereitung A: Vergleichszubereitung mit 10 Gew.-% Zucker
Zubereitung B: Vergleichszubereitung mit 8 Gew.-% Zucker
Zubereitung C-I: erfindungsgemäße zuckerreduzierte Zubereitungen mit 8 Gew.-% Zucker

| | Zubereitung (Einsatz in Gew.-%) | | | | | | |
|---|---|---|---|---|---|---|---|
| Inhaltsstoff | **A** | **B** | **C** | **D** | **E** | **F** | **G** |
| Saccharose | 0 | 8 | 8 | 8 | - | - | - |
| Glucose/Fructose-Sirup aus Mais, enthaltend 55 Gew.-% Fructose | - | - | - | - | 8 | 8 | 8 |
| Balansin A | 0,005 | - | 0,003 | 0,0025 | - | 0,003 | 0,0025 |
| Balansin B | - | 0,005 | - | 0,0025 | 0,005 | - | 0,0025 |
| Phosphorsäure | 0,07 | 0,07 | 0,07 | 0,07 | 0,07 | 0,07 | 0,07 |
| Citronensäure | 0,06 | 0,06 | 0,06 | 0,06 | 0,06 | 0,06 | 0,06 |
| Zuckercouleur | 0,14 | 0,14 | 0,14 | 0,14 | 0,14 | 0,14 | 0,14 |
| Coffein | 0,01 | 0,01 | 0,01 | 0,01 | 0,01 | 0,01 | 0,01 |
| Getränke-Emulsion Typ "Cola" | 0,05 | 0,05 | 0,05 | 0,05 | 0,05 | 0,05 | 0,05 |
| Wasser | auf 100 % auffüllen | | | | | | |

Die Zutaten wurden in der angegebenen Reihenfolge gemischt und mit Wasser auf 100 % aufgefüllt. Die Mischungen werden in Glasflaschen gefüllt und carbonisiert.

### Anwendungsbeispiel 12: Kaugummi

| **Teil** | **Inhaltsstoff** | **Einsatz in Gew.-%** |
|---|---|---|
| A | Kaugummibase, Company "Jagum T" | 30,95 |
| B | Sorbit, pulverisiert | 39,00 |
| | Isomalt^{®} (Palatinit GmbH) | 9,50 |
| | Xylit | 2,00 |
| | Mannit | 3,00 |
| | Aspartam^{®} | 0,10 |
| | Acesulfam^{®} K | 0,10 |
| | Emulgum^{®} (Colloides Naturels, Inc.) | 0,30 |
| C | Sorbitol, 70% | 14,00 |
| | Glycerin | 1,00 |
| D | Balansin A | 0,05 |

Teile A bis D werden gemischt und intensiv geknetet. Die Rohmasse kann z.B. in Form von dünnen Streifen zu verzehrsfertigen Kaugummis verarbeitet werden.

### Anwendungsbeispiel 13: Zahnpasta

| **Teil** | **Inhaltsstoff** | **Einsatz in Gew.-%** |
|---|---|---|
| A | demineralisiertes Wasser | 23,08 |
| | Sorbitol (70%) | 45,00 |
| | Solbrol^{®} M, Natriumsalz (Bayer AG, p-Hydroxybenzoesäurealkylester) | 0,15 |
| | Trinatriumphosphat | 0,10 |
| | Balansin A | 0,02 |
| | Natriummonofluorphosphat | 1,12 |
| | Polyethylenglycol 1500 | 5,00 |
| B | Sident 9 (abrasives Siliciumdioxid) | 10,00 |
| | Sident 22 S (verdickendes Siliciumdioxid) | 8,00 |
| | Natriumcarboxymethylcellulose | 0,90 |
| | Titandioxid | 0,50 |
| C | demineralisertes Wasser | 4,53 |
| | Natriumlaurylsulfat | 1,50 |
| D | Pfefferminzaroma | 0,1 |

Die Inhaltsstoffe der Teile A und B werden jeweils für sich vorgemischt und zusammen unter Vakuum bei 25 - 30°C 30 min gut verrührt. Teil C wird vorgemischt und zu A und B gegeben; D wird hinzugefügt und die Mischung unter Vakuum bei 25 - 30°C 30 min gut verrührt. Nach Entspannung ist die Zahnpasta fertig und kann abgefüllt werden.

### Anwendungsbeispiel 14: Zuckerfreie Hartkaramelle

| **Inhaltsstoff** | **Gehalt (Angaben in Gew.-%)** | | | |
|---|---|---|---|---|
| | **A** | **B** | **C** | **D** |
| Palatinit, Typ M | 75,00 | 74,00 | 75,50 | 75,00 |
| Citronensäure | - | 1,0 | 0,5 | - |
| Wasser | 24,88 | 24,842 | 23,87 | 24,8815 |
| Farbstoff gelb | - | 0,01 | - | - |
| Farbstoff rot | - | - | 0,01 | - |
| Farbstoff blau | 0,01 | - | - | 0,01 |
| Pfefferminzaroma | 0,1 | - | - | 0,1 |
| Zitronenaroma | - | 0,1 | - | - |
| Rotfruchtaroma | - | - | 0,1 | - |
| Rebaudiosid A 98 % | - | 0,040 | - | - |
| Balansin A | 0,010 | 0,005 | 0,020 | 0,005 |
| Hesperetin | - | 0,001 | - | 0,001 |
| Phloretin | - | 0,002 | - | - |
| 3',7-Dihydroxy-4'-methoxyflavan gemäß EP 2 253 226 | - | - | - | 0,0025 |

Palatinit wurde gegebenenfalls nach Zugabe der Zitronensäure mit Wasser gemischt und die Mischung bei 165 °C aufgeschmolzen und anschließend auf 115°C abgekühlt. Das Aroma und die anderen Bestandteile wurden zugegeben und nach dem Durchmischen in Formen gegossen, nach dem Erstarren aus den Formen entfernt und anschließend einzeln verpackt.

### Anwendungsbeispiel 15: Zuckerreduzierter Kochpudding

Die Zubereitungen A und B sind Vergleichszubereitungen mit vollem (Zubereitung A) bzw. reduziertem Zuckergehalt (Zubereitung B)

| | **Zubereitung (Angaben in Gew.-%)** | | | | | |
|---|---|---|---|---|---|---|
| **Inhaltsstoff** | **A** | **B** | **C** | **D** | **E** | **F** |
| Sucrose | 7,8 % | 5,4 % | 5,4 % | 5,4 % | 5,4 % | 5,4 % |
| Stärke | 3,0 % | 3,0 % | 3,0 % | 3,0 % | 3,0 % | 3,0 % |
| Magermilch-pulver | 1,5 % | 1,5 % | 1,5 % | 1,5 % | 1,5 % | 1,5 % |
| Aubygel MR50 | 0,5 % | 0,5 % | 0,5 % | 0,5 % | 0,5 % | 0,5 % |
| Balansin A | - | - | 0,01 % | 0,005 % | 0,005 % | 0,005 % |
| Extrakt (z.B. von PlantExtrakt) aus Rubus suavissimus, enthaltend 5 Gew.-% Rubusosid, bezogen auf das Gesamtgewicht des Extraktes | - | - | - | - | 0,010 % | 0,005 % |
| Hesperetin | - | - | - | 0,001 % | - | 0,001 % |
| Phloretin | - | - | - | 0,002 % | - | 0,001 % |
| 3',7-Dihydroxy-4'-methoxyflavan gemäß EP 2 253 226 | - | - | - | - | - | 0,001 % |
| Vanilleschoten-Extrakt, sprühgetrocknet, Symrise | 0,1 % | 0,1 % | 0,1 % | 0,1 % | 0,1 % | 0,1 % |
| Milch 1,5 % Fettanteil | auf 100 % auffüllen | | | | | |

Die festen Stoffe wurden vorgelegt und mit der Milch aufgerührt. Die Mischung wurde auf 95 °C für 2 min unter gutem Rühren aufgewärmt, abgefüllt und auf 5 - 8 °C abgekühlt.

### Anwendungsbeispiel 16: Fettarme Joghurts

Zubereitung A mit Zucker ist eine Vergleichszubereitung. Erfindungsgemäße Zubereitungen mit Süßstoffmischung und Extrakt aus Beispiel 2) (B-D)

| | **Zubereitung (Angaben in Gew.-%)** | | | |
|---|---|---|---|---|
| **Inhaltsstoff** | **A** | **B** | **C** | **D** |
| Sucrose | 10 | 8 | 6 | - |
| Fruchtzubereitung Erdbeere | 10 | 10 | 10 | 10 |
| Rebaudiosid A 98 % | - | - | - | 0,050 |
| Balansin A | - | 0,010 | 0,005 | 0,010 |
| Extrakt aus Rubus suavissimus (z.B. von PlantExtrakt) enthaltend 5 Gew.-% Rubusosid, bezogen auf das Gesamtgewicht des Extraktes | - | - | 0,010 | - |
| Hesperetin | - | 0,001 | 0,001 | 0,001 |
| Phloretin | - | - | 0,002 | - |
| Hopmoeriodictyol-Natriumsalz | - | - | - | 0,005 |
| Natürliches Erdbeeraroma | 0,1 % | 0,1 % | 0,1 % | 0,1 % |
| Joghurt, 0,1 % Fett | auf 100 % auffüllen | | | |

Die Inhaltsstoffe wurden gemischt und bei 5°C gekühlt.

### Anwendungsbeispiel 17: Milchmischgetränke

Zubereitung A ist eine Vergleichszubereitung mit Zucker
Zubereitung B bis D sind Erfindungsgemäße Zubereitungen

| | **Zubereitung (Angaben in Gew.-%)** | | | |
|---|---|---|---|---|
| **Inhaltsstoff** | **A** | **B** | **C** | **D** |
| **Sucrose** | 10 | 8 | 7 | - |
| Fructose | - | - | 0,5 | - |
| Rebaudiosid A 98 % | - | - | - | 0,040 |
| Balansin A | - | 0,010 | 0,005 | 0,010 |
| Extrakt aus Rubus suavissimus (z.B. von PlantExtrakt) enthaltend 5 Gew.-% Rubusosid, bezogen auf das Gesamtgewicht des Extraktes, | - | - | 0,010 | - |
| Hesperetin | - | 0,003 | 0,002 | 0,005 |
| Phloretin | - | - | 0,002 | - |
| Homoeriodictyol-Natriumsalz | - | - | - | 0,002 |
| Vanille-Aroma | 0,1 | 0,1 | 0,1 | 0,1 |
| H-Milch, 1,5 % Fett | auf 100 % auffüllen | | | |

Die Inhaltsstoffe wurden gemischt, mit Milch aufgefüllt, gut gerührt, in Flaschen abgefüllt und bei 5°C gekühlt gelagert.

### Anwendungsbeispiel 18: Zuckerreduziertes Tomatenketchup

Vergleichszubereitung mit Zucker (A)
Vergleichszubereitung mit reduziertem Zuckeranteil (B)
Erfindungsgemäße Zubereitungen (C-I)

| | **Zubereitung (Angaben in Gew.-%)** | | | | | | | |
|---|---|---|---|---|---|---|---|---|
| **Inhaltsstoff** | **A** | **B** | **C** | **E** | **F** | **G** | **H** | **I** |
| Kochsalz | 2 | 2 | 2 | 2 | 2 | 2 | 2 | 2 |
| Stärke, Farinex WM 55 | 1 | 1 | 1 | 1 | 1 | 1 | 1 | 1 |
| Sucrose | 12 | 9,6 | 9,2 | 8,4 | 9,6 | 9,6 | 8,4 | 4,2 |
| Tomaten-Konzentrat 2-fach | 40 | 40 | 40 | 40 | 30 | 30 | 30 | 30 |
| Glucosesirup 80 Brix | 18 | 18 | 18 | 18 | 18 | 18 | 18 | 18 |
| Branntweinessig 10% | 7 | 7 | 7 | 7 | 3 | 3 | 3 | 3 |
| Rebaudiosid A 98 % | - | - | - | - | - | - | - | 0,05 |
| Balansin A | - | - | 0,01 | 0,005 | 0,005 | 0,01 | 0,005 | 0,01 |
| Extrakt aus Rubus suavissimus (z.B. von PlantExtrakt) enthaltend 5 Gew.-% Rubusosid, bezogen auf das Gesamtgewicht des Extraktes | - | - | - | - | - | - | 0,01 | - |
| Hesperetin 2,5%ig in 1,2-Propylenglycol | - | - | - | - | 0,1 | - | 0,1 | - |
| Phloretin 2,5% in 1,2-Propylenglycol | - | - | - | 0,2 | 0,2 | - | - | 0,3 |
| Wasser | auf 100 % auffüllen | | | | | | | |

Die Inhaltsstoffe werden in der angegebenen Reihenfolge gemischt und das fertige Ketchup mit Hilfe eines Rührwerkes homogenisiert, in Flaschen abgefüllt und sterilisiert.

### Anwendungsbeispiel 19: Zuckerreduzierte Eiscremes

Vergleichszubereitung mit Zucker (A)
Vergleichszubereitung mit reduziertem Zuckeranteil (B)
Erfindungsgemäße Zubereitungen (C-F)

| | **Zubereitung (Gehalt in Gew.-%)** | | | | | |
|---|---|---|---|---|---|---|
| **Inhaltsstoff** | **A** | **B** | **C** | **D** | **E** | **F** |
| Pflanzenfett, Schmelzbereich 35 - 40 °C | 20,00 | 20,00 | 20,00 | 20,00 | 20,00 | 20,00 |
| Zucker (Saccharose) | 12,00 | 8,00 | 8,00 | 8,00 | 8,00 | 8,00 |
| Magermilchpulver | 5,00 | 5,00 | 5,00 | 5,00 | 5,00 | 5,00 |
| Glucosesirup 72 % Trockensubstanz | 5,00 | 5,00 | 5,00 | 5,00 | 5,00 | 5,00 |
| Emulgator SE 30 (Grindstedt Products, Dänemark) | 0,65 | 0,65 | 0,65 | 0,65 | 0,65 | 0,65 |
| Aroma, enthaltend 0,1 % Diacetyl und 1 % Vanillin | 0,20 | 0,20 | 0,20 | 0,20 | 0,20 | 0,20 |
| Balansin A | - | - | 0,01 | 0,005 | 0,01 | 0,005 |
| Extrakt aus Rubus suavissimus (z.B. von Plant-Extrakt) enthaltend 5 Gew.-% Rubusosid, bezogen auf das Gesamtgewicht des Extraktes, | - | - | - | - | - | 0,010 |
| Hesperetin 2,5%ig in 1,2-Propylenglycol | - | - | - | 0,10 | - | 0,10 |
| Phloretin 2,5% in 1,2-Propylenglycol | - | - | - | - | 0,05 | 0,05 |
| Magermilch | auf 100 % auffüllen | | | | | |

Das Pflanzenfett wurde auf 58°C erwärmt. Magermilch und Glucosesirup wurden auf 55°C erhitzt und Zucker, Magermilchpulver sowie Emulgator und Aroma hinzugefügt und die Mischung ins Pflanzenfett gegeben. Die Mischung wurde mit Hilfe eines Durchflusshochdruckhomogenisators homogenisieret (180 / 50 bar). Die erhaltene Masse wurde 1 min lang bei 78°C temperiert, anschließend auf 2 - 4 °C abgekühlt und 10 h zur Reifung bei dieser Temperatur inkubiert. Danach wurde die gereifte Masse abgefüllt und bei -18°C eingefroren gelagert.

### Anwendungsbeispiel 20: Für Diabetiker geeignetes Eis

Es wurde ein für Diabetiker geeignetes Eis aus folgenden Zutaten hergestellt und in Portionen zu je 95 mL in Becher abgefüllt:
Eingedickte entrahmte Milch, Fructosesirup, Erdbeerstücke und Erbeerpüree (15 Gew.- %), Pflanzenfett, Diätschokoladensplitter (3,5 Gew.-%, mit Emulgator Sojalecithin), Molkenerzeugnis, Rote Bete Saft, Johannisbrotkernmehl, Guarkernmehl, Carrageen, Emulgator (E 471), Gelatine, Säuerungsmittel Citronensäure, 0.1 Gew.-% Erdbeer-Aroma (enthaltend 1 Gew.-% Balansin A, bezogen auf das Gesamtgewicht des Erdbeer-Aromas), Farbstoff Carotin.
Nährwert (pro 95 mL):
Eiweiss 1,8 g, Kohlenhydrate 13,3 g (davon Fructose 9,5 g), Fett 4,2 g.

### Anwendungsbeispiel 21: Diätschokolade auf Basis von Maltit

Es wurde eine für Diabetiker geeignete Schokolade aus folgenden Zutaten hergestellt und in rechteckige Tafeln gegossen:
Maltit, Haselnussmasse, Kakaobutter, Magermilchpulver, Kakaomasse, Inulin, Butterreinfett, Emulgator Sojalecithine, 0.1 Gew.-% Vanille-Aroma (enthaltend Vanilleschoten-Extrakt, Vanillin und 5 Gew.-% Balansin A, bezogen auf das Gesamtgewicht des Vanille-Aromas).
Nährwert (pro 100 g):
Eiweiss 8 g, Kohlenhydrate 43 g (davon Maltit 34 g), Fett 34 g.

### Anwendungsbeispiel 2: Diätschokolade auf Basis von Fructose

Es wurde eine für Diabetiker geeignete Schokolade aus folgenden Zutaten hergestellt und in rechteckige Tafeln gegossen:
Kakaomasse, Fructose, Magermilchpulver, Kakaobutter, Inulin, Butterreinfett, Emulgator Sojalecithin, Walnüsse, Speisesalz, 0,1 Gew.-% Vanille-Aroma (enthaltend Vanillin und 1 Gew.-% Balansin A aus Beispiel 2, bezogen auf das Gesamtgewicht des Vanille-Aromas).
Nährwert (pro 100 g):
Eiweiss 8,8 g, Kohlenhydrate 34 g (davon Fructose 23 g, Lactose 7,5 g, Saccharose 1,4 g), Fett 36 g; Ballaststoffe 18,5 (davon 12,2 g Inulin); Natrium: 0,10 g. Kakao-Anteil mindestens 50 Gew.-%.

### Anwendungsbeispiel 23: Zuckerreduzierte Müslimischung

| Nr. | | A (Gew.-%) | B (Gew.-%) |
|---|---|---|---|
| 1 | Haferflocken | 17,00 | 18,90 |
| 2 | Knusprige Haferflocken Cluster | 10,00 | 12,00 |
| 3 | Reis Crispies | 16,90 | 17,80 |
| 4 | Cornflakes | 16,50 | 17,50 |
| 5 | Korinthen | 3,50 | 3,50 |
| 6 | Haselnüsse, zerhackt | 2,50 | 2,50 |
| 7 | Glucose Sirup aus Weizen, DE 30 | 9,50 | 9,50 |
| 8 | Saccharose | 20,00 | 14,00 |
| 9 | Wasser | 4,00 | 4,00 |
| 10 | Citronensäurepulver, wasserfrei | 0,10 | 0,10 |
| 11 | Aromakomposition D aus Anwendungsbeispiel 5 | - | 0,20 |

Jeweils Bestandteile Nr. 1 bis 6 in einer Drehtrommel mischen (Mix 1). Jeweils Bestandteile Nr. 7 bis 9 erwärmen und Bestandteil Nr. 10 zugeben (sowie in Rezeptur B zusätzlich den Bestandteil Nr. 11 zugeben) (Mix 2). Jeweils Mix 2 zu Mix 1 geben und gut mischen. Zuletzt die resultierende Müslimischung auf ein Backblech geben und in einem Ofen bei 130°C für 8 Minuten trocknen.

### Anwendungsbeispiel 24: Zuckerreduzierte Fruchtgummis

| | A (Gew.-%) | B (Gew.-%) |
|---|---|---|
| Wasser | 23,70 | 25,70 |
| Saccharose | 34,50 | 8,20 |
| Glucosesirup, DE 40 | 31,89 | 30,09 |
| Iso Syrup C* Tru Sweet 01750 (Cerestar GmbH) | 1,50 | 2,10 |
| Gelatine 240 Bloom | 8,20 | 9,40 |
| Polydextrose (Litesse^{®} Ultra, Danisco Cultor GmbH) | - | 24,40 |
| Gelber und roter Farbstoff | 0,01 | 0,01 |
| Citronensäure | 0,20 | |
| Kirscharoma, enthaltend 1 Gew.-% Balansin A und 0,3 Gew.-% Phloretin, bezogen auf das Kirscharoma | - | 0,10 |

Polydextrose ist ein selbst nicht süß schmeckendes Polysaccharid mit niedrigem Brennwert.

### Anwendungsbeispiel 25: Schoko-Cappuccino-Eiscreme

| | A (Gew.-%) | B (Gew.-%) |
|---|---|---|
| Glucose-Fructose-Sirup | 14,30 | 14,30 |
| Saccharose | 10,00 | 7,50 |
| Magermilchpulver | 5,00 | 5,00 |
| Sahne (36% Fettanteil) | 24,00 | 24,00 |
| Emulgator und Stabilisator Cremodan^{®} 709VEG (Danisco) | 0,50 | 0,50 |
| Kakaopulver | 5,975 | 5,975 |
| Carrageenan | 0,025 | 0,025 |
| Wasser | 40,20 | 42,50 |
| Cappuccino-Aroma enthaltend 1 Gew.-% Balansin B und 1 Gew.-% Homoeriodictyol-Natriumsalz, bezogen auf das Aroma | - | 0,20 |

## Patentansprüche

1. Verbindung der Formel (I) oder physiologisch akzeptables Salz einer Verbindung der Formel (I) 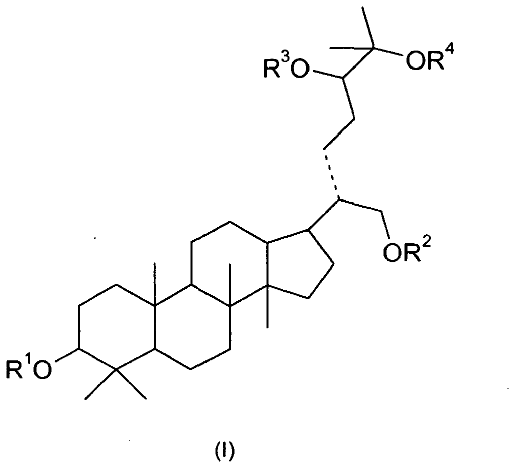 wobei
die gestrichelte Linie eine Einfach- oder eine Doppelbindung darstellt, und
R¹, R², R³ und R⁴ jeweils unabhängig voneinander Wasserstoff oder einen Zuckerrest darstellen, dabei vorzugsweise einen Monosaccharidrest oder einen Oligosaccharidrest,
wobei das Gegenkation des physiologisch akzeptablen Salzes der Verbindung der Formel (I) vorzugsweise ausgewählt ist aus der Gruppe bestehend aus einfach positiv geladenen Kationen der ersten Haupt- und Nebengruppe, Ammoniumoin, Trialkylammoniumionen, zweiwertig geladenen Kationen der zweiten Nebengruppe, dreiwertigen Kationen der dritten Haupt- und Nebengruppe, und bevorzugt ausgewählt ist aus der Gruppe bestehend aus Na⁺, K⁺, NH₄⁺, Ca²⁺, Mg²⁺, Al³⁺ und Zn²⁺.

2. Verbindung der Formel (I) oder physiologisch akzeptables Salz einer Verbindung der Formel (I) nach Anspruch 1, wobei
zumindest einer der Reste R¹, R², R³ und R⁴ Wasserstoff bedeutet, und
zumindest einer der Reste R¹, R², R³ und R⁴ einen Zuckerrest bedeutet, wobei vorzugsweise der Zuckerrest ausgewählt ist bzw. die Zuckerreste jeweils unabhängig voneinander ausgewählt sind aus der Gruppe bestehend aus
(i) den Monosaccharidresten Glucosyl, Mannosyl, Galactosyl, Rhamnosyl, Fucosyl, Arabinosyl und Ribosyl,
und
(ii) den Oligosaccharidresten aus 2 bis 10 Einfachzuckerbausteinen, wobei die Einfachzuckerbausteine vorzugsweise ausgewählt sind aus der Gruppe bestehend aus Glucose, Mannose, Galactose, Rhamnose, Fucose, Arabinose und Ribose.

3. Verbindung oder physiologisch akzeptables Salz nach einem der vorangehenden Ansprüche, wobei
zwei oder drei der Reste R², R³ und R⁴ Wasserstoff bedeuten, und
der Rest R¹ sowie optional einer der Reste R², R³ und R⁴ ein Zuckerrest bedeutet, wobei vorzugsweise der Zuckerrest ausgewählt ist bzw. die Zuckerreste jeweils unabhängig voneinander ausgewählt sind aus der Gruppe bestehend aus
(i) den Monosaccharidresten Glucopyranosyl, Mannopyranosyl, Galactopyranosyl, Rhamnopyranosyl, Fucopyranosyl, Arabinosyl und Ribosyl,
und
(ii) den Oligosaccharidresten aus 2 bis 8 Einfachzuckerbausteinen, wobei die Einfachzuckerbausteine vorzugsweise ausgewählt sind aus der Gruppe bestehend aus Glucopyranose, Mannopyranose, Galactopyranose, Rhamnopyranose, Fucopyranose, Arabinose und Ribose.

4. Verbindung oder physiologisch akzeptables Salz nach einem der vorangehenden Ansprüche, wobei die Verbindung eine Verbindung der Formel (II) oder das physiologisch akzeptable Salz ein physiologisch akzeptables Salz der Verbindung der Formel (II) 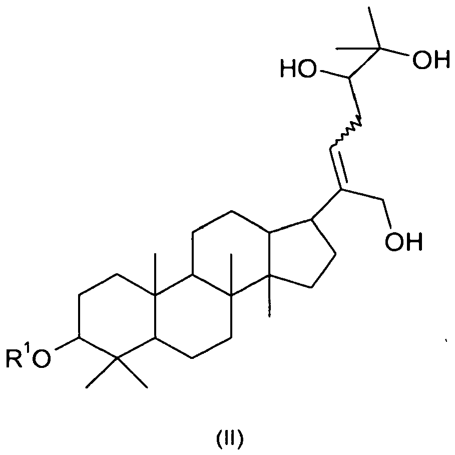 ist, wobei
R¹ ein Mono-, Di-, Tri-, Tetra- oder Pentasaccharidrest ist, vorzugsweise ausgewählt aus der Gruppe bestehend aus
(i) Glucosyl, Mannosyl, Galactosyl, Rhamnosyl, Fucosyl, Arabinosyl und Ribosyl, und
(ii) den Oligosaccharidresten aus 2 bis 5 Einfachzuckerbausteinen, wobei die Einfachzuckerbausteine ausgewählt sind aus der Gruppe bestehend aus Glucose, Mannose, Galactose, Rhamnose, Fucose, Arabinose und Ribose.

5. Verbindung oder physiologisch akzeptables Salz nach einem der vorangehenden Ansprüche, wobei die Verbindung eine Verbindung der Formel (III) oder das physiologisch akzeptable Salz ein physiologisch akzeptables Salz der Verbindung der Formel (III) 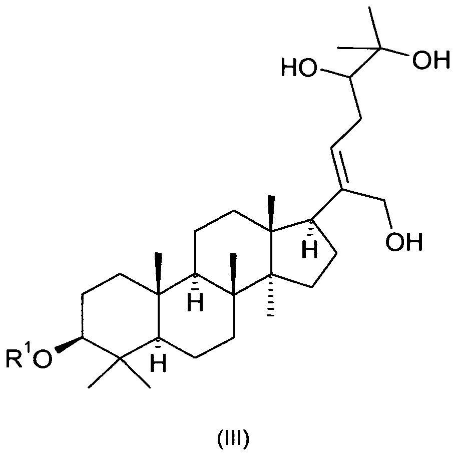 ist, wobei
R¹ ein Mono-, Di-, Tri-, Tetra- oder Pentasaccharidrest ist ausgewählt aus der Gruppe bestehend aus
(i) Glucosyl, Mannosyl, Galactosyl, Rhamnosyl, Fucosyl, Arabinosyl und Ribosyl, und
(ii) den Oligosaccharidresten aus 2 bis 5 Einfachzuckerbausteinen, wobei die Einfachzuckerbausteine ausgewählt sind aus der Gruppe bestehend aus Glucose, Mannose, Galactose, Rhamnose, Fucose, Arabinose und Ribose.

6. Verbindung der Formel (III) oder physiologisch akzeptables Salz eine Verbindung der Formel (III) nach Anspruch 5, wobei
R¹ ein Mono-, Di-, Tri-, Tetra- oder Pentasaccharidrest ist ausgewählt aus der Gruppe bestehend aus
(i) Glucosyl, Galactosyl und Rhamnosyl,
und
(ii) den Oligosaccharidresten aus 2 bis 4 Einfachzuckerbausteinen, wobei die Einfachzuckerbausteine ausgewählt sind aus der Gruppe bestehend aus Glucose, Galactose und Rhamnose, wobei vorzugsweise die Einfachzuckerbausteine ihrerseits miteinander an den Positionen 2, 4 und/oder 6 verknüpft sind.

7. Gemisch umfassend oder bestehend aus
- zwei, drei oder mehreren Verbindungen wie in einem der vorangehenden Ansprüche definiert, oder
- ein, zwei oder mehreren verschiedene Verbindungen wie in einem der vorangehenden Ansprüche definiert und einem, zwei oder mehreren verschiedenen physiologisch akzeptablen Salzen einer, zweier oder mehrerer verschiedener Verbindungen wie in einem der vorangehenden Ansprüche definiert, oder
- zwei, drei oder mehreren physiologisch akzeptablen Salzen der Verbindungen wie in einem der vorangehenden Ansprüche definiert,
wobei, sofern in dem Gemisch ein oder mehrere physiologisch akzeptable Salze einer oder mehrerer Verbindungen wie in einem der vorangehenden Ansprüche definiert vorhanden ist bzw. sind, vorzugsweise die Gegenkationen sämtlicher physiologisch akzeptabler Salze der Verbindungen wie in einem der vorangehenden Ansprüche definiert ausgewählt sind aus der Gruppe bestehend aus Na⁺, K⁺, NH₄⁺, Ca²⁺, Mg²⁺, Al³⁺ und Zn²⁺.

8. Pflanzlicher Extrakt, vorzugsweise pflanzlicher Extrakt von *Mycetia balansae,* umfassend eine oder mehrere Verbindungen wie in einem der Ansprüche 1 bis 6 definiert oder ein physiologisch akzeptables Salz einer Verbindung wie in einem der Ansprüche 1 bis 6 definiert, oder ein Gemisch nach Anspruch 7,
wobei vorzugsweise die Gesamtmenge dieser Verbindungen und der physiologisch akzeptablen Salze dieser Verbindungen im Bereich von 0,00001 bis 99 Gew.-% liegt, bevorzugt im Bereich von 0,0001 bis 95 Gew.-%, besonders bevorzugt im Bereich von 0,0005 bis 80 Gew.-%, insbesondere bevorzugt im Bereich von 0,001 bis 30 Gew.-%, bezogen auf die Trockenmasse des Extraktes.

9. Extrakt nach Anspruch 8, erhältlich oder erhalten durch ein Verfahren mit folgendem Schritt:
a) ein- oder mehrfache Extraktion von pflanzlichem Material von *Mycetia balansae* mit einem, vorzugsweise flüssigen, Extraktionsmittel ausgewählt aus der Gruppe bestehend aus Wasser, Ethanol, Methanol, 1-Propanol, 2-Propanol, Glycerin, 1,2-Propandiol, superkritischem Kohlendioxid, Essigsäureethylester und deren Mischungen,
sowie gegebenenfalls einem oder mehreren weiteren der folgenden Schritte:
b) gegebenenfalls Aufkonzentrierung des in Schritt a) erhaltenen Primärextraktes, vorzugsweise durch ein oder mehrere evaporative oder pervaporative Verfahren,
c) gegebenenfalls Behandlung des gegebenenfalls in Schritt b) aufkonzentrierten Primärextraktes mit oder an Adsorbentien, vorzugsweise ausgewählt aus der Gruppe bestehend aus Kieselgel, modifiziertes Kieselgel, Aktivkohle, Zeolith, Bentonit, Kieselgur, Aluminiumoxid, basischer oder saurer oder neutraler, optional makroporöser, lonentauscher, bevorzugt im Batch- oder Säulenverfahren, gegebenenfalls unter Zuhilfenahme weiterer Extraktionsmittel, wodurch ein aufgereinigter Extrakt (Sekundärextrakt) erhalten wird,
d) gegebenenfalls Trocknung des in Schritt c) erhaltenen Sekundärextraktes, vorzugsweise durch ein evaporatives oder pervaporatives Verfahren,
e) gegebenenfalls Mischen des in Schritt d) erhaltenen getrockneten Sekundärextraktes mit einem geeigneten Verdünnungsmittel oder einem Gemisch zweier oder mehrerer Verdünnungsmittel, bevorzugt ausgewählt aus der Gruppe bestehend aus Ethanol, Isopropanol, 1,2-Propylenglycol, Pflanzenöltriglyceriden, Diacetin, Triacetin und Glycerin, wobei vorzugsweise eine Lösung erhalten wird.

10. Oral konsumierbare Zubereitung, umfassend eine sensorisch wirksame Menge einer Verbindung wie in einem der Ansprüche 1 bis 6 definiert bzw. eines physiologisch akzeptablen Salzes einer Verbindung wie in einem der Ansprüche 1 bis 6 definiert, eines Gemisches nach Anspruch 7, und/oder eines Extraktes nach Anspruch 8 oder 9, wobei die oral konsumierbare Zubereitung vorzugsweise ausgewählt ist aus der Gruppe bestehend aus der Ernährung dienenden Zubereitungen, der Nahrungsergänzung dienenden Zubereitungen, dem Genuss dienenden Zubereitungen, den oralen pharmazeutischen Zubereitungen, den Mundpflegeprodukten, den Aromakompositionen und den kosmetischen Zubereitungen.

11. Oral konsumierbare Zubereitung nach Anspruch 10, zusätzlich umfassend ein, zwei, drei, vier, fünf, sechs, sieben, acht, neun, zehn oder mehrere weitere Stoffe, ausgewählt aus den folgenden Gruppen (a1) bis (a5):
(a1) Aromastoffe, wobei vorzugsweise ein, zwei, drei, vier, fünf oder mehrere der Aromastoffe ausgewählt sind aus der Gruppe bestehend aus: Vanillin, Ethylvanillin, 2-Hydrox-4-methoxybenzaldehyd, Ethylvanillinisobutyrat (= 3-Ethoxy-4-isobutyryloxybenzaldehyd), Furaneol^{®} (2,5-Dimethyl-4-hydroxy-3(2H)-furanon) und Abkömmlinge (z.B. Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und dessen Abkömmlinge (z.B. Ethylmaltol), Cumarin und dessen Abkömmlinge, gamma-Lactone (z.B. gamma-Undecalacton, gamma-Nonalacton), delta-Lactone (z.B. 4-Methyldeltalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)furanon, Fruchtester und Fruchtlactone (z.B. Essigsäure-n-butylester, Essigsäureisoamylester, Propionsäureethylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat), 4-(p-Hydroxyphenyl)-2-butanon, 1,1-Dimethoxy-2,2,5-trimethyl-4-hexan, 2,6-Dimethyl-5-hepten-1-al und Phenylacetaldehyd;
(a2) Kohlenhydrate ausgewählt aus der Gruppe bestehend aus Saccharose, Trehalose, Lactose, Maltose, Melizitose, Melibiose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glyceraldehyd, Maltodextrine und pflanzliche Zubereitungen enthaltend einen oder mehrere der genannten Kohlenhydrate, vorzugsweise in einem Anteil von zumindest 5 Gew. %, bevorzugt zumindest 15 Gew.-%, wobei die Kohlenhydrate auch als natürlich vorkommende oder künstlich hergestellte Mischung vorliegen können, dabei insbesondere als Honig, Invertzuckersirup oder hochangereicherter Fructose-Sirup aus Maisstärke, und den physiologisch akzeptablen Salzen dieser Kohlenhydrate, insbesondere den Natrium-, Kalium-, Calcium oder Ammoniumsalzen;
(a3) Zuckeralkohole, vorzugsweise natürlich vorkommende Zuckeralkohole ausgewählt aus der Gruppe bestehend aus Glycerin, Erythritol, Threitol, Arabitol, Ribitol, Xylitol, Sorbitol, Mannitol, Maltitol, Isomaltit, Dulcitol, Lactitol, und den physiologisch akzeptablen Salzen dieser Zuckeralkohole, insbesondere den Natrium-, Kalium-, Calcium oder Ammoniumsalzen;
(a4) natürlich vorkommende Süßstoffe, vorzugsweise ausgewählt aus der Gruppe bestehend aus
(a4-1)Miraculin, Monellin, Mabinlin, Thaumatin, Curculin, Brazzein, Pentaidin, D-Phenylalanin, D-Tryptophan, und aus natürlichen Quellen gewonnene Extrakte oder Fraktionen, enthaltend diese Aminosäuren und/oder Proteine, und den physiologisch akzeptablen Salzen dieser Aminosäuren und/oder Proteine, insbesondere den Natrium-, Kalium-, Calcium oder Ammoniumsalzen;
(a4-2)Neohesperidindihydrochalkon, Naringindihydrochalkon, Steviosid, Steviolbiosid, Rebaudiosiden, insbesondere Rebaudiosid A, Rebaudiosid B, Rebaudiosid C, Rebaudiosid D, Rebaudiosid E, Rebaudiosid F, Rebaudiosid G, Rebaudiosid H, Dulcosiden und Rubusosid, Suavioside A, Suavioside B, Suavioside G, Suavioside H, Suavioside I, Suavioside J, Baiyunoside 1 Baiyunoside 2, Phlomisoside 1, Phlomisoside 2, Phlomisoside 3, sowie Phlomisoside 4, Abrusoside A, Abrusoside B, Abrusoside C, Abrusoside D, Cyclocaryoside A und Cyclocaryoside I, Oslandin, Polypodosid A, Strogin 1, Strogin 2, Strogin 4, Selligueanin A, Dihydroquercetin-3-acetat, Perillartin, Telosmosid A₁₅, Periandrin I-V, Pterocaryosiden, Cyclocaryosiden, Mukuroziosiden, trans-Anethol, trans-Cinnamaldehyd, Bryosiden, Bryonosiden, Bryonodulcosiden, Carnosiflosiden, Scandenosiden, Gypenosiden, Trilobatin, Phloridzin, Dihydroflavanolen, Hematoxylin, Cyanin, Chlorogensäure, Albiziasaponin, Telosmosiden, Gaudichaudiosid, Mogrosiden, Mogrosid V, Hernandulcinen, Monatin, Phyllodulcin, Glycyrrhetinsäure und deren Derivaten, insbesondere deren Glycosiden wie Glycyrrhizin, und den physiologisch akzeptablen Salzen dieser Verbindungen, insbesondere den Natrium-, Kalium-, Calcium oder Ammoniumsalzen;
(a4-3) Extrakte oder angereicherte Fraktionen der Extrakte, ausgewählt aus der Gruppe bestehend aus Thaumatococcus-Extrakten (Katemfestaude), Extrakten aus *Stevia* ssp. (insbesondere *Stevia rebaudiana*), Swingle-Extrakten (*Momordica* bzw. *Siratia grosvenorii,* Luo-Han-Guo), Extrakten aus *Glycerrhyzia* ssp. (insbesondere *Glycerrhyzia glabra*), Extrakten aus *Rubus* ssp. (insbesondere *Rubus suavissimus*), Citrus-Extrakten und Extrakten aus *Lippia dulcis;*
(a5) synthetische süß schmeckende Stoffe, vorzugsweise ausgewählt aus der Gruppe bestehend aus Magap, Natriumcyclamat oder anderen physiologisch akzeptablen Salzen der Cyclamsäure, Acesulfam K oder anderen physiologisch akzeptablen Salzen, Neohesperidindihydrochalkon, Naringindihydrochalkon, Saccharin, Saccharin-Natriumsalz, Aspartam, Superaspartam, Neotam, Alitam, Advantam, Perillartin, Sucralose, Lugduname, Carrelame, Sucrononate und Sucrooctate.

12. Oral konsumierbare Zubereitung nach einem der Ansprüche 10 oder 11, wobei die Gesamtmenge an Verbindungen der Formel (I) im Bereich von 0,01 ppm bis 95 Gew.-% liegt, vorzugsweise im Bereich von 0,1 ppm bis 90 Gew.-%, bevorzugt im Bereich von 1 ppm bis 50 Gew.-%, weiter bevorzugt im Bereich von 1 ppm bis 20 Gew.-%, insbesondere bevorzugt im Bereich von 1 ppm bis 5 Gew.-%, bezogen auf die Gesamtmasse der Zubereitung.

13. Verfahren zum (a) Vermitteln eines süßen Geschmackseindrucks und/oder Verstärken eines süßen Geschmackseindrucks eines, zweier oder mehrerer süß schmeckender Stoffe und/oder (b) Herstellen einer oral konsumierbaren Zubereitung nach einem der Ansprüche 10 bis 12, mit folgenden Schritten:
a) Bereitstellen einer Verbindung wie in einem der Ansprüche 1 bis 6 definiert bzw. eines physiologisch akzeptablen Salzes einer Verbindung wie in einem der Ansprüche 1 bis 6 definiert, eines Gemisches nach Anspruch 7 und/oder eines Extraktes nach Anspruch 8 oder 9,
b) Bereitstellen einer oral konsumierbaren Zubereitung, vorzugsweise umfassend einen, zwei oder mehrere weitere süß schmeckende Stoffe,
c) in Kontakt bringen oder Vermischen der in Schritt a) und b) bereitgestellten Bestandteile.

14. Verfahren nach Anspruch 13, umfassend die Schritte
a-i) Herstellen eines Extraktes, vorzugsweise eines Extraktes nach Anspruch 8 oder 9,
umfassend eine Verbindung wie in einem der Ansprüche 1 bis 6 definiert, eines physiologisch akzeptablen Salzes einer Verbindung wie in einem der Ansprüche 1 bis 6 definiert, oder eines Gemisches nach Anspruch 7,
durch Extrahieren von pflanzlichem Material, vorzugsweise pflanzlichem Material von *Mycetia balansae;*
a-ii) optional Weiterverarbeiten des in Schritt (a-i) hergestellten Extraktes zu einem weiterverarbeiteten Produkt umfassend eine Verbindung wie in einem der Ansprüche 1 bis 6 definiert, eines physiologisch akzeptablen Salzes einer Verbindung wie in einem der Ansprüche 1 bis 6 definiert, eines Gemisches nach Anspruch 7,
b) Bereitstellen einer oral konsumierbaren Zubereitung, vorzugsweise umfassend einen, zwei oder mehrere weitere süß schmeckende Stoffe,
c) in Kontakt bringen oder Vermischen der oral konsumierbaren Zubereitung aus Schritt b), welche vorzugsweise einen, zwei oder mehrere weitere süß schmeckende Stoffe umfasst, mit dem in Schritt a-i) hergestellten Extrakt bzw. dem in Schritt a-ii) hergestellten weiterverarbeiteten Produkt.

15. Verwendung einer Verbindung wie in einem der Ansprüche 1 bis 6 definiert bzw. eines physiologisch akzeptablen Salzes einer Verbindung wie in einem der Ansprüche 1 bis 6 definiert, eines Gemisches nach Anspruch 7, oder eines Extraktes nach Anspruch 8 oder 9
zum Erzeugung eines Süßeindruckes in einer oral konsumierbaren Zubereitung oder zur Verstärkung des Süßeindruckes einer oral konsumierbaren Zubereitung umfassend mindestens einen weiteren, vorzugsweise natürlich vorkommenden, süß schmeckenden Stoff.

## Claims

1. Compound of formula (I) or physiologically acceptable salt of a compound of formula (I) 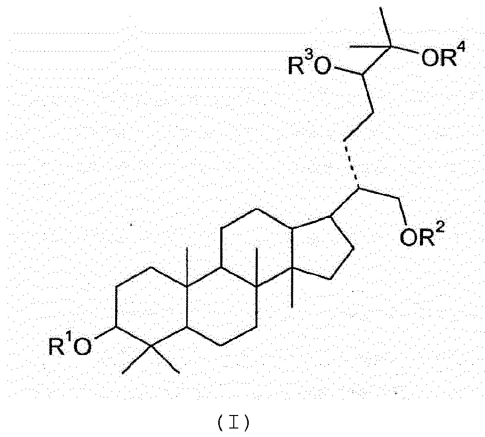 wherein
the dotted line represents a single or a double bond, and
R¹, R², R³ and R⁴ each independently from one another represents hydrogen or a sugar moiety, whereby preferably a monosaccharide or an oligosaccharide moiety,
wherein the counter-cation of the physiologically acceptable salt of the compound of formula (I)is preferably selected from the group consisting of single positive charged cation of the first main group and subgroup, ammonium ion, trialkylammonium ions, divalent charged cation of the second subgroup, trivalent cation of the third main group and subgroup, and preferably selected from the group consisting of Na⁺, K⁺, NH₄⁺, Ca²⁺, Mg²⁺, Al³⁺ and Zn²⁺.

2. Compound of formula (I) or physiologically acceptable salt of a compound of formula (I)according to claim 1, wherein
at least one of the moieties R¹, R², R³ and R⁴ represents hydrogen, and
at least one of the moieties R¹, R², R³ and R⁴ represents a sugar moiety, wherein the sugar moiety is preferably selected from, respectively the sugar moieties are independently selected from the group consisting of
(i) the monosaccharide moieties glucosyl, mannosyl, galactosyl, rhamnosyl, fucosyl, arabinosyl and ribosyl,
and
(ii) the oligosaccharide moieties of 2 to 10 monosaccharides components, wherein the monosaccharide components are preferably selected from the group consisting of glucose, mannose, galactose, rhamnose, fucose, arabinose and ribose.

3. Compound or physiologically acceptable salt according to any of the preceding claims, wherein
two or three of the moieties R², R³ and R⁴ represent a hydrogen, and
the moiety R¹ plus optionally one of the moieties R², R³ and R⁴ represent a sugar moiety, wherein preferably the sugar moiety is selcetd from, respectively the sugar moieties are independently selected from the group consisting of
(i) the monosaccharide moieties glucopyranosyl, mannopyranosyl, galactopyranosyl, rhamnopyranosyl, fucopyranosyl, arabinosyl and ribosyl,
and
(ii) the oligosaccharide moieties of 2 to 8 monosaccharide components, wherein the monosaccharide components are preferably selected from the group consisting of glucopyranose, mannopyranose, galactopyranose, rhamnopyranose, fucopyranose, arabinose and ribose.

4. Compound or physiologically acceptable salt according to any of the preceding claims, wherein the compound is a compound of formula(II) or the physiologically acceptable salt is a physiologically acceptable salt of the compound of formula (II) 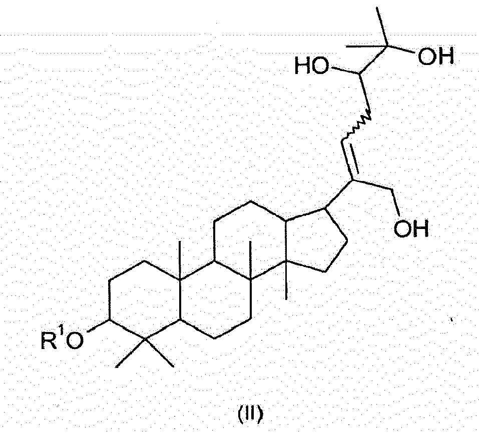 wherein
R¹ is a mono-, di-, tri-, tetra-, or pentasaccharide moiety, preferably selected from the group consisting of
(i) glucosyl, mannosyl, galactosyl, rhamnosyl, fucosyl, arabinosyl and ribosyl,
and
(ii) the oligosaccharide moieties of 2 to 5 monosaccharide components, wherein the monosaccharide components are preferably selected from the group consisting of glucose, mannose, galactose, rhamnose, fucose, arabinose and ribose.

5. Compound or physiologically acceptable salt according to any of the preceding claims, wherein the compound is a compound of formula(III) or the physiologically acceptable salt of a physiologically acceptable salt of the compound of formula(III) 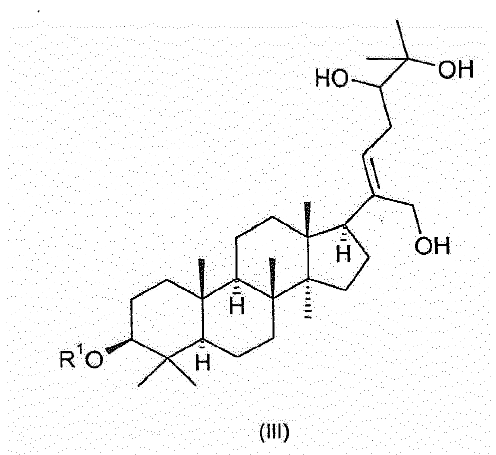 wherein
R¹ is a mono-, di-, tri-, tetra- or pentasaccharide moiety selected from the group consisting of
(i) glucosyl, mannosyl, galactosyl, rhamnosyl, fucosyl, arabinosyl and ribosyl,
and
(ii) the oligosaccharide moieties of 2 to 5 monosaccharide components, wherein the monosaccharide components are preferably selected from the group consisting of glucose, mannose, galactose, rhamnose, fucose, arabinose and ribose.

6. Compound of formula (III) or physiologically acceptable salt of a compound of formula (III) according to claim 5, wherein
R¹ is a mono-, di-, tri-, tetra- or pentasaccharide moiety selected from the group consisting of
(i) glucosyl, galactosyl and rhamnosyl,
and
(ii) the oligosaccharide moieties of 2 to 4 monosaccharide components, wherein the monosaccharide components are selected from the group consisting of glucose, galactose, rhamnose, wherein preferably the monosaccharide components on their part are linked together at the positions 2,4, and/or 6.

7. Mixture, comprising or consisting of
- two, three or more compounds as defined in any of the preceding claims, or
- one, two or more different compounds as defined in any of the preceding claims and one, two or more different physiologically acceptable salts of one, two or more different compounds as defined in any of the preceding claims, or
- two, three or more physiologically acceptable salts of the compounds as defined in any of the preceding claims,
wherein, as long as in the mixture one or more physiologically acceptable salts of one or more compounds as defined in any of the preceding claims is or are present, preferably the counter-cations of all physiologically acceptable salts of the compounds as defined in any of the preceding claims are selected from the group consisting of Na⁺, K⁺, NH₄⁺, Ca²⁺, Mg²⁺, Al³⁺ and Zn²⁺.

8. Plant extract, preferably plant extract from *Mycetia balansae,* comprising one or more compounds as defined in any of the preceding claims 1 to 6 or a physiological acceptable salt of a compound as defined in any of the preceding claims 1 to 6, or a mixture according to claim 7,
wherein, preferably the total amount of these compounds and the physiological acceptable salts of these compounds are in the range from 0,00001 to 99 wt.%, preferably in the range from 0,0001 to 95 wt.%, particularly preferred in the range from 0,0005 to 80 wt.%, more praticularly preferred in the range from 0,001 to 30 wt.%,referring to dry solid of the extract.

9. Extract according to claim 8, obtainable or obtained according to a process comprising the following step:
a) one time or manifold extraction of plant materials from *Mycetia balansae* with, preferably a liquid eluent selected from the group consisting of water, ethanol, methanol 1-propanol, 2-propanol, glycerole, 1,2-propanediol, supercritical carbon dioxide, ethyl acetate and the mixture thereof, as well as when applicable one or more of the following steps :
b) when applicable, concentration of the in step a) obtained primary extract, preferably through one or more evaporative or pervaporative procedure,
c) when applicable, treatment of the optionally in step b) obtained concentrated primary extract with or on adsorbent materials, preferably selected from the group consisting of silicagel, modified silicagel, active carbon, zeolite, bentonite,kieselguhr, aluminium oxide, alkaline or acidic or neutral, optionally macro porous ion exchanger, preferably in batch- or column process, when applicable, with the help of additional eluent, whereby a purified extract (secondary extract) is obtained,
d) when applicable, dehydration of the in step c) obtained secondary extract, preferably through a evaporative or pervaporative process,
e) when applicable, mixing of the in step d) dried secondary extract with a suitable diluent or a mixture of two or more diluents, preferably selected from the group consisting of ethanol, isopropanol, 1,2-propylenglycol, vegetable oil triglycerides, diacetin, triacetin and glycerine, whereby preferably a solution is obtained.

10. Oral consumable preparation, comprising a sensory effective amount of a compound as defined in any of the claims 1 to 6, respectively a physiologically acceptable salt of a compound as defined in any of the claims 1 to 6, a mixture according to claim 7,and / or an extract according to claim 8 or 9,
whereby the oral consumable preparation is preferably selected from the group consisting of the nutrition alimentary preparations, the dietary supplement serving preparations, the consumption serving preparations, the oral pharmaceutical preparations, the oral hygiene products, the aroma compositions and the cosmetical preparations.

11. Oral consumable preparation according to claim 10, additionally comprising one, two, three, four, five, six, seven, eight, nine, ten or more further substances selected from the groups (a1) to (a5):
(a1) flavouring compounds, whereby preferably one, two, three, four, five or more of the flavouring compounds are selected from the group consisting of : vanilline, ethylvanillin, 2-hydroxy-4-méthoxybenzaldehyde, ethylvanillin isobutyrate (= 3-ethoxy-4-isobutyryloxybenzaldehyde), Furaneol^{®} (2,5-dimethyl-4-hydroxy-3(2H)-furanone) and derivatives (e.g. homofuraneol, 2-ethyl-4-hydroxy-5- methyl-3(2H)-furanone), homofuronol (2-ethyl-5-methyl-4-hydroxy-3(2H)-furanone and 5-ethyl-2-methyl-4-hydroxy-3(2H)-furanone), maltol and its derivatives (e.g. ethylmaltol), coumarine and its derivatives, gamma-lactone (e.g. gamma-undecalactone, gamma-nonalactone), delta-lactone (e.g. 4-methyldeltalactone, massoilactone, deltadecalactone, tuberolactone), methyl sorbate, la divanillin, 4-hydroxy-2(or 5)-ethyl-5(or 2)-methyl-3(2H)furanone, 2-hydroxy-3-methyl-2-cyclopentenone, 3-hydroxy-4,5-dimethyl-2(5H)-furanone, fruitester and fruitlactones (e.g. acetic acid-n-butylester, isoamylacetate, ethyl propionate, ethyl butyrate, n-butyl butyrate de, isoamyl butyrate, 3-methyl-ethylbutyrate, ethylhexanoate, n-hexanoic acid allylester, n-hexanoic acid-n- butylester, ethyloctanoate, ethyl-3-methyl-3-phenylglycidate, ethyl-2-trans-4-cis-decadienoate), 4-(p-hydroxyphenyl)-2-butanone, 1,1-dimethoxy-2,2,5-trimethyl-4-hexane, 2,6-dimethyl-5-hepten-l-al and phenylacetaldehyde;
(a2) carbohydrates selected from the group consisting of saccharose, trehalose, lactose, maltose, melizitose, melibiose, raffinose, palatinose, lactulose, D-fructose, D-glucose, D-galactose, L-rhamnose, D-sorbose, D-mannose, D-tagatose, D-arabinose, L-arabinose, D-ribose, D-glyceraldehyde, maltodextrines and herbal preparations consisting of one or more of the said carbohydrates, preferably in a ratio from at least 5 wt.-%, preferably at least 15 wt.-%, whereby the carbohydrates may be also available as natural sources or as artfificial manufactured mixture, especially present as honey, invert sugar syrup or high-concentrated fructose-syrup from corn starch, and the physiologically acceptable salts of these carbohydrates, in particular the sodium, potassium, calcium or ammonium salts;
(a3) sugar alcohols, preferably naturally occurring sugar alcohols selected from the group consisting of glycerole, erythritol, threitol, arabitol, ribitol, xylitol, sorbitol, mannitol, maltitol, isomaltitol, dulcitol, lactitol, and the physiologically acceptable salts of these sugar alcohols, in particular the sodium, potassium, calcium or ammonium salts;
(a4) naturally occurring sweetener agent, preferably selected from the group consisting of
(a4-1) miraculin, monellin, mabinlin, thaumatin, curculin, brazzein, pentaidin, D-phenylalanine, D-tryptophan, and extracts or fractions attained from natural sources, consisting of these amino acids and/ or proteins, and the physiologically acceptable salts of these amino acids and / or proteins, in particular the sodium, potassium, calcium or ammonium salts;
(a4-2) neohesperidin dihydrochalcone, naringin dihydrochalcone, steviol glycoside, steviolbioside, rebaudiosides, in particular rebaudioside A, rebaudioside B, rebaudioside C, rebaudioside D, rebaudioside E, rebaudioside F, rebaudioside G, rebaudioside H, dulcosides, suavioside A, suavioside B, suavioside G, suavioside H, suavioside I, suavioside J, baiyunoside 1, baiyunoside 2, phlomisoside 1, phlomisoside 2, phlomisoside 3, as well as phlomisoside 4, abrusoside A, abrusoside B, abrusoside C, abrusoside D, cyclocaryoside A and cyclocaryoside I, oslandine, polypodoside A, strogine 1, strogine 2, strogine 4, selligueanin A, dihydroquercetine-3-acetate, perillartine, telosmoside A₁₅, periandrine I-V, pterocaryosides, cyclocaryosides, mukuroziosides, trans-anethole, trans-cinnamaldehyde, bryosides, bryonosides, bryonodulcosides, carnosiflosides, scandenosides, gypenosides, trilobatine, phloridzine, dihydroflavanols, hematoxyline, cyanine, chlorogenic acid, albiziasaponine, telosmosides, gaudichaudioside, mogrosides, mogroside V, hernandulcines, monatine, phyllodulcine, glycyrrhetinic acid and its derivatives, in particular their glycosides like glycyrrhizin, and the physiologically acceptable salts of these components, in particular the sodium, potassium, calcium or ammonium salts;
(a4-3) extracts or enriched fractions of the extracts, selected from the group consisting of *Thaumatococcus* extracts(katemfe), extracts from *Stevia ssp.* (in particuar *Stevia rebaudiana*), swingle-extracts (*Momordica* and *Siratia grosvenorii,* Luo-Han-Guo), extracts from *Glycerrhyzia* ssp. (in particular *Glycerrhyzia glabra*), extracts from *Rubus ssp.* (in particuar *Rubus suavissimus*), citrus-extracts and extracts from *Lippia dulcis;*
(a5) synthetically sweet tasting substances, preferably selected from the group consisting of magap, sodium cyclamate, or other physiologically acceptable salts of the cyclamatic acid, acesulfame K or other physiologically acceptable salts, neohesperidin dihydrochalcone, naringin dihydrochalcone, saccharin, saccharin-sodium saltsel, super-aspartame, neotame, alitame, advantame, perillartine, sucralose, lugduname, carrelame, sucrononate and sucrooctate.

12. Oral consumable preparation according to any of the claims 10 or 11, wherein the total amount of the component of the formula (I) is in the range from 0,01 ppm to 95 wt.-%, preferably in the range from 0,1 ppm to 90 wt.-%, preferably in the range from 1 ppm to 50 wt.-%, further preferably in the range from 1 ppm to 20 wt.-%, particularly preferred in the range from 1 ppm to 5 wt.-%, referring to the total mass of the preparation.

13. Method of (a) imparting a sweet impression taste and/or enhancing a sweet impression taste of one, two or several sweet tasting substances and/or (b) producing an oral consumable preparation according to any of the claims 10 to 12, with the following steps :
a) providing a compound as defined in any of the claims 1 to 6, respectively a physiologically acceptable salt of a compound as defined in any of the claims 1 to 6, a mixture according to claim 7 and/or an extract according to claim 8 or 9,
b) providing an oral consumable preparation, preferably comprising one, two or more further sweet tasting substances,
c) bringing in contact or mixing of the provided component from step a) and b).

14. Method according to claim 13, comprising the steps
a-i) producing of an extract, preferably an extract according to claim 8 or 9,
comprising a compound as defined in any of the claims 1 to 6, a physiologically acceptable salt of a compound as defined in any of the claims 1 to 6, or a mixture according to claim 7,
through extraction of plant material, preferably plant material from *Mycetia balansae ;*
a-ii) optionally, subsequent processing of the in step (a-i) produced extract to a subsequent processed product, comprising a compound as defined in any of the claims 1 to 6, a physiologically acceptable salt of a compound as defined in any of the claims 1 to 6, or a mixture according to claim 7,
b) providing an oral consumable preparation, preferably comprising one, two or more further sweet tasting substances,
c) bringing in contact or mixing the oral consumable preparation from step b), which preferably comprises one, two, or more of the further sweet tasting substances, with the produced extract from step a-i), respectively with the subsequent processed product from step a-ii).

15. Use of a compound as defined in any of the claims 1 to 6, respectively a physiologically acceptable salt of a compound as defined in any of the claims 1 to 6, or a mixture according to claim 7, or an extract according to claim 8 or 9,
for the generation of a sweet impresseion in an oral consumable preparation or for the enhancement of the sweet impression of an oral consumable preparation, comprising at least an additional, preferably natural ocurring sweet tasting substance.

## Revendications

1. Composé de formule (I) ou sel physiologiquement acceptable d'un composé de formule (I) 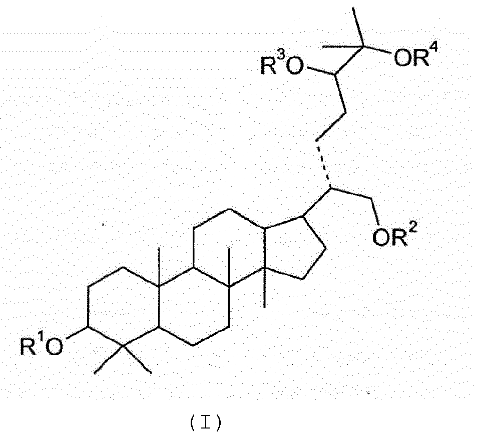 dans laquelle
le tireté représente une liaison simple ou une liaison double et
R¹, R², R³ et R⁴ représentent chacun indépendamment les uns des autres un atome d'hydrogène ou un radical glucidique, dont de préférence un radical monosaccharidique ou un radical oligosaccharidique,
le cation opposé du sel physiologiquement acceptable du composé de formule (I) étant choisi de préférence dans le groupe constitué par des cations à une seule charge positive des groupes IA et IB, l'ion ammonium, les ions trialkylammonium, des cations à deux charges du groupe IIB, des cations trivalents des groupes IIIA et IIIB, et est choisi de façon particulièrement préférée dans le groupe constitué par Na⁺, K⁺, NH₄⁺, Ca²⁺, Mg²⁺, Al³⁺ et Zn²⁺.

2. Composé de formule (I) ou sel physiologiquement acceptable d'un composé de formule (I) selon la revendication 1, dans lequel
au moins l'un des radicaux R¹, R², R³ et R⁴ représente un atome d'hydrogène, et
au moins l'un des radicaux R¹, R², R³ et R⁴ représente un radical glucidique, de préférence le radical glucidique étant choisi ou les radicaux glucidiques étant choisis chacun indépendamment les uns des autres dans le groupe constitué par
(I) les radicaux monosaccharidiques glucosyle, mannosyle, galactosyle, rhamnosyle, fucosyle, arabinosyle et ribosyle,
et
(II) les radicaux oligosaccharidiques constitués de 2 à 10 composants monoglucidiques, les composants monoglucidiques étant choisis de préférence dans le groupe constitué par le glucose, le mannose, le galactose, le rhamnose, le fucose, l'arabinose et le ribose.

3. Composé ou sel physiologiquement acceptable selon l'une quelconque des revendications précédentes, dans lequel
deux ou trois des radicaux R², R³ et R⁴ représentent des atomes d'hydrogène, et
le radical R¹ ainsi qu'en option l'un des radicaux R², R³ et R⁴ représente(nt) un radical glucidique, de préférence le radical glucidique étant choisi ou les radicaux glucidiques étant choisis chacun indépendamment les uns des autres dans le groupe constitué par
(I) les radicaux monosaccharidiques glucopyranosyle, mannopyranosyle, galactopyranosyle, rhamno-pyranosyle, fucopyranosyle, arabinosyle et ribosyle,
et
(II) les radicaux oligosaccharidiques constitués de 2 à 8 composants monoglucidiques, les composants monoglucidiques étant de préférence choisis dans le groupe constitué par le glucopyranose, le mannopyranose, le galactopyranose, le rhamnopyranose, le fucopyranose, l'arabinose et le ribose.

4. Composé ou sel physiologiquement acceptable selon l'une quelconque des revendications précédentes, le composé étant un composé de formule (II) ou le sel physiologiquement acceptable étant un sel physiologiquement acceptable du composé de formule (II) 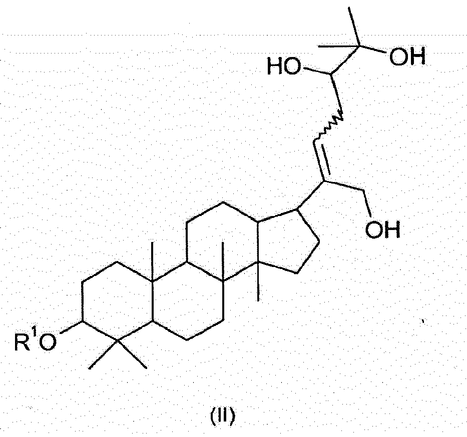 dans laquelle
R¹ est un radical mono-, di-, tri-, tétra- ou penta-saccharidique, de préférence choisi dans le groupe constitué par
(I) les radicaux glucosyle, mannosyle, galactosyle, rhamnosyle, fucosyle, arabinosyle et ribosyle,
et
(II) les radicaux oligosaccharidiques constitués de 2 à 5 composants monoglucidiques, les composants monoglucidiques étant choisis dans le groupe constitué par le glucose, le mannose, le galactose, le rhamnose, le fucose, l'arabinose et le ribose.

5. Composé ou sel physiologiquement acceptable selon l'une quelconque des revendications précédentes, le composé étant un composé de formule (III) ou le sel physiologiquement acceptable étant un sel physiologiquement acceptable du composé de formule (III) 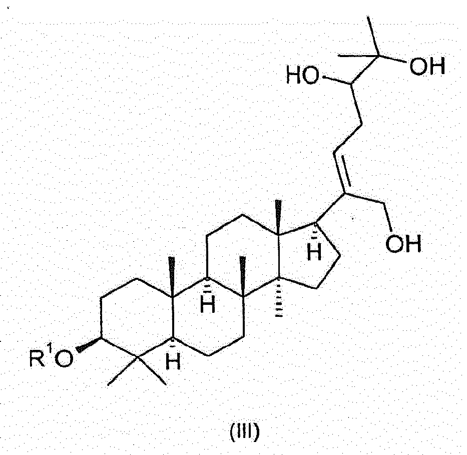 dans laquelle
R¹ est un radical mono-, di-, tri-, tétra- ou penta-saccharidique, choisi dans le groupe constitué par
(I) les radicaux glucosyle, mannosyle, galactosyle, rhamnosyle, fucosyle, arabinosyle et ribosyle,
et
(II) les radicaux oligosaccharidiques constitués de 2 à 5 composants monoglucidiques, les composants monoglucidiques étant choisis dans le groupe constitué par le glucose, le mannose, le galactose, le rhamnose, le fucose, l'arabinose et le ribose.

6. Composé de formule (III) ou sel physiologiquement acceptable d'un composé de formule (III) selon la revendication 5, où
R¹ est un radical mono-, di-, tri-, tétra- ou penta-saccharidique, de préférence choisi dans le groupe constitué par
(I) les radicaux glucosyle, galactosyle et rhamnosyle,
et
(II) les radicaux oligosaccharidiques constitués de 2 à 4 composants monoglucidiques, les composants monoglucidiques étant choisis dans le groupe constitué par le glucose, le galactose et le rhamnose, de préférence les composants monoglucidiques étant pour leur part rattachés les uns aux autres au niveau des positions 2, 4 et/ou 6.

7. Mélange comprenant ou consistant en
- deux, trois ou plus de trois composés tels que définis dans l'une quelconque des revendications précédentes, ou
- un, deux ou plus de deux composés différents tels que définis dans l'une quelconque des revendications précédentes et un, deux ou plus de deux sels différents, physiologiquement acceptables, d'un, de deux ou de plus de deux composés différents tels que définis dans l'une quelconque des revendications précédentes, ou
- deux, trois ou plus de trois sels physiologiquement acceptables des composés tels que définis dans l'une quelconque des revendications précédentes,
lorsqu'un ou plusieurs sels physiologiquement acceptables d'un ou de plusieurs composés tels que définis dans l'une quelconque des revendications précédentes est ou sont présents dans le mélange, de préférence les cations opposés de tous les sels physiologiquement acceptables des composés tels que définis dans l'une quelconque des revendications précédentes étant choisis dans le groupe constitué par Na⁺, K⁺, NH₄⁺, Ca²⁺, Mg²⁺, Al³⁺ et Zn²⁺.

8. Extrait végétal, de préférence extrait végétal de *Mycetia balansae,* comprenant un ou plusieurs composés tels que définis dans l'une quelconque des revendications 1 à 6 ou un sel physiologiquement acceptable d'un composé tel que défini dans l'une quelconque des revendications 1 à 6, ou un mélange selon la revendication 7,
de préférence la quantité totale de ces composés et des sels physiologiquement acceptables de ces composés se situant dans la plage de 0,00001 à 99 % en poids, encore mieux dans s la plage de 0,0001 à 95 % en poids, de façon particulièrement préférée dans la plage de 0,0005 à 80 % en poids, de façon plus particulièrement préférée dans la plage de 0,001 à 30 % en poids, par rapport à la masse sèche de l'extrait.

9. Extrait selon la revendication 8, obtenu ou pouvant être obtenu par un procédé comprenant l'étape suivants :
a) extraction une ou plusieurs fois de matière végétale de *Mycetia balansae* à l'aide d'un agent d'extraction, de préférence liquide, choisi dans le groupe constitué par l'eau, l'éthanol, le méthanol, le 1-propanol, le 2-propanol, le glycérol, le 1,2-propanediol, le dioxyde de carbone supercritique, l'acétate d'éthyle et des mélanges de ceux-ci,
ainsi qu'éventuellement une ou plusieurs des étapes suivantes :
b) éventuellement concentration de l'extrait primaire obtenu dans l'étape a), de préférence par un ou plusieurs procédés d'évaporation ou de pervaporation,
c) éventuellement traitement de l'extrait primaire éventuellement concentré dans l'étape b), par ou sur des adsorbants, de préférence choisis dans le groupe constitué par le gel de silice, un gel de silice modifié, le charbon actif, une zéolithe, la bentonite, le kieselguhr, l'oxyde d'aluminium, un échangeur d'ions basique ou acide ou neutre, en option macroporeux, de préférence dans le procédé sur colonne ou en mode discontinu, éventuellement en ayant recours à d'autres agents d'extraction, de sorte qu'on obtient un extrait purifié (extrait secondaire),
d) éventuellement séchage de l'extrait secondaire obtenu dans l'étape c), de préférence par un procédé d'évaporation ou de pervaporation,
e) éventuellement mélange de l'extrait secondaire séché obtenu dans l'étape d) avec un diluant convenable ou un mélange de deux ou plus de deux diluants, de préférence choisi(s) dans le groupe constitué par l'éthanol, l'isopropanol, le 1,2-propylèneglycol, des triglycérides d'huiles végétales, la diacétine, la triacétine et le glycérol, avec obtention de préférence d'une solution.

10. Préparation consommable par voie orale, comprenant une quantité à activité sensorielle d'un composé tel que défini dans l'une quelconque des revendications 1 à 6 ou d'un sel physiologiquement acceptable d'un composé tel que défini dans l'une quelconque des revendications 1 à 6, d'un mélange selon la revendication 7, et/ou d'un extrait selon la revendication 8 ou 9,
la préparation consommable par voie orale étant de préférence choisie dans le groupe constitué par les préparations servant à l'alimentation, les préparations servant de complément nutritionnel, les préparations servant comme autres produits de consommation, les préparations pharmaceutiques orales, les produits de soin buccal, les compositions d'arômes et les préparations cosmétiques.

11. Préparation consommable par voie orale selon la revendication 10, en outre comprenant un, deux, trois, quatre, cinq, six, sept, huit, neuf, dix ou plus de dix autres substances, choisies dans les groupes (a1) à (a5) suivantes :
(a1) arômes, de préférence un, deux, trois, quatre, cinq ou plus de cinq des arômes étant choisis dans le groupe constitué par la vanilline, l'éthylvanilline, le 2-hydroxy-4-méthoxy-benzaldéhyde, l'isobutyrate d'éthylvanilline (= 3-éthoxy-4-isobutyryloxybenzaldéhyde), le Furaneol^{®} (2,5-diméthyl-4-hydroxy-3(2H)-furanone) et dérivés (par exemple homofuranéol, 2-éthyl-4-hydroxy-5-méthyl-3(2H)-furanone), homofuronol (2-éthyl-5-méthyl-4-hydroxy-3(2H)-furanone et 5-éthyl-2-méthyl-4-hydroxy-3(2H)-furanone), le maltol et ses dérivés (par exemple éthylmaltol), la coumarine et ses dérivés, une gamma-lactone (par exemple gamma-undécalactone, gamma-nonalactone), une delta-lactone (par exemple 4-méthyldeltalactone, massoilactone, deltadécalactone, tubérolactone), le sorbate de méthyle, la divanilline, la 4-hydroxy-2(ou 5)-éthyl-5(ou 2)-méthyl-3(2H)-furanone, la 2-hydroxy-3-méthyl-2-cyclopenténone, la 3-hydroxy-4,5-diméthyl-2(5H)-furanone, des esters de fruits et lactones de fruits (par exemple acétate de n-butyle, acétate d'isoamyle, propionate d'éthyle, butyrate d'éthyle, butyrate de n-butyle, butyrate d'isoamyle, 3-méthyl-butyrate d'éthyle, n-hexanoate d'éthyle, n-hexanoate d'allyle, n-hexanoate de n-butyle, n-octanoate d'éthyle, 3-méthyl-3-phénylglycidate d'éthyle, 2-trans-4-cis-décadiénoate d'éthyle), la 4-(p-hydroxyphényl)-2-butanone, le 1,1-diméthoxy-2,2,5-triméthyl-4-hexane, le 2,6-diméthyl-5-heptén-1-al et le phénylacétaldéhyde ;
(a2) glucides choisis dans le groupe constitué par le saccharose, le tréhalose, le lactose, le maltose, le mélizitose, le mélibiose, le raffinose, le palatinose, le lactulose, le D-fructose, le D-glucose, le D-galactose, le L-rhamnose, le D-sorbose, le D-mannose, le D-tagatose, le D-arabinose, le L-arabinose, le D-ribose, le D-glycéraldéhyde, les maltodextrines et des préparations végétales contenant un ou plusieurs des glucides nommés, de préférence en une proportion d'au moins 5 % en poids, encore mieux d'au moins 15 % en poids, les glucides pouvant également se trouver sous forme de mélange existant dans la nature ou préparé artificiellement, dans le cas présent notamment sous forme de miel, de sirop de sucre inverti ou de fructose hautement enrichi provenant d'amidon de maïs, et les sels physiologiquement acceptables de ces glucides, en particulier les sels de sodium, potassium, calcium ou d'ammonium ;
(a3) alcools glucidiques, de préférence alcools glucidiques existant dans la nature, choisis dans le groupe constitué par le glycérol, l'érythritol, le thréitol, l'arabitol, le ribitol, le xylitol, le sorbitol, le mannitol, le maltitol, l'isomaltitol, le dulcitol, le lactitol, et les sels physiologiquement acceptables de ces alcools glucidiques, en particulier les sels de sodium, potassium, calcium ou d'ammonium ;
(a4) édulcorants existant dans la nature, de préférence choisis dans le groupe constitué par
(a4-1) la miraculine, la monelline, la mabinline, la thaumatine, la curculine, la brazzéine, la pentaïdine, la D-phénylalanine, le D-tryptophane, et des fractions ou extraits obtenus à partir de sources naturelles, qui contiennent ces acides aminés et/ou ces protéines, et les sels physiologiquement acceptables de ces acides aminés et/ou de ces protéines, en particulier les sels de sodium, potassium, calcium ou d'ammonium ;
(a4-2) la néohespéridine dihydrochalcone, la naringine dihydrochalcone, le stévioside, le stéviolbioside, les rebaudiosides, en particulier le rebaudioside A, le rebaudioside B, le rebaudioside C, le rebaudioside D, le rebaudioside E, le rebaudioside F, le rebaudioside G, le rebaudioside H, les dulcosides et le rubusoside, le suavioside A, le suavioside B, le suavioside G, le suavioside H, le suavioside I, le suavioside J, le baiyunoside 1, le baiyunoside 2, le phlomisoside 1, le phlomisoside 2, le phlomisoside 3, ainsi que le phlomisoside 4, l'abrusoside A, l'abrusoside B, l'abrusoside C, l'abrusoside D, le cyclocaryoside A et le cyclocaryoside I, l'oslandine, le polypodoside A, la strogine 1, la strogine 2, la strogine 4, la selliguéanine A, le 3-acétate de dihydroquercétine, la périllartine, le télosmoside A₁₅, la périandrine I-V, les ptérocaryosides, les cyclocaryosides, les mukuroziosides, le trans-anéthol, le trans-cinnamaldéhyde, les bryosides, les bryonosides, les bryonodulcosides, les carnosiflosides, les scandénosides, les gypénosides, la trilobatine, la phloridzine, les dihydroflavanols, l'hématoxyline, la cyanine, l'acide chlorogénique, l'albiziasaponine, les télosmosides, le gaudichaudioside, les mogrosides, le mogroside V, les hernandulcines, la monatine, la phyllodulcine, l'acide glycyrrhétinique et ses dérivés, en particulier ses glycosides tels que la glycyrrhizine, et les sels physiologiquement acceptables de ces composés, en particulier les sels de sodium, potassium, calcium ou d'ammonium ;
(a4-3) extraits ou fractions enrichies des extraits, choisis dans le groupe constitué par les extraits de *Thaumatococcus* (katemfe), les extraits de *Stevia ssp.* (en particulier *Stevia rebaudiana*), les extraits de Luo Han (*Momordica* ou *Siratia grosvenorii,* Luo-Han-Guo), les extraits de *Glycerrhyzia ssp.* (en particulier *Glycerrhyzia glabra*), les extraits de *Rubus* ssp. (en particulier *Rubus suavissimus*), les extraits de *Citrus* et les extraits de *Lippia dulcis ;*
(a5) substances synthétiques à saveur sucrée, de préférence choisies dans le groupe constitué par magap, le cyclamate de sodium ou d'autres sels physiologiquement acceptables de l'acide cyclamique, l'acésulfame K ou d'autres sels physiologiquement acceptables, la néohespéridine dihydrochalcone, la naringine dihydrochalcone, la saccharine, la saccharine-sel de sodium, l'aspartame, le super-aspartame, le néotame, l'alitame, l'advantame, la perillartine, le sucralose, le lugduname, le carrélame, le sucrononate et le sucro-octate.

12. Préparation consommable par voie orale selon l'une quelconque des revendications 10 et 11, dans laquelle la quantité totale des composés de formule (I) se situe dans la plage de 0,01 ppm à 95 % en poids, de préférence dans la plage de 0,1 ppm à 90 % en poids, encore mieux dans la plage de 1 ppm à 50 % en poids, de façon particulièrement préférée dans la plage de 1 ppm à 20 % en poids, de façon plus particulièrement préférée dans la plage de 1 ppm à 5 % en poids, par rapport au poids total de la préparation.

13. Procédé pour (a) l'induction d'une impression de saveur sucrée et/ou l'accentuation d'une impression de saveur sucrée d'une, de deux ou de plus de deux substances à saveur sucrée et/ou (b) la production d'une préparation consommable par voie orale selon l'une quelconque des revendications 10 à 12, comportant les étapes suivantes :
a) disposition d'un composé tel que défini dans l'une quelconque des revendications 1 à 6 ou d'un sel physiologiquement acceptable d'un composé tel que défini dans l'une quelconque des revendications 1 à 6, d'un mélange selon la revendication 7 et/ou d'un extrait selon la revendication 8 ou 9,
b) disposition d'une préparation consommable par voie orale, de préférence comprenant une, deux ou plus de deux autres substances à saveur sucrée,
c) mise en contact ou mélange des composants fournis dans les étapes a) et b).

14. Procédé selon la revendication 13, comprenant les étapes consistant à
a-i) fournir un extrait, de préférence un extrait selon la revendication 8 ou 9,
comprenant un composé tel que défini dans l'une quelconque des revendications 1 à 6, un sel physiologiquement acceptable d'un composé tel que défini dans l'une quelconque des revendications 1 à 6, ou un mélange selon la revendication 7,
par extraction de matière végétale, de préférence de matière végétale de *Mycetia balansae ;*
a-ii) en option transformer l'extrait préparé dans l'étape (a-i) en un produit transformé comprenant un composé tel que défini dans l'une quelconque des revendications 1 à 6, un sel physiologiquement acceptable d'un composé tel que défini dans l'une quelconque des revendications 1 à 6, un mélange selon la revendication 7,
b) disposition d'une préparation consommable par voie orale, de préférence comprenant une, deux ou plus de deux autres substances à saveur sucrée,
c) mise en contact ou mélange de la préparation consommable par voie orale, provenant de l'étape b), qui de préférence comprend une, deux ou plus de deux autres substances à saveur sucrée, avec l'extrait préparé dans l'étape a-i) ou le produit transformé préparé dans l'étape a-ii).

15. Utilisation d'un composé tel que défini dans l'une quelconque des revendications 1 à 6 ou d'un sel physiologiquement acceptable d'un composé tel que défini dans l'une quelconque des revendications 1 à 6, d'un mélange selon la revendication 7, ou d'un extrait selon la revendication 8 ou 9,
pour la production d'une impression sucrée dans une préparation consommable par voie orale ou pour l'accentuation de l'impression sucrée d'une préparation consommable par voie orale, comprenant au moins une autre substance à saveur sucrée, de préférence existant dans la nature.
